# EUROPEAN PATENT SPECIFICATION

(11) **EP 1 071 774 B1**
(45) Date of publication and mention of the grant of the patent: **22.11.2006**
(21) Application number: 99922714.3
(22) Date of filing: 16.04.1999
(51) Int. Cl.: C12N 15/12, C12N 15/11, C12N 15/63, C12N 15/62, C07K 14/47, C07K 16/18, A61K 31/70, A61K 38/17, A61K 48/00, G01N 33/50, G01N 33/53, G01N 33/68, C12Q 1/68, A01K 67/027

(54) **ISOLATED SH3 GENES ASSOCIATED WITH MYELOPROLIFERATIVE DISORDERS AND LEUKEMIA, AND USES THEREOF**
ISOLIERTE SH3 GENE, DIE MIT MYELOPROLIFERATIVEN ERKRANKUNGEN UND LEUKÄMIE VERBUNDEN SIND, UND DEREN VERWENDUNGEN
GENES SH3 ASSOCIES AU SYNDROME MYELOPROLIFERATIF ET A LA LEUCEMIE, ET UTILISATION DE CES GENES

(30) Priority: 16.04.1998 US 82007 P
(43) Date of publication of application: 31.01.2001
(73) Proprietor: Cedar-Sinai Health System, et al, Los Angeles, CA 90048-1865 (US)
(72) Inventor: KORENBERG, Julie, R., Los Angeles, CA 90046 (US); CHEN, Xiao-Ning, Arcadia, CA 91006 (US)
(74) Representative: Mercer, Christopher Paul
(86) International application number: PCT/US1999/008371
(87) International publication number: WO 1999/053062

(56) References cited:
- WO-A-96/31625
- WO-A-99/55728
- US-A- 5 717 067
- CHEN H AND ANTONARAKIS S: "The SH3D1A gene maps to human chromosome 21q22.1-->q22.2." CYTOGENET. CELL GENET., vol. 78, 1997, pages 213-215, XP000857354 cited in the application -& SPARKS A ET AL: "Cloning of ligand targets: systematic isolation of SH3 domain-containing proteins" NAT. BIOTECHNOL., vol. 14, no. 6, June 1996 (1996-06), pages 741-744, XP002124425 cited in the application -& DATABASE SWISSPROT [Online] Accession Number:Q15811, 1 November 1997 (1997-11-01) CHEN H ET AL: "Intersectin (SH3 domain-containing protein SH3P17) / ITSN or SH3D1A / Human" XP002124426

### SEQUENCE LISTING

<110> Korenberg, Julie R
   Chen, Xiao-Ning
<120> ISOLATED SH3 GENES ASSOCIATED WITH MYELOPROLIFERATIVE DISORDERS AND LEUKEMIA, AND USES THEREOF
<130> 2320-1-001PCT
<140> PCT/US99/08371
   <141> 1999-04-16
<150> 60/082,007
   <151> 1998-04-16
<160> 109
<170> PatentIn Ver. 2.0
<210> 1
   <211> 5199
   <212> DNA
   <213> Homo sapiens
<400> 1
   caaaagaatt ccgggtacgg cggctcgcga ggaagaatcc cgagcgggct ccgggacgga 60
   cagagaggcg ggcggggatg gtgtgcgggg ctgcggctcc tgcgtccctc ccagcggcgc 120
   gtgagcggca ctgatttgtc cctggggcgg cagcgcggac ccgcccggag atgaggcgtc 180
   gattagcaag gtaaaagtaa cagaaccatg gctcagtttc caacaccttt tggtggcagc 240
   ctggatatct gggccataac tgtagaggaa agagcgaagc atgatcagca gttccatagt 300
   ttaaagccaa tatctggatt cattactggt gatcaagcta gaaacttttt ttttcaatct 360
   gggttacctc aacctgtttt agcacagata tgggcactag ctgacatgaa taatgatgga 420
   agaatggatc aagtggagtt ttccatagct atgaaactta tcaaactgaa gctacaagga 480
   tatcagctac cctctgcact tccccctgtc atgaaacagc aaccagttgc tatttctagc 540
   gcaccagcat ttggtatggg aggtatcgcc agcatgccac cgcttacagc tgttgctcca 600
   gtgccaatgg gatccattcc agttgttgga atgtctccaa ccctagtatc ttctgttccc 660
   acagcagctg tgccccccct ggctaacggg gctccccctg ttatacaacc tctgcctgca 720
   tttgctcatc ctgcagccac attgccaaag agttcttcct ttagtagatc tggtccaggg 780
   tcacaactaa acactaaatt acaaaaggca cagtcatttg atgtggccag tgtcccacca 840
   gtggcagagt gggctgttcc tcagtcatca agactgaaat acaggcaatt attcaatagt 900
   catgacaaaa ctatgagtgg acacttaaca ggtccccaag caagaactat tcttatgcag 960
   tcaagtttac cacaggctca gctggcttca atatggaatc tttctgacat tgatcaagat 1020
   ggaaaactta cagcagagga atttatcctg gcaatgcacc tcattgatgt agctatgtct 1080
   ggccaaccac tgccacctgt cctgcctcca gaatacattc caccttcttt tagaagagtt 1140
   cgatctggca gtggtatatc tgtcataagc tcaacatctg tagatcagag gctaccagag 1200
   gaaccagttt tagaagatga acaacaacaa ttagaaaaga aattacctgt aacgtttgaa 1260
   gataagaagc gggagaactt tgaacgtggc aacctggaac tggagaaacg aaggcaagct 1320
   ctcctggaac agcagcgcaa ggagcaggag cgcctggccc agctggagcg ggcggagcag 1380
   gagaggaagg agcgtgagcg ccaggagcaa gagcgcaaaa gacaactgga actggagaag 1440
   caactggaaa agcagcggga gctagaacgg cagagagagg aggagaggag gaaagaaatt 1500
   gagaggcgag aggctgcaaa acgggaactt gaaaggcaac gacaacttga gtgggaacgg 1560
   aatcgaaggc aagaactact aaatcaaaga aacaaagaac aagaggacat agttgtactg 1620
   aaagcaaaga aaaagacttt ggaatttgaa ttagaagctc taaatgataa aaagcatcaa 1680
   ctagaaggga aacttcaaga tatcagatgt cgattgacca cccaaaggca agaaattgag 1740
   agcacaaaca aatctagaga gttgagaatt gccgaaatca cccatctaca gcaacaatta 1800
   caggaatctc agcaaatgct tggaagactt attccagaaa aacagatact caatgaccaa 1860
   ttaaaacaag ttcagcagaa cagtttgcac agagattcac ttgttacact taaaagagcc 1920
   ttagaagcaa aagaactagc tcggcagcac ctacgagacc aactggatga agtggagaaa 1980
   gaaactagat caaaactaca ggagattgat attttcaata atcagctgaa ggaactaaga 2040
   gaaatacaca ataagcaaca actccagaag caaaagtcca tggaggctga acgactgaaa 2100
   cagaaagaac aagaacgaaa gatcatagaa ttagaaaaac aaaaagaaga agcccaaaga 2160
   cgagctcagg aaagggacaa gcagtggctg gagcatgtgc agcaggagga cgagcatcag 2220
   agaccaagaa aactccacga agaggaaaaa ctgaaaaggg aggagagtgt caaaaagaag 2280
   gatggcgagg aaaaaggcaa acaggaagca caagacaagc tgggtcggct tttccatcaa 2340
   caccaagaac cagctaagcc agctgtccag gcaccctggt ccactgcaga aaaaggtcca 2400
   cttaccattt ctgcacagga aaatgtaaaa gtggtgtatt accgggcact gtaccccttt 2460
   gaatccagaa gccatgatga aatcactatc cagccaggag acatagtcat ggtggatgaa 2520
   agccaaactg gagaacccgg ctggcttgga ggagaattaa aaggaaagac agggtggttc 2580
   cctgcaaact atgcagagaa aatcccagaa aatgaggttc ccgctccagt gaaaccagtg 2640
   actgattcaa catctgcccc tgcccccaaa ctggccttgc gtgagacccc cgcccctttg 2700
   gcagtaacct cttcagagcc ctccacgacc cctaataact gggccgactt cagctccacg 2760
   tggcccacca gcacgaatga gaaaccagaa acggataact gggatgcatg ggcagcccag 2820
   ccctctctca ccgttccaag tgccggccag ttaaggcaga ggtccgcctt tactccagcc 2880
   acggccactg gctcctcccc gtctcctgtg ctaggccagg gtgaaaaggt ggaggggcta 2940
   caagctcaag ccctatatcc ttggagagcc aaaaaagaca accacttaaa ttttaacaaa 3000
   aatgatgtca tcaccgtcct ggaacagcaa gacatgtggt ggtttggaga agttcaaggt 3060
   cagaagggtt ggttccccaa gtcttacgtg aaactcattt cagggcccat aaggaagtct 3120
   acaagcatgg attctggttc ttcagagagt cctgctagtc taaagcgagt agcctctcca 3180
   gcagccaagc cggtcgtttc gggagaagaa attgcccagg ttattgcctc atacaccgcc 3240
   accggccccg agcagctcac tctcgcccct ggtcagctga ttttgatccg aaaaaagaac 3300
   ccaggtggat ggtgggaagg agagctgcaa gcacgtggga aaaagcgcca gataggctgg 3360
   ttcccagcta attatgtaaa gcttctaagc cctgggacga gcaaaatcac tccaacagag 3420
   ccacctaagt caacagcatt agcggcagtg tgccaggtga ttgggatgta cgactacacc 3480
   gcgcagaatg acgatgagct ggccttcaac aagggccaga tcatcaacgt cctcaacaag 3540
   gaggaccctg actggtggaa aggagaagtc aatggacaag tggggctctt cccatccaat 3600
   tatgtgaagc tgaccacaga catggaccca agccagcaat gaatcatatg ttgtccatcc 3660
   ccccctcagg cttgaaagtc ctcaaagaga cccactatcc catatcactg cccagaggga 3720
   tgatgggaga tgcagccttg atcatgtgac ttccagcatg atcacctact gccttctgag 3780
   tagaagaact cactgcagag cagtttacct cattttacct tagttgcatg tgatcgcaat 3840
   gtttgagtta ttacttgcag agataggagc aaaaattaca aaaacacaca gggtagtggg 3900
   tccttttgtg gctttcctag ttactcaaat tgactttccc ccacctttgc acaggtgctt 3960
   tcaatagttt taaaattatt tttaaatata tattttagct ttttaataaa caaaataaat 4020
   aaatgacttc tttgctattt tggttttgca aaaagaccca ctatcaagga atgctgcatg 4080
   tgctattaaa aattgttcca aatgtccata aatctgagac ttgatgtatt ttttcatttt 4140
   gtccagtgtt accaactaaa ttgctgcagt ttggggcttt tcccccttac catagaagtg 4200
   cagaggagtt cagtatctct gttttaaaga cgtatagaat gagcccaatt aaagcgaagg 4260
   tgattgtgct tgtttgtgtg tatcagctgt accttgttga gcatgtaata catcctgtac 4320
   ataagaaatt agttctttcc atggcaaagc tattaccttg tacgatgctc taatcatatt 4380
   gcatttaatt ttattttgca acagtgacct tgtagccaca tgagaaagca ctctgtgttt 4440
   ttgttcggtc tcagatttat ctggttgagt tggtgttttg tttggggttt ttaattttgc 4500
   gtgtttgcat agcataaaat cagtagacaa caccactgag gtcgttacga tcaacgatat 4560
   ccacagtctc tttttagtct ctgttacatg aagttttatt ccagttactt ttcatggaat 4620
   gacctatttt gaacaagtaa ttttcttgac aagaaagaat gtatagaagt ctccctgcaa 4680
   ttaatttcca atgtttacat tttttaacta ggactgtgga atttctacag attaatatga 4740
   aatggagctc atggtccgtt tgtgtgttag atatgctgta gctgaagccc tgtttgtctt 4800
   ttaaacacta gttggaagct ctcaataaaa atgcctgctg ctcacagcac agaaaatggg 4860
   gcagggggag cctcaagcac aatctagctg tcctcctaaa gactctgtaa tgctcaatcc 4920
   ccttgcgttc tcccggcgct gtcgggaggc tgtgctggtg gtcgtgtaga ggtccttttc 4980
   ctttcaaatg gtgcagagag agaggacctt tcctccttgt tcagttgcaa ttcagtattt 5040
   tcacggatat gaatgtaaaa tatataaata tataaacctg aggatttaac aaatgtaaaa 5100
   caaccttttg aattagttcc gagtatagat aattaaattt ttaaaacaaa agtaaaaaaa 5160
   aaaaaaaaaa aaaaaaaaaa aaaagtcgac gcggccgcg                       5199
<210> 2
   <211> 1143
   <212> PRT
   <213> Homo sapiens
<400> 2
   Met Ala Gln Phe Pro Thr Pro Phe Gly Gly Ser Leu Asp Ile Trp Ala
   1               5                   10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Ala Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
   130 135 140
   Thr Leu Val Ser Ser Val Pro Thr Ala Ala Val Pro Pro Leu Ala Asn
   145 150 155 160
   Gly Ala Pro Pro Val Ile Gln Pro Leu Pro Ala Phe Ala His Pro Ala
   165 170 175
   Ala Thr Leu Pro Lys Ser Ser Ser Phe Ser Arg Ser Gly Pro Gly Ser
   180 185 190
   Gln Leu Asn Thr Lys Leu Gln Lys Ala Gln Ser Phe Asp Val Ala Ser
   195 200 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385                 390                 395                 400
   Gln Glu Arg Lys Arg Gln Leu Glu Leu Glu Lys Gln Leu Glu Lys Gln
                   405                 410                 415
   Arg Glu Leu Glu Arg Gln Arg Glu Glu Glu Arg Arg Lys Glu Ile Glu
               420                 425                 430
   Arg Arg Glu Ala Ala Lys Arg Glu Leu Glu Arg Gln Arg Gln Leu Glu
           435                 440                 445
   Trp Glu Arg Asn Arg Arg Gln Glu Leu Leu Asn Gln Arg Asn Lys Glu
       450                 455                 460
   Gln Glu Asp Ile Val Val Leu Lys Ala Lys Lys Lys Thr Leu Glu Phe
   465                 470                 475                 480
   Glu Leu Glu Ala Leu Asn Asp Lys Lys His Gln Leu Glu Gly Lys Leu
                   485                 490                 495
   Gln Asp Ile Arg Cys Arg Leu Thr Thr Gln Arg Gln Glu Ile Glu Ser
               500                 505                 510
   Thr Asn Lys Ser Arg Glu Leu Arg Ile Ala Glu Ile Thr His Leu Gln
           515                 520                 525
   Gln Gln Leu Gln Glu Ser Gln Gln Met Leu Gly Arg Leu Ile Pro Glu
       530                 535                 540
   Lys Gln Ile Leu Asn Asp Gln Leu Lys Gln Val Gln Gln Asn Ser Leu
   545                 550                 555                 560
   His Arg Asp Ser Leu Val Thr Leu Lys Arg Ala Leu Glu Ala Lys Glu
                   565                 570                 575
   Leu Ala Arg Gln His Leu Arg Asp Gln Leu Asp Glu Val Glu Lys Glu
               580                 585                 590
   Thr Arg Ser Lys Leu Gln Glu Ile Asp Ile Phe Asn Asn Gln Leu Lys
           595                 600                 605
   Glu Leu Arg Glu Ile His Asn Lys Gln Gln Leu Gln Lys Gln Lys Ser
       610                 615                 620
   Met Glu Ala Glu Arg Leu Lys Gln Lys Glu Gln Glu Arg Lys Ile Ile
   625                 630                 635                 640
   Glu Leu Glu Lys Gln Lys Glu Glu Ala Gln Arg Arg Ala Gln Glu Arg
   645 650 655
   Asp Lys Gln Trp Leu Glu His Val Gln Gln Glu Asp Glu His Gln Arg
   660 665 670
   Pro Arg Lys Leu His Glu Glu Glu Lys Leu Lys Arg Glu Glu Ser Val
   675 680 685
   Lys Lys Lys Asp Gly Glu Glu Lys Gly Lys Gln Glu Ala Gln Asp Lys
   690 695 700
   Leu Gly Arg Leu Phe His Gln His Gln Glu Pro Ala Lys Pro Ala Val
   705 710 715 720
   Gln Ala Pro Trp Ser Thr Ala Glu Lys Gly Pro Leu Thr Ile Ser Ala
   725 730 735
   Gln Glu Asn Val Lys Val Val Tyr Tyr Arg Ala Leu Tyr Pro Phe Glu
   740 745 750
   Ser Arg Ser His Asp Glu Ile Thr Ile Gln Pro Gly Asp Ile Val Met
   755 760 765
   Val Asp Glu Ser Gln Thr Gly Glu Pro Gly Trp Leu Gly Gly Glu Leu
   770 775 780
   Lys Gly Lys Thr Gly Trp Phe Pro Ala Asn Tyr Ala Glu Lys Ile Pro
   785 790 795 800
   Glu Asn Glu Val Pro Ala Pro Val Lys Pro Val Thr Asp Ser Thr Ser
   805 810 815
   Ala Pro Ala Pro Lys Leu Ala Leu Arg Glu Thr Pro Ala Pro Leu Ala
   820 825 830
   Val Thr Ser Ser Glu Pro Ser Thr Thr Pro Asn Asn Trp Ala Asp Phe
   835 840 845
   Ser Ser Thr Trp Pro Thr Ser Thr Asn Glu Lys Pro Glu Thr Asp Asn
   850 855 860
   Trp Asp Ala Trp Ala Ala Gln Pro Ser Leu Thr Val Pro Ser Ala Gly
   865 870 875 880
   Gln Leu Arg Gln Arg Ser Ala Phe Thr Pro Ala Thr Ala Thr Gly Ser
   885 890 895
   Ser Pro Ser Pro Val Leu Gly Gln Gly Glu Lys Val Glu Gly Leu Gln
               900                 905                 910
   Ala Gln Ala Leu Tyr Pro Trp Arg Ala Lys Lys Asp Asn His Leu Asn
           915                 920                 925
   Phe Asn Lys Asn Asp Val Ile Thr Val Leu Glu Gln Gln Asp Met Trp
       930                 935                 940
   Trp Phe Gly Glu Val Gln Gly Gln Lys Gly Trp Phe Pro Lys Ser Tyr
   945                 950                 955                 960
   Val Lys Leu Ile Ser Gly Pro Ile Arg Lys Ser Thr Ser Met Asp Ser
                   965                 970                 975
   Gly Ser Ser Glu Ser Pro Ala Ser Leu Lys Arg Val Ala Ser Pro Ala
               980                 985                 990
   Ala Lys Pro Val Val Ser Gly Glu Glu Ile Ala Gln Val Ile Ala Ser
           995                 1000                1005
   Tyr Thr Ala Thr Gly Pro Glu Gln Leu Thr Leu Ala Pro Gly Gln Leu
       1010                1015                1020
   Ile Leu Ile Arg Lys Lys Asn Pro Gly Gly Trp Trp Glu Gly Glu Leu
   1025                1030                1035                1040
   Gln Ala Arg Gly Lys Lys Arg Gln Ile Gly Trp Phe Pro Ala Asn Tyr
                   1045                1050                1055
   Val Lys Leu Leu Ser Pro Gly Thr Ser Lys Ile Thr Pro Thr Glu Pro
               1060                1065                1070
   Pro Lys Ser Thr Ala Leu Ala Ala Val Cys Gln Val Ile Gly Met Tyr
           1075                1080                1085
   Asp Tyr Thr Ala Gln Asn Asp Asp Glu Leu Ala Phe Asn Lys Gly Gln
       1090                1095                1100
   Ile Ile Asn Val Leu Asn Lys Glu Asp Pro Asp Trp Trp Lys Gly Glu
   1105                1110                1115                1120
   Val Asn Gly Gln Val Gly Leu Phe Pro Ser Asn Tyr Val Lys Leu Thr
                   1125                1130                1135
   Thr Asp Met Asp Pro Ser Gln
               1140
<210> 3
   <211> 5458
   <212> DNA
   <213> Homo sapiens
<400> 3
   gcacgagagg gagcgaagga ggtagagaag agtggaggcg ccaggggagg gagcgtagct 60
   tggttgctcc gtagtacggc ggctcgcgag gaagaatccc gagcgggctc cgggacggac 120
   agagaggcgg gcggggatgg tgtgcggggc tgcggctcct gcgtccctcc cagcggcgcg 180
   tgagcggcac tgatttgtcc ctggggcggc agcgcggacc cgcccggaga tgaggcgtcg 240
   attagcaagg taaaagtaac agaaccatgg ctcagtttcc aacacctttt ggtggcagcc 300
   tggatatctg ggccataact gtagaggaaa gagcgaagca tgatcagcag ttccatagtt 360
   taaagccaat atctggattc attactggtg atcaagctag aaactttttt tttcaatctg 420
   ggttacctca acctgtttta gcacagatat gggcactagc tgacatgaat aatgatggaa 480
   gaatggatca agtggagttt tccatagcta tgaaacttat caaactgaag ctacaaggat 540
   atcagctacc ctctgcactt ccccctgtca tgaaacagca accagttgct atttctagcg 600
   caccagcatt tggtatggga ggtatcgcca gcatgccacc gcttacagct gttgctccag 660
   tgccaatggg atccattcca gttgttggaa tgtctccaac cctagtatct tctgttccca 720
   cagcagctgt gccccccctg gctaacgggg ctccccctgt tatacaacct ctgcctgcat 780
   ttgctcatcc tgcagccaca ttgccaaaga gttcttcctt tagtagatct ggtccagggt 840
   cacaactaaa cactaaatta caaaaggcac agtcatttga tgtggccagt gtcccaccag 900
   tggcagagtg ggctgttcct cagtcatcaa gactgaaata caggcaatta ttcaatagtc 960
   atgacaaaac tatgagtgga cacttaacag gtccccaagc aagaactatt cttatgcagt 1020
   caagtttacc acaggctcag ctggcttcaa tatggaatct ttctgacatt gatcaagatg 1080
   gaaaacttac agcagaggaa tttatcctgg caatgcacct cattgatgta gctatgtctg 1140
   gccaaccact gccacctgtc ctgcctccag aatacattcc accttctttt agaagagttc 1200
   gatctggcag tggtatatct gtcataagct caacatctgt agatcagagg ctaccagagg 1260
   aaccagtttt agaagatgaa caacaacaat tagaaaagaa attacctgta acgtttgaag 1320
   ataagaagcg ggagaacttt gaacgtggca acctggaact ggagaaacga aggcaagctc 1380
   tcctggaaca gcagcgcaag gagcaggagc gcctggccca gctggagcgg gcggagcagg 1440
   agaggaagga gcgtgagcgc caggagcaag agcgcaaaag acaactggaa ctggagaagc 1500
   aactggaaaa gcagcgggag ctagaacggc agagagagga ggagaggagg aaagaaattg 1560
   agaggcgaga ggctgcaaaa cgggaacttg aaaggcaacg acaacttgag tgggaacgga 1620
   atcgaaggca agaactacta aatcaaagaa acaaagaaca agaggacata gttgtactga 1680
   aagcaaagaa aaagactttg gaatttgaat tagaagctct aaatgataaa aagcatcaac 1740
   tagaagggaa acttcaagat atcagatgtc gattgaccac ccaaaggcaa gaaattgaga 1800
   gcacaaacaa atctagagag ttgagaattg ccgaaatcac ccatctacag caacaattac 1860
   aggaatctca gcaaatgctt ggaagactta ttccagaaaa acagatactc aatgaccaat 1920
   taaaacaagt tcagcagaac agtttgcaca gagattcact tgttacactt aaaagagcct 1980
   tagaagcaaa agaactagct cggcagcacc tacgagacca actggatgaa gtggagaaag 2040
   aaactagatc aaaactacag gagattgata ttttcaataa tcagctgaag gaactaagag 2100
   aaatacacaa taagcaacaa ctccagaagc aaaagtccat ggaggctgaa cgactgaaac 2160
   agaaagaaca agaacgaaag atcatagaat tagaaaaaca aaaagaagaa gcccaaagac 2220
   gagctcagga aagggacaag cagtggctgg agcatgtgca gcaggaggac gagcatcaga 2280
   gaccaagaaa actccacgaa gaggaaaaac tgaaaaggga ggagagtgtc aaaaagaagg 2340
   atggcgagga aaaaggcaaa caggaagcac aagacaagct gggtcggctt ttccatcaac 2400
   accaagaacc agctaagcca gctgtccagg caccctggtc cactgcagaa aaaggtccac 2460
   ttaccatttc tgcacaggaa aatgtaaaag tggtgtatta ccgggcactg tacccctttg 2520
   aatccagaag ccatgatgaa atcactatcc agccaggaga catagtcatg gttaaagggg 2580
   aatgggtgga tgaaagccaa actggagaac ccggctggct tggaggagaa ttaaaaggaa 2640
   agacagggtg gttccctgca aactatgcag agaaaatccc agaaaatgag gttcccgctc 2700
   cagtgaaacc agtgactgat tcaacatctg cccctgcccc caaactggcc ttgcgtgaga 2760
   cccccgcccc tttggcagta acctcttcag agccctccac gacccctaat aactgggccg 2820
   acttcagctc cacgtggccc accagcacga atgagaaacc agaaacggat aactgggatg 2880
   catgggcagc ccagccctct ctcaccgttc caagtgccgg ccagttaagg cagaggtccg 2940
   cctttactcc agccacggcc actggctcct ccccgtctcc tgtgctaggc cagggtgaaa 3000
   aggtggaggg gctacaagct caagccctat atccttggag agccaaaaaa gacaaccact 3060
   taaattttaa caaaaatgat gtcatcaccg tcctggaaca gcaagacatg tggtggtttg 3120
   gagaagttca aggtcagaag ggttggttcc ccaagtctta cgtgaaactc atttcagggc 3180
   ccataaggaa gtctacaagc atggattctg gttcttcaga gagtcctgct agtctaaagc 3240
   gagtagcctc tccagcagcc aagccggtcg tttcgggaga agaatttatt gccatgtaca 3300
   cttacgagag ttctgagcaa ggagatttaa cctttcagca aggggatgtg attttggtta 3360
   ccaagaaaga tggtgactgg tggacaggaa cagtgggcga caaggccgga gtcttccctt 3420
   ctaactatgt gaggcttaaa gattcagagg gctctggaac tgctgggaaa acagggagtt 3480
   taggaaaaaa acctgaaatt gcccaggtta ttgcctcata caccgccacc ggccccgagc 3540
   agctcactct cgcccctggt cagctgattt tgatccgaaa aaagaaccca ggtggatggt 3600
   gggaaggaga gctgcaagca cgtgggaaaa agcgccagat aggctggttc ccagctaatt 3660
   atgtaaagct tctaagccct gggacgagca aaatcactcc aacagagcca cctaagtcaa 3720
   cagcattagc ggcagtgtgc caggtgattg ggatgtacga ctacaccgcg cagaatgacg 3780
   atgagctggc cttcaacaag ggccagatca tcaacgtcct caacaaggag gaccctgact 3840
   ggtggaaagg agaagtcaat ggacaagtgg ggctcttccc atccaattat gtgaagctga 3900
   ccacagacat ggacccaagc cagcaatgaa tcatatgttg tccatccccc cctcaggctt 3960
   gaaagtcctc aaagagaccc actatcccat atcactgccc agagggatga tgggagatgc 4020
   agccttgatc atgtgacttc cagcatgatc acctactgcc ttctgagtag aagaactcac 4080
   tgcagagcag tttacctcat tttaccttag ttgcatgtga tcgcaatgtt tgagttatta 4140
   cttgcagaga taggagcaaa aattacaaaa acacacaggg tagtgggtcc ttttgtggct 4200
   ttcctagtta ctcaaattga ctttccccca cctttgcaca ggtgctttca atagttttaa 4260
   aattattttt aaatatatat tttagctttt taataaacaa aataaataaa tgacttcttt 4320
   gctattttgg ttttgcaaaa agacccacta tcaaggaatg ctgcatgtgc tattaaaaat 4380
   tgttccaaat gtccataaat ctgagacttg atgtattttt tcattttgtc cagtgttacc 4440
   aactaaattg tgcagtttgg ggcttttccc ccttaccata gaagtgcaga ggagttcagt 4500
   atctctgttt taaagacgta tagaatgagc ccaattaaag cgaaggtgtt tgtgcttgtt 4560
   tgtgtgtatc agctgtacct tgttgagcat gtaatacatc ctgtacataa gaaattagtt 4620
   ctttccatgg caaagctatt accttgtacg atgctctaat catattgcat ttaattttat 4680
   tttgcacagt gaccttgtag ccacatgaga aagcactctg tgtttttgtt cggtctcaga 4740
   tttatctggt tgagttggtg ttttgtttgg ggtttttaat tttgcgtgtt tgcatagcat 4800
   aaaatcagta gacaacacca ctgaggtcgt tacgatcaac gatatccaca gtctcttttt 4860
   agtctctgtt acatgaagtt ttattccagt tacttttcat ggaatgacct attttgaaca 4920
   agtaattttc ttgacaagaa agaatgtata gaagtctccc tgcaattaat ttccaatgtt 4980
   tacatttttt aactagactg tggaatttct acagattaat atgaaatgga gctcatggtc 5040
   cgtttgtgtg ttagatatgc tgtagctgaa gccctgtttg tcttttaaac actagttgga 5100
   agctctcaat aaaaatgcct gctgctcaca gcacagaaaa tggggcaggg ggagcctcaa 5160
   gcacaatcta gctgtcctcc taaagactct gtaatgctca ctcccctcgc gttctcccgg 5220
   cgctgtcggg aggctgtgct ggtggtcgtg tagaggtcct tctcctttca catggtgcag 5280
   agagcgagga cctctcctcc tcgttcagtt gcacttcagt attttcacgg atatgaatgt 5340
   aaaatatata aatatataaa cctgcggctt taacaactgt aatacaacct tttgaattag 5400
   ttccgtgtat agataattaa attcttcata caaaagttaa aaaaaaaaaa aaaaaaaa 5458
<210> 4
   <211> 1220
   <212> PRT
   <213> Homo sapiens
<400> 4
   Met Ala Gln Phe Pro Thr Pro Phe Gly Gly Ser Leu Asp Ile Trp Ala
   1               5                   10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Ala Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
       130                 135                 140
   Thr Leu Val Ser Ser Val Pro Thr Ala Ala Val Pro Pro Leu Ala Asn
   145                 150                 155                 160
   Gly Ala Pro Pro Val Ile Gln Pro Leu Pro Ala Phe Ala His Pro Ala
                   165                 170                 175
   Ala Thr Leu Pro Lys Ser Ser Ser Phe Ser Arg Ser Gly Pro Gly Ser
               180                 185                 190
   Gln Leu Asn Thr Lys Leu Gln Lys Ala Gln Ser Phe Asp Val Ala Ser
           195                 200                 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385 390 395 400
   Gln Glu Arg Lys Arg Gln Leu Glu Leu Glu Lys Gln Leu Glu Lys Gln
   405 410 415
   Arg Glu Leu Glu Arg Gln Arg Glu Glu Glu Arg Arg Lys Glu Ile Glu
   420 425 430
   Arg Arg Glu Ala Ala Lys Arg Glu Leu Glu Arg Gln Arg Gln Leu Glu
   435 440 445
   Trp Glu Arg Asn Arg Arg Gln Glu Leu Leu Asn Gln Arg Asn Lys Glu
   450 455 460
   Gln Glu Asp Ile Val Val Leu Lys Ala Lys Lys Lys Thr Leu Glu Phe
   465 470 475 480
   Glu Leu Glu Ala Leu Asn Asp Lys Lys His Gln Leu Glu Gly Lys Leu
   485 490 495
   Gln Asp Ile Arg Cys Arg Leu Thr Thr Gln Arg Gln Glu Ile Glu Ser
   500 505 510
   Thr Asn Lys Ser Arg Glu Leu Arg Ile Ala Glu Ile Thr His Leu Gln
   515 520 525
   Gln Gln Leu Gln Glu Ser Gln Gln Met Leu Gly Arg Leu Ile Pro Glu
   530 535 540
   Lys Gln Ile Leu Asn Asp Gln Leu Lys Gln Val Gln Gln Asn Ser Leu
   545 550 555 560
   His Arg Asp Ser Leu Val Thr Leu Lys Arg Ala Leu Glu Ala Lys Glu
   565 570 575
   Leu Ala Arg Gln His Leu Arg Asp Gln Leu Asp Glu Val Glu Lys Glu
   580 585 590
   Thr Arg Ser Lys Leu Gln Glu Ile Asp Ile Phe Asn Asn Gln Leu Lys
   595 600 605
   Glu Leu Arg Glu Ile His Asn Lys Gln Gln Leu Gln Lys Gln Lys Ser
   610 615 620
   Met Glu Ala Glu Arg Leu Lys Gln Lys Glu Gln Glu Arg Lys Ile Ile
   625 630 635 640
   Glu Leu Glu Lys Gln Lys Glu Glu Ala Gln Arg Arg Ala Gln Glu Arg
   645 650 655
   Asp Lys Gln Trp Leu Glu His Val Gln Gln Glu Asp Glu His Gln Arg
   660 665 670
   Pro Arg Lys Leu His Glu Glu Glu Lys Leu Lys Arg Glu Glu Ser Val
   675 680 685
   Lys Lys Lys Asp Gly Glu Glu Lys Gly Lys Gln Glu Ala Gln Asp Lys
   690 695 700
   Leu Gly Arg Leu Phe His Gln His Gln Glu Pro Ala Lys Pro Ala Val
   705 710 715 720
   Gln Ala Pro Trp Ser Thr Ala Glu Lys Gly Pro Leu Thr Ile Ser Ala
   725 730 735
   Gln Glu Asn Val Lys Val Val Tyr Tyr Arg Ala Leu Tyr Pro Phe Glu
   740 745 750
   Ser Arg Ser His Asp Glu Ile Thr Ile Gln Pro Gly Asp Ile Val Met
   755 760 765
   Val Lys Gly Glu Trp Val Asp Glu Ser Gln Thr Gly Glu Pro Gly Trp
   770 775 780
   Leu Gly Gly Glu Leu Lys Gly Lys Thr Gly Trp Phe Pro Ala Asn Tyr
   785 790 795 800
   Ala Glu Lys Ile Pro Glu Asn Glu Val Pro Ala Pro Val Lys Pro Val
   805 810 815
   Thr Asp Ser Thr Ser Ala Pro Ala Pro Lys Leu Ala Leu Arg Glu Thr
   820 825 830
   Pro Ala Pro Leu Ala Val Thr Ser Ser Glu Pro Ser Thr Thr Pro Asn
   835 840 845
   Asn Trp Ala Asp Phe Ser Ser Thr Trp Pro Thr Ser Thr Asn Glu Lys
   850 855 860
   Pro Glu Thr Asp Asn Trp Asp Ala Trp Ala Ala Gln Pro Ser Leu Thr
   865 870 875 880
   Val Pro Ser Ala Gly Gln Leu Arg Gln Arg Ser Ala Phe Thr Pro Ala
   885 890 895
   Thr Ala Thr Gly Ser Ser Pro Ser Pro Val Leu Gly Gln Gly Glu Lys
   900 905 910
   Val Glu Gly Leu Gln Ala Gln Ala Leu Tyr Pro Trp Arg Ala Lys Lys
   915 920 925
   Asp Asn His Leu Asn Phe Asn Lys Asn Asp Val Ile Thr Val Leu Glu
   930 935 940
   Gln Gln Asp Met Trp Trp Phe Gly Glu Val Gln Gly Gln Lys Gly Trp
   945 950 955 960
   Phe Pro Lys Ser Tyr Val Lys Leu Ile Ser Gly Pro Ile Arg Lys Ser
   965 970 975
   Thr Ser Met Asp Ser Gly Ser Ser Glu Ser Pro Ala Ser Leu Lys Arg
   980 985 990
   Val Ala Ser Pro Ala Ala Lys Pro Val Val Ser Gly Glu Glu Phe Ile
   995 1000 1005
   Ala Met Tyr Thr Tyr Glu Ser Ser Glu Gln Gly Asp Leu Thr Phe Gln
   1010 1015 1020
   Gln Gly Asp Val Ile Leu Val Thr Lys Lys Asp Gly Asp Trp Trp Thr
   1025 1030 1035 1040
   Gly Thr Val Gly Asp Lys Ala Gly Val Phe Pro Ser Asn Tyr Val Arg
   1045 1050 1055
   Leu Lys Asp Ser Glu Gly Ser Gly Thr Ala Gly Lys Thr Gly Ser Leu
   1060 1065 1070
   Gly Lys Lys Pro Glu Ile Ala Gln Val Ile Ala Ser Tyr Thr Ala Thr
   1075 1080 1085
   Gly Pro Glu Gln Leu Thr Leu Ala Pro Gly Gln Leu Ile Leu Ile Arg
   1090 1095 1100
   Lys Lys Asn Pro Gly Gly Trp Trp Glu Gly Glu Leu Gln Ala Arg Gly
   1105 1110 1115 1120
   Lys Lys Arg Gln Ile Gly Trp Phe Pro Ala Asn Tyr Val Lys Leu Leu
   1125 1130 1135
   Ser Pro Gly Thr Ser Lys Ile Thr Pro Thr Glu Pro Pro Lys Ser Thr
   1140 1145 1150
   Ala Leu Ala Ala Val Cys Gln Val Ile Gly Met Tyr Asp Tyr Thr Ala
   1155 1160 1165
   Gln Asn Asp Asp Glu Leu Ala Phe Asn Lys Gly Gln Ile Ile Asn Val
   1170 1175 1180
   Leu Asn Lys Glu Asp Pro Asp Trp Trp Lys Gly Glu Val Asn Gly Gln
   1185 1190 1195 1200
   Val Gly Leu Phe Pro Ser Asn Tyr Val Lys Leu Thr Thr Asp Met Asp
   1205 1210 1215
   Pro Ser Gln Gln
   1220
<210> 5
   <211> 23
   <212> PRT
   <213> Homo sapiens
<220>
   <223> From Seq ID 5 to ID 38, there are 34 pretein sequences translated from Seq ID No. 3. Together, they form the whole protein sequence.
<400> 5
   Thr Arg Gly Ser Glu Gly Gly Arg Glu Glu Trp Arg Arg Gln Gly Arg
    1               5                  10                  15
   Glu Arg Ser Leu Val Ala Pro
                20
<210> 6
   <211> 52
   <212> PRT
   <213> Homo sapiens
<400> 6
   Tyr Gly Gly Ser Arg Gly Arg Ile Pro Ser Gly Leu Arg Asp Gly Gln
    1               5                  10                  15
   Arg Gly Gly Arg Gly Trp Cys Ala Gly Leu Arg Leu Leu Arg Pro Ser
               20                  25                  30
   Gln Arg Arg Val Ser Gly Thr Asp Leu Ser Leu Gly Arg Gln Arg Gly
           35                  40                  45
   Pro Ala Arg Arg
       50
<210> 7
   <211> 3
   <212> PRT
   <213> Homo sapiens
<400> 7
   Gly Val Asp
    1
<210> 8
   <211> 1227
   <212> PRT
   <213> Homo sapiens
<400> 8
   Gln Gly Lys Ser Asn Arg Thr Met Ala Gln Phe Pro Thr Pro Phe Gly
    1               5                  10                  15
   Gly Ser Leu Asp Ile Trp Ala Ile Thr Val Glu Glu Arg Ala Lys His
               20                  25                  30
   Asp Gln Gln Phe His Ser Leu Lys Pro Ile Ser Gly Phe Ile Thr Gly
           35                  40                  45
   Asp Gln Ala Arg Asn Phe Phe Phe Gln Ser Gly Leu Pro Gln Pro Val
       50                  55                  60
   Leu Ala Gln Ile Trp Ala Leu Ala Asp Met Asn Asn Asp Gly Arg Met
   65                  70                  75                  80
   Asp Gln Val Glu Phe Ser Ile Ala Met Lys Leu Ile Lys Leu Lys Leu
                   85                  90                  95
   Gln Gly Tyr Gln Leu Pro Ser Ala Leu Pro Pro Val Met Lys Gln Gln
               100                 105                 110
   Pro Val Ala Ile Ser Ser Ala Pro Ala Phe Gly Met Gly Gly Ile Ala
           115                 120                 125
   Ser Met Pro Pro Leu Thr Ala Val Ala Pro Val Pro Met Gly Ser Ile
       130                 135                 140
   Pro Val Val Gly Met Ser Pro Thr Leu Val Ser Ser Val Pro Thr Ala
   145                 150                 155                 160
   Ala Val Pro Pro Leu Ala Asn Gly Ala Pro Pro Val Ile Gln Pro Leu
                   165                 170                 175
   Pro Ala Phe Ala His Pro Ala Ala Thr Leu Pro Lys Ser Ser Ser Phe
               180                 185                 190
   Ser Arg Ser Gly Pro Gly Ser Gln Leu Asn Thr Lys Leu Gln Lys Ala
           195                 200                 205
   Gln Ser Phe Asp Val Ala Ser Val Pro Pro Val Ala Glu Trp Ala Val
       210                 215                 220
   Pro Gln Ser Ser Arg Leu Lys Tyr Arg Gln Leu Phe Asn Ser His Asp
   225 230 235 240
   Lys Thr Met Ser Gly His Leu Thr Gly Pro Gln Ala Arg Thr Ile Leu
   245 250 255
   Met Gln Ser Ser Leu Pro Gln Ala Gln Leu Ala Ser Ile Trp Asn Leu
   260 265 270
   Ser Asp Ile Asp Gln Asp Gly Lys Leu Thr Ala Glu Glu Phe Ile Leu
   275 280 285
   Ala Met His Leu Ile Asp Val Ala Met Ser Gly Gln Pro Leu Pro Pro
   290 295 300
   Val Leu Pro Pro Glu Tyr Ile Pro Pro Ser Phe Arg Arg Val Arg Ser
   305 310 315 320
   Gly Ser Gly Ile Ser Val Ile Ser Ser Thr Ser Val Asp Gln Arg Leu
   325 330 335
   Pro Glu Glu Pro Val Leu Glu Asp Glu Gln Gln Gln Leu Glu Lys Lys
   340 345 350
   Leu Pro Val Thr Phe Glu Asp Lys Lys Arg Glu Asn Phe Glu Arg Gly
   355 360 365
   Asn Leu Glu Leu Glu Lys Arg Arg Gln Ala Leu Leu Glu Gln Gln Arg
   370 375 380
   Lys Glu Gln Glu Arg Leu Ala Gln Leu Glu Arg Ala Glu Gln Glu Arg
   385 390 395 400
   Lys Glu Arg Glu Arg Gln Glu Gln Glu Arg Lys Arg Gln Leu Glu Leu
   405 410 415
   Glu Lys Gln Leu Glu Lys Gln Arg Glu Leu Glu Arg Gln Arg Glu Glu
   420 425 430
   Glu Arg Arg Lys Glu Ile Glu Arg Arg Glu Ala Ala Lys Arg Glu Leu
   435 440 445
   Glu Arg Gln Arg Gln Leu Glu Trp Glu Arg Asn Arg Arg Gln Glu Leu
   450 455 460
   Leu Asn Gln Arg Asn Lys Glu Gln Glu Asp Ile Val Val Leu Lys Ala
   465 470 475 480
   Lys Lys Lys Thr Leu Glu Phe Glu Leu Glu Ala Leu Asn Asp Lys Lys
   485 490 495
   His Gln Leu Glu Gly Lys Leu Gln Asp Ile Arg Cys Arg Leu Thr Thr
   500 505 510
   Gln Arg Gln Glu Ile Glu Ser Thr Asn Lys Ser Arg Glu Leu Arg Ile
   515 520 525
   Ala Glu Ile Thr His Leu Gln Gln Gln Leu Gln Glu Ser Gln Gln Met
   530 535 540
   Leu Gly Arg Leu Ile Pro Glu Lys Gln Ile Leu Asn Asp Gln Leu Lys
   545 550 555 560
   Gln Val Gln Gln Asn Ser Leu His Arg Asp Ser Leu Val Thr Leu Lys
   565 570 575
   Arg Ala Leu Glu Ala Lys Glu Leu Ala Arg Gln His Leu Arg Asp Gln
   580 585 590
   Leu Asp Glu Val Glu Lys Glu Thr Arg Ser Lys Leu Gln Glu Ile Asp
   595 600 605
   Ile Phe Asn Asn Gln Leu Lys Glu Leu Arg Glu Ile His Asn Lys Gln
   610 615 620
   Gln Leu Gln Lys Gln Lys Ser Met Glu Ala Glu Arg Leu Lys Gln Lys
   625 630 635 640
   Glu Gln Glu Arg Lys Ile Ile Glu Leu Glu Lys Gln Lys Glu Glu Ala
   645 650 655
   Gln Arg Arg Ala Gln Glu Arg Asp Lys Gln Trp Leu Glu His Val Gln
   660 665 670
   Gln Glu Asp Glu His Gln Arg Pro Arg Lys Leu His Glu Glu Glu Lys
   675 680 685
   Leu Lys Arg Glu Glu Ser Val Lys Lys Lys Asp Gly Glu Glu Lys Gly
   690 695 700
   Lys Gln Glu Ala Gln Asp Lys Leu Gly Arg Leu Phe His Gln His Gln
   705 710 715 720
   Glu Pro Ala Lys Pro Ala Val Gln Ala Pro Trp Ser Thr Ala Glu Lys
   725 730 735
   Gly Pro Leu Thr Ile Ser Ala Gln Glu Asn Val Lys Val Val Tyr Tyr
   740 745 750
   Arg Ala Leu Tyr Pro Phe Glu Ser Arg Ser His Asp Glu Ile Thr Ile
   755 760 765
   Gln Pro Gly Asp Ile Val Met Val Lys Gly Glu Trp Val Asp Glu Ser
   770 775 780
   Gln Thr Gly Glu Pro Gly Trp Leu Gly Gly Glu Leu Lys Gly Lys Thr
   785 790 795 800
   Gly Trp Phe Pro Ala Asn Tyr Ala Glu Lys Ile Pro Glu Asn Glu Val
   805 810 815
   Pro Ala Pro Val Lys Pro Val Thr Asp Ser Thr Ser Ala Pro Ala Pro
   820 825 830
   Lys Leu Ala Leu Arg Glu Thr Pro Ala Pro Leu Ala Val Thr Ser Ser
   835 840 845
   Glu Pro Ser Thr Thr Pro Asn Asn Trp Ala Asp Phe Ser Ser Thr Trp
   850 855 860
   Pro Thr Ser Thr Asn Glu Lys Pro Glu Thr Asp Asn Trp Asp Ala Trp
   865 870 875 880
   Ala Ala Gln Pro Ser Leu Thr Val Pro Ser Ala Gly Gln Leu Arg Gln
   885 890 895
   Arg Ser Ala Phe Thr Pro Ala Thr Ala Thr Gly Ser Ser Pro Ser Pro
   900 905 910
   Val Leu Gly Gln Gly Glu Lys Val Glu Gly Leu Gln Ala Gln Ala Leu
   915 920 925
   Tyr Pro Trp Arg Ala Lys Lys Asp Asn His Leu Asn Phe Asn Lys Asn
   930 935 940
   Asp Val Ile Thr Val Leu Glu Gln Gln Asp Met Trp Trp Phe Gly Glu
   945 950 955 960
   Val Gln Gly Gln Lys Gly Trp Phe Pro Lys Ser Tyr Val Lys Leu Ile
   965 970 975
   Ser Gly Pro Ile Arg Lys Ser Thr Ser Met Asp Ser Gly Ser Ser Glu
   980 985 990
   Ser Pro Ala Ser Leu Lys Arg Val Ala Ser Pro Ala Ala Lys Pro Val
   995 1000 1005
   Val Ser Gly Glu Glu Phe Ile Ala Met Tyr Thr Tyr Glu Ser Ser Glu
   1010 1015 1020
   Gln Gly Asp Leu Thr Phe Gln Gln Gly Asp Val Ile Leu Val Thr Lys
   1025 1030 1035 1040
   Lys Asp Gly Asp Trp Trp Thr Gly Thr Val Gly Asp Lys Ala Gly Val
   1045 1050 1055
   Phe Pro Ser Asn Tyr Val Arg Leu Lys Asp Ser Glu Gly Ser Gly Thr
   1060 1065 1070
   Ala Gly Lys Thr Gly Ser Leu Gly Lys Lys Pro Glu Ile Ala Gln Val
   1075 1080 1085
   Ile Ala Ser Tyr Thr Ala Thr Gly Pro Glu Gln Leu Thr Leu Ala Pro
   1090 1095 1100
   Gly Gln Leu Ile Leu Ile Arg Lys Lys Asn Pro Gly Gly Trp Trp Glu
   1105 1110 1115 1120
   Gly Glu Leu Gln Ala Arg Gly Lys Lys Arg Gln Ile Gly Trp Phe Pro
   1125 1130 1135
   Ala Asn Tyr Val Lys Leu Leu Ser Pro Gly Thr Ser Lys Ile Thr Pro
   1140 1145 1150
   Thr Glu Pro Pro Lys Ser Thr Ala Leu Ala Ala Val Cys Gln Val Ile
   1155 1160 1165
   Gly Met Tyr Asp Tyr Thr Ala Gln Asn Asp Asp Glu Leu Ala Phe Asn
   1170 1175 1180
   Lys Gly Gln Ile Ile Asn Val Leu Asn Lys Glu Asp Pro Asp Trp Trp
   1185 1190 1195 1200
   Lys Gly Glu Val Asn Gly Gln Val Gly Leu Phe Pro Ser Asn Tyr Val
   1205 1210 1215
   Lys Leu Thr Thr Asp Met Asp Pro Ser Gln Gln
   1220 1225
<210> 9
   <211> 10
   <212> PRT
   <213> Homo sapiens
<400> 9
   Ile Ile Cys Cys Pro Ser Pro Pro Gln Ala
   1 5 10
<210> 10
   <211> 15
   <212> PRT
   <213> Homo sapiens
<400> 10
   Lys Ser Ser Lys Arg Pro Thr Ile Pro Tyr His Cys Pro Glu Gly
   1 5 10 15
<210> 11
   <211> 5
   <212> PRT
   <213> Homo sapiens
<400> 11
   Trp Glu Met Gln Pro
   1 5
<210> 12
   <211> 13
   <212> PRT
   <213> Homo sapiens
<400> 12
   Ser Cys Asp Phe Gln His Asp His Leu Leu Pro Ser Glu
   1 5 10
<210> 13
   <211> 20
   <212> PRT
   <213> Homo sapiens
<400> 13
   Lys Asn Ser Leu Gln Ser Ser Leu Pro His Phe Thr Leu Val Ala Cys
   1 5 10 15
   Asp Arg Asn Val
<210> 14
   <211> 28
   <212> PRT
   <213> Homo sapiens
<400> 14
   Val Ile Thr Cys Arg Asp Arg Ser Lys Asn Tyr Lys Asn Thr Gln Gly
   1 5 10 15
   Ser Gly Ser Phe Cys Gly Phe Pro Ser Tyr Ser Asn
   20 25
<210> 15
   <211> 30
   <212> PRT
   <213> Homo sapiens
<400> 15
   Leu Ser Pro Thr Phe Ala Gln Val Leu Ser Ile Val Leu Lys Leu Phe
   1 5 10 15
   Leu Asn Ile Tyr Phe Ser Phe Leu Ile Asn Lys Ile Asn Lys
   20 25 30
<210> 16
   <211> 20
   <212> PRT
   <213> Homo sapiens
<400> 16
   Leu Leu Cys Tyr Phe Gly Phe Ala Lys Arg Pro Thr Ile Lys Glu Cys
   1 5 10 15
   Cys Met Cys Tyr
   20
<210> 17
   <211> 34
   <212> PRT
   <213> Homo sapiens
<400> 17
   Lys Leu Phe Gln Met Ser Ile Asn Leu Arg Leu Asp Val Phe Phe His
   1 5 10 15
   Phe Val Gln Cys Tyr Gln Leu Asn Cys Ala Val Trp Gly Phe Ser Pro
   20 25 30
   Leu Pro
<210> 18
   <211> 13
   <212> PRT
   <213> Homo sapiens
<400> 18
   Lys Cys Arg Gly Val Gln Tyr Leu Cys Phe Lys Asp Val
   1 5 10
<210> 19
   <211> 4
   <212> PRT
   <213> Homo sapiens
<400> 19
   Asn Glu Pro Asn
   1
<210> 20
   <211> 15
   <212> PRT
   <213> Homo sapiens
<400> 20
   Ser Glu Gly Val Cys Ala Cys Leu Cys Val Ser Ala Val Pro Cys
   1 5 10 15
<210> 21
   <211> 7
   <212> PRT
   <213> Homo sapiens
<400> 21
   Ala Cys Asn Thr Ser Cys Thr
   1 5
<210> 22
   <211> 29
   <212> PRT
   <213> Homo sapiens
<400> 22
   Glu Ile Ser Ser Phe His Gly Lys Ala Ile Thr Leu Tyr Asp Ala Leu
   1 5 10 15
   Ile Ile Leu His Leu Ile Leu Phe Cys Thr Val Thr Leu
   20 25
<210> 23
   <211> 33
   <212> PRT
   <213> Homo sapiens
<400> 23
   Pro His Glu Lys Ala Leu Cys Val Phe Val Arg Ser Gln Ile Tyr Leu
   1 5 10 15
   Val Glu Leu Val Phe Cys Leu Gly Phe Leu Ile Leu Arg Val Cys Ile
   20 25 30
   Ala
<210> 24
   <211> 2
   <212> PRT
   <213> Homo sapiens
<400> 24
   Asn Gln
   1
<210> 25
   <211> 16
   <212> PRT
   <213> Homo sapiens
<400> 25
   Thr Thr Pro Leu Arg Ser Leu Arg Ser Thr Ile Ser Thr Val Ser Phe
   1 5 10 15
<210> 26
   <211> 14
   <212> PRT
   <213> Homo sapiens
<400> 26
   Ser Leu Leu His Glu Val Leu Phe Gln Leu Leu Phe Met Glu
   1 5 10
<210> 27
   <211> 5
   <212> PRT
   <213> Homo sapiens
<400> 27
   Pro Ile Leu Asn Lys
   1 5
<210> 28
   <211> 2
   <212> PRT
   <213> Homo sapiens
<400> 28
   Phe Ser
   1
<210> 29
   <211> 29
   <212> PRT
   <213> Homo sapiens
<400> 29
   Gln Glu Arg Met Tyr Arg Ser Leu Pro Ala Ile Asn Phe Gln Cys Leu
   1 5 10 15
   His Phe Leu Thr Arg Leu Trp Asn Phe Tyr Arg Leu Ile
   20 25
<210> 30
   <211> 9
   <212> PRT
   <213> Homo sapiens
<400> 30
   Asn Gly Ala His Gly Pro Phe Val Cys
   1 5
<210> 31
   <211> 4
   <212> PRT
   <213> Homo sapiens
<400> 31
   Ile Cys Cys Ser
   1
<210> 32
   <211> 33
   <212> PRT
   <213> Homo sapiens
<400> 32
   Ser Pro Val Cys Leu Leu Asn Thr Ser Trp Lys Leu Ser Ile Lys Met
    1               5                  10                  15
   Pro Ala Ala His Ser Thr Glu Asn Gly Ala Gly Gly Ala Ser Ser Thr
               20                  25                  30
   Ile
<210> 33
   <211> 3
   <212> PRT
   <213> Homo sapiens
<400> 33
   Leu Ser Ser
   1
<210> 34
   <211> 50
   <212> PRT
   <213> Homo sapiens
<400> 34
   Arg Leu Cys Asn Ala His Ser Pro Arg Val Leu Pro Ala Leu Ser Gly
    1               5                  10                  15
   Gly Cys Ala Gly Gly Arg Val Glu Val Leu Leu Leu Ser His Gly Ala
               20                  25                  30
   Glu Ser Glu Asp Leu Ser Ser Ser Phe Ser Cys Thr Ser Val Phe Ser
           35                  40                  45
   Arg Ile
       50
<210> 35
   <211> 1
   <212> PRT
   <213> Homo sapiens
<400> 35
   Met
   1
<210> 36
   <211> 2
   <212> PRT
   <213> Homo sapiens
<400> 36
   Asn Ile
   1
<210> 37
   <211> 22
   <212> PRT
   <213> Homo sapiens
<400> 37
   Ile Tyr Lys Pro Ala Ala Leu Thr Thr Val Ile Gln Pro Phe Glu Leu
    1               5                  10                  15
   Val Pro Cys Ile Asp Asn
               20
<210> 38
   <211> 12
   <212> PRT
   <213> Homo sapiens
<400> 38
   Ile Leu His Thr Lys Val Lys Lys Lys Lys Lys Lys
    1               5                  10
<210> 39
   <211> 5195
   <212> DNA
   <213> Homo sapiens
<400> 39
   agagtggagg cgccagggga gggagcgtag cttggttgct ccgtagtacg gcggctcgcg 60
   aggaagaatc ccgagcgggc tccgggacgg acagagaggc gggcggggat ggtgtgcggg 120
   gctgcggctc ctgcgtccct cccagcggcg cgtgagcggc actgatttgt ccctggggcg 180
   gcagcgcgga cccgcccgga gatgaggcgt cgattagcaa ggtaaaagta acagaaccat 240
   ggctcagttt ccaacacctt ttggtggcag cctggatatc tgggccataa ctgtagagga 300
   aagagcgaag catgatcagc agttccatag tttaaagcca atatctggat tcattactgg 360
   tgatcaagct agaaactttt tttttcaatc tgggttacct caacctgttt tagcacagat 420
   atgggcacta gctgacatga ataatgatgg aagaatggat caagtggagt tttccatagc 480
   tatgaaactt atcaaactga agctacaagg atatcagcta ccctctgcac ttccccctgt 540
   catgaaacag caaccagttg ctatttctag cgcaccagca tttggtatgg gaggtatcgc 600
   cagcatgcca ccgcttacag ctgttgctcc agtgccaatg ggatccattc cagttgttgg 660
   aatgtctcca accctagtat cttctgttcc cacagcagct gtgccccccc tggctaacgg 720
   ggctccccct gttatacaac ctctgcctgc atttgctcat cctgcagcca cattgccaaa 780
   gagttcttcc tttagtagat ctggtccagg gtcacaacta aacactaaat tacaaaaggc 840
   acagtcattt gatgtggcca gtgtcccacc agtggcagag tgggctgttc ctcagtcatc 900
   aagactgaaa tacaggcaat tattcaatag tcatgacaaa actatgagtg gacacttaac 960
   aggtccccaa gcaagaacta ttcttatgca gtcaagttta ccacaggctc agctggcttc 1020
   aatatggaat ctttctgaca ttgatcaaga tggaaaactt acagcagagg aatttatcct 1080
   ggcaatgcac ctcattgatg tagctatgtc tggccaacca ctgccacctg tcctgcctcc 1140
   agaatacatt ccaccttctt ttagaagagt tcgatctggc agtggtatat ctgtcataag 1200
   ctcaacatct gtagatcaga ggctaccaga ggaaccagtt ttagaagatg aacaacaaca 1260
   attagaaaag aaattacctg taacgtttga agataagaag cgggagaact ttgaacgtgg 1320
   caacctggaa ctggagaaac gaaggcaagc tctcctggaa cagcagcgca aggagcagga 1380
   gcgcctggcc cagctggagc gggcggagca ggagaggaag gagcgtgagc gccaggagca 1440
   agagcgcaaa agacaactgg aactggagaa gcaactggaa aagcagcggg agctagaacg 1500
   gcagagagag gaggagagga ggaaagaaat tgagaggcga gaggctgcaa aacgggaact 1560
   tgaaaggcaa cgacaacttg agtgggaacg gaatcgaagg caagaactac taaatcaaag 1620
   aaacaaagaa caagaggaca tagttgtact gaaagcaaag aaaaagactt tggaatttga 1680
   attagaagct ctaaatgata aaaagcatca actagaaggg aaacttcaag atatcagatg 1740
   tcgattgacc acccaaaggc aagaaattga gagcacaaac aaatctagag agttgagaat 1800
   tgccgaaatc acccatctac agcaacaatt acaggaatct cagcaaatgc ttggaagact 1860
   tattccagaa aaacagatac tcaatgacca attaaaacaa gttcagcaga acagtttgca 1920
   cagagattca cttgttacac ttaaaagagc cttagaagca aaagaactag ctcggcagca 1980
   cctacgagac caactggatg aagtggagaa agaaactaga tcaaaactac aggagattga 2040
   tattttcaat aatcagctga aggaactaag agaaatacac aataagcaac aactccagaa 2100
   gcaaaagtcc atggaggctg aacgactgaa acagaaagaa caagaacgaa agatcataga 2160
   attagaaaaa caaaaagaag aagcccaaag acgagctcag gaaagggaca agcagtggct 2220
   ggagcatgtg cagcaggagg acgagcatca gagaccaaga aaactccacg aagaggaaaa 2280
   actgaaaagg gaggagagtg tcaaaaagaa ggatggcgag gaaaaaggca aacaggaagc 2340
   acaagacaag ctgggtcggc ttttccatca acaccaagaa ccagctaagc cagctgtcca 2400
   ggcaccctgg tccactgcag aaaaaggtcc acttaccatt tctgcacagg aaaatgtaaa 2460
   agtggtgtat taccgggcac tgtacccctt tgaatccaga agccatgatg aaatcactat 2520
   ccagccagga gacatagtca tggtggatga aagccaaact ggagaacccg gctggcttgg 2580
   aggagaatta aaaggaaaga cagggtggtt ccctgcaaac tatgcagaga aaatcccaga 2640
   aaatgaggtt cccgctccag tgaaaccagt gactgattca acatctgccc ctgcccccaa 2700
   actggccttg cgtgagaccc ccgccccttt ggcagtaacc tcttcagagc cctccacgac 2760
   ccctaataac tgggccgact tcagctccac gtggcccacc agcacgaatg agaaaccaga 2820
   aacggataac tgggatgcat gggcagccca gccctctctc accgttccaa gtgccggcca 2880
   gttaaggcag aggtccgcct ttactccagc cacggccact ggctcctccc cgtctcctgt 2940
   gctaggccag ggtgaaaagg tggaggggct acaagctcaa gccctatatc cttggagagc 3000
   caaaaaagac aaccacttaa attttaacaa aaatgatgtc atcaccgtcc tggaacagca 3060
   agacatgtgg tggtttggag aagttcaagg tcagaagggt tggttcccca agtcttacgt 3120
   gaaactcatt tcagggccca taaggaagtc tacaagcatg gattctggtt cttcagagag 3180
   tcctgctagt ctaaagcgag tagcctctcc agcagccaag ccggtcgttt cgggagaaga 3240
   atttattgcc atgtacactt acgagagttc tgagcaagga gatttaacct ttcagcaagg 3300
   ggatgtgatt ttggttacca agaaagatgg tgactggtgg acaggaacag tgggcgacaa 3360
   ggccggagtc ttcccttcta actatgtgag gcttaaagat tcagagggct ctggaactgc 3420
   tgggaaaaca gggagtttag gaaaaaaacc tgaaattgcc caggttattg cctcatacac 3480
   cgccaccggc cccgagcagc tcactctcgc ccctggtcag ctgattttga tccgaaaaaa 3540
   gaacccaggt ggatggtggg aaggagagct gcaagcacgt gggaaaaagc gccagatagg 3600
   ctggttccca gctaattatg taaagcttct aagccctggg acgagcaaaa tcactccaac 3660
   agagccacct aagtcaacag cattagcggc agtgtgccag gtgattggga tgtacgacta 3720
   caccgcgcag aatgacgatg agctggcctt caacaagggc cagatcatca acgtcctcaa 3780
   caaggaggac cctgactggt ggaaaggaga agtcaatgga caagtggggc tcttcccatc 3840
   caattatgtg aagctgacca cagacatgga cccaagccag caatgaatca tatgttgtcc 3900
   atccccccct caggcttgaa agtccttttg tggctttcct agttactcaa attgactttc 3960
   ccccaccttt gcacaggtgc tttcaatagt tttaaaatta tttttaaata tatattttag 4020
   ctttttaata aacaaaataa ataaatgact tctttgctat tttggttttg caaaaagacc 4080
   cactatcaag gaatgctgca tgtgctatta aaaattgttc caaatgtcca taaatctgag 4140
   acttgatgta ttttttcatt ttgtccagtg ttaccaacta aattgtgcag tttggggctt 4200
   ttccccctta ccatagaagt gcagaggagt tcagtatctc tgttttaaag acgtatagaa 4260
   tgagcccaat taaagcgaag gtgtttgtgc ttgtttgtgt gtatcagctg taccttgttg 4320
   agcatgtaat acatcctgta cataagaaat tagttctttc catggcaaag ctattacctt 4380
   gtacgatgct ctaatcatat tgcatttaat tttattttgc acagtgacct tgtagccaca 4440
   tgagaaagca ctctgtgttt ttgttcggtc tcagatttat ctggttgagt tggtgttttg 4500
   tttggggttt ttaattttgc gtgtttgcat agcataaaat cagtagacaa caccactgag 4560
   gtcgttacga tcaacgatat ccacagtctc tttttagtct ctgttacatg aagttttatt 4620
   ccagttactt ttcatggaat gacctatttt gaacaagtaa ttttcttgac aagaaagaat 4680
   gtatagaagt ctccctgcaa ttaatttcca atgtttacat tttttaacta gactgtggaa 4740
   tttctacaga ttaatatgaa atggagctca tggtccgttt gtgtgttaga tatgctgtag 4800
   ctgaagccct gtttgtcttt taaacactag ttggaagctc tcaataaaaa tgcctgctgc 4860
   tcacagcaca gaaaatgggg cagggggagc ctcaagcaca atctagctgt cctcctaaag 4920
   actctgtaat gctcactccc ctcgcgttct cccggcgctg tcgggaggct gtgctggtgg 4980
   tcgtgtagag gtccttctcc tttcacatgg tgcagagagc gaggacctct cctcctcgtt 5040
   cagttgcact tcagtatttt cacggatatg aatgtaaaat atataaatat ataaacctgc 5100
   ggctttaaca actgtaatac aaccttttga attagttccg tgtatagata attaaattct 5160
   tcatacaaaa gttaaaaaaa aaaaaaaaaa aaaaa                             5195
<210> 40
   <211> 1215
   <212> PRT
   <213> Homo sapiens
<400> 40
   Met Ala Gln Phe Pro Thr Pro Phe Gly Gly Ser Leu Asp Ile Trp Ala
   1               5                   10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Ala Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
       130                 135                 140
   Thr Leu Val Ser Ser Val Pro Thr Ala Ala Val Pro Pro Leu Ala Asn
   145                 150                 155                 160
   Gly Ala Pro Pro Val Ile Gln Pro Leu Pro Ala Phe Ala His Pro Ala
                   165                 170                 175
   Ala Thr Leu Pro Lys Ser Ser Ser Phe Ser Arg Ser Gly Pro Gly Ser
   180 185 190
   Gln Leu Asn Thr Lys Leu Gln Lys Ala Gln Ser Phe Asp Val Ala Ser
   195 200 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385 390 395 400
   Gln Glu Arg Lys Arg Gln Leu Glu Leu Glu Lys Gln Leu Glu Lys Gln
   405 410 415
   Arg Glu Leu Glu Arg Gln Arg Glu Glu Glu Arg Arg Lys Glu Ile Glu
   420 425 430
   Arg Arg Glu Ala Ala Lys Arg Glu Leu Glu Arg Gln Arg Gln Leu Glu
   435 440 445
   Trp Glu Arg Asn Arg Arg Gln Glu Leu Leu Asn Gln Arg Asn Lys Glu
   450 455 460
   Gln Glu Asp Ile Val Val Leu Lys Ala Lys Lys Lys Thr Leu Glu Phe
   465 470 475 480
   Glu Leu Glu Ala Leu Asn Asp Lys Lys His Gln Leu Glu Gly Lys Leu
   485 490 495
   Gln Asp Ile Arg Cys Arg Leu Thr Thr Gln Arg Gln Glu Ile Glu Ser
   500 505 510
   Thr Asn Lys Ser Arg Glu Leu Arg Ile Ala Glu Ile Thr His Leu Gln
   515 520 525
   Gln Gln Leu Gln Glu Ser Gln Gln Met Leu Gly Arg Leu Ile Pro Glu
   530 535 540
   Lys Gln Ile Leu Asn Asp Gln Leu Lys Gln Val Gln Gln Asn Ser Leu
   545 550 555 560
   His Arg Asp Ser Leu Val Thr Leu Lys Arg Ala Leu Glu Ala Lys Glu
   565 570 575
   Leu Ala Arg Gln His Leu Arg Asp Gln Leu Asp Glu Val Glu Lys Glu
   580 585 590
   Thr Arg Ser Lys Leu Gln Glu Ile Asp Ile Phe Asn Asn Gln Leu Lys
   595 600 605
   Glu Leu Arg Glu Ile His Asn Lys Gln Gln Leu Gln Lys Gln Lys Ser
   610 615 620
   Met Glu Ala Glu Arg Leu Lys Gln Lys Glu Gln Glu Arg Lys Ile Ile
   625 630 635 640
   Glu Leu Glu Lys Gln Lys Glu Glu Ala Gln Arg Arg Ala Gln Glu Arg
   645 650 655
   Asp Lys Gln Trp Leu Glu His Val Gln Gln Glu Asp Glu His Gln Arg
   660 665 670
   Pro Arg Lys Leu His Glu Glu Glu Lys Leu Lys Arg Glu Glu Ser Val
   675 680 685
   Lys Lys Lys Asp Gly Glu Glu Lys Gly Lys Gln Glu Ala Gln Asp Lys
   690 695 700
   Leu Gly Arg Leu Phe His Gln His Gln Glu Pro Ala Lys Pro Ala Val
   705 710 715 720
   Gln Ala Pro Trp Ser Thr Ala Glu Lys Gly Pro Leu Thr Ile Ser Ala
   725 730 735
   Gln Glu Asn Val Lys Val Val Tyr Tyr Arg Ala Leu Tyr Pro Phe Glu
   740 745 750
   Ser Arg Ser His Asp Glu Ile Thr Ile Gln Pro Gly Asp Ile Val Met
   755 760 765
   Val Asp Glu Ser Gln Thr Gly Glu Pro Gly Trp Leu Gly Gly Glu Leu
   770 775 780
   Lys Gly Lys Thr Gly Trp Phe Pro Ala Asn Tyr Ala Glu Lys Ile Pro
   785 790 795 800
   Glu Asn Glu Val Pro Ala Pro Val Lys Pro Val Thr Asp Ser Thr Ser
   805 810 815
   Ala Pro Ala Pro Lys Leu Ala Leu Arg Glu Thr Pro Ala Pro Leu Ala
   820 825 830
   Val Thr Ser Ser Glu Pro Ser Thr Thr Pro Asn Asn Trp Ala Asp Phe
   835 840 845
   Ser Ser Thr Trp Pro Thr Ser Thr Asn Glu Lys Pro Glu Thr Asp Asn
   850 855 860
   Trp Asp Ala Trp Ala Ala Gln Pro Ser Leu Thr Val Pro Ser Ala Gly
   865 870 875 880
   Gln Leu Arg Gln Arg Ser Ala Phe Thr Pro Ala Thr Ala Thr Gly Ser
   885 890 895
   Ser Pro Ser Pro Val Leu Gly Gln Gly Glu Lys Val Glu Gly Leu Gln
   900 905 910
   Ala Gln Ala Leu Tyr Pro Trp Arg Ala Lys Lys Asp Asn His Leu Asn
   915 920 925
   Phe Asn Lys Asn Asp Val Ile Thr Val Leu Glu Gln Gln Asp Met Trp
   930 935 940
   Trp Phe Gly Glu Val Gln Gly Gln Lys Gly Trp Phe Pro Lys Ser Tyr
   945 950 955 960
   Val Lys Leu Ile Ser Gly Pro Ile Arg Lys Ser Thr Ser Met Asp Ser
   965 970 975
   Gly Ser Ser Glu Ser Pro Ala Ser Leu Lys Arg Val Ala Ser Pro Ala
   980 985 990
   Ala Lys Pro Val Val Ser Gly Glu Glu Phe Ile Ala Met Tyr Thr Tyr
   995 1000 1005
   Glu Ser Ser Glu Gln Gly Asp Leu Thr Phe Gln Gln Gly Asp Val Ile
   1010 1015 1020
   Leu Val Thr Lys Lys Asp Gly Asp Trp Trp Thr Gly Thr Val Gly Asp
   1025 1030 1035 1040
   Lys Ala Gly Val Phe Pro Ser Asn Tyr Val Arg Leu Lys Asp Ser Glu
   1045 1050 1055
   Gly Ser Gly Thr Ala Gly Lys Thr Gly Ser Leu Gly Lys Lys Pro Glu
   1060 1065 1070
   Ile Ala Gln Val Ile Ala Ser Tyr Thr Ala Thr Gly Pro Glu Gln Leu
   1075 1080 1085
   Thr Leu Ala Pro Gly Gln Leu Ile Leu Ile Arg Lys Lys Asn Pro Gly
   1090 1095 1100
   Gly Trp Trp Glu Gly Glu Leu Gln Ala Arg Gly Lys Lys Arg Gln Ile
   1105 1110 1115 1120
   Gly Trp Phe Pro Ala Asn Tyr Val Lys Leu Leu Ser Pro Gly Thr Ser
   1125 1130 1135
   Lys Ile Thr Pro Thr Glu Pro Pro Lys Ser Thr Ala Leu Ala Ala Val
   1140 1145 1150
   Cys Gln Val Ile Gly Met Tyr Asp Tyr Thr Ala Gln Asn Asp Asp Glu
   1155 1160 1165
   Leu Ala Phe Asn Lys Gly Gln Ile Ile Asn Val Leu Asn Lys Glu Asp
   1170 1175 1180
   Pro Asp Trp Trp Lys Gly Glu Val Asn Gly Gln Val Gly Leu Phe Pro
   1185 1190 1195 1200
   Ser Asn Tyr Val Lys Leu Thr Thr Asp Met Asp Pro Ser Gln Gln
   1205 1210 1215
<210> 41
   <211> 14
   <212> PRT
   <213> Homo sapiens
<220>
   <223> From Seq ID 41 to ID 70, there are 30 pretein sequences translated from Seq ID No. 6. Together, they form the whole protein sequence.
<400> 41
   Glu Trp Arg Arg Gln Gly Arg Glu Arg Ser Leu Val Ala Pro
   1               5                   10
<210> 42
   <211> 52
   <212> PRT
   <213> Homo sapiens
<400> 42
   Tyr Gly Gly Ser Arg Gly Arg Ile Pro Ser Gly Leu Arg Asp Gly Gln
   1               5                   10                  15
   Arg Gly Gly Arg Gly Trp Cys Ala Gly Leu Arg Leu Leu Arg Pro Ser
               20                  25                  30
   Gln Arg Arg Val Ser Gly Thr Asp Leu Ser Leu Gly Arg Gln Arg Gly
           35                  40                  45
   Pro Ala Arg Arg
       50
<210> 43
   <211> 3
   <212> PRT
   <213> Homo sapiens
<400> 43
   Gly Val Asp
   1
<210> 44
   <211> 1222
   <212> PRT
   <213> Homo sapiens
<400> 44
   Gln Gly Lys Ser Asn Arg Thr Met Ala Gln Phe Pro Thr Pro Phe Gly
   1 5 10 15
   Gly Ser Leu Asp Ile Trp Ala Ile Thr Val Glu Glu Arg Ala Lys His
   20 25 30
   Asp Gln Gln Phe His Ser Leu Lys Pro Ile Ser Gly Phe Ile Thr Gly
   35 40 45
   Asp Gln Ala Arg Asn Phe Phe Phe Gln Ser Gly Leu Pro Gln Pro Val
   50 55 60
   Leu Ala Gln Ile Trp Ala Leu Ala Asp Met Asn Asn Asp Gly Arg Met
   65 70 75 80
   Asp Gln Val Glu Phe Ser Ile Ala Met Lys Leu Ile Lys Leu Lys Leu
   85 90 95
   Gln Gly Tyr Gln Leu Pro Ser Ala Leu Pro Pro Val Met Lys Gln Gln
   100 105 110
   Pro Val Ala Ile Ser Ser Ala Pro Ala Phe Gly Met Gly Gly Ile Ala
   115 120 125
   Ser Met Pro Pro Leu Thr Ala Val Ala Pro Val Pro Met Gly Ser Ile
   130 135 140
   Pro Val Val Gly Met Ser Pro Thr Leu Val Ser Ser Val Pro Thr Ala
   145 150 155 160
   Ala Val Pro Pro Leu Ala Asn Gly Ala Pro Pro Val Ile Gln Pro Leu
   165 170 175
   Pro Ala Phe Ala His Pro Ala Ala Thr Leu Pro Lys Ser Ser Ser Phe
   180 185 190
   Ser Arg Ser Gly Pro Gly Ser Gln Leu Asn Thr Lys Leu Gln Lys Ala
   195 200 205
   Gln Ser Phe Asp Val Ala Ser Val Pro Pro Val Ala Glu Trp Ala Val
   210 215 220
   Pro Gln Ser Ser Arg Leu Lys Tyr Arg Gln Leu Phe Asn Ser His Asp
   225 230 235 240
   Lys Thr Met Ser Gly His Leu Thr Gly Pro Gln Ala Arg Thr Ile Leu
   245 250 255
   Met Gln Ser Ser Leu Pro Gln Ala Gln Leu Ala Ser Ile Trp Asn Leu
   260 265 270
   Ser Asp Ile Asp Gln Asp Gly Lys Leu Thr Ala Glu Glu Phe Ile Leu
   275 280 285
   Ala Met His Leu Ile Asp Val Ala Met Ser Gly Gln Pro Leu Pro Pro
   290 295 300
   Val Leu Pro Pro Glu Tyr Ile Pro Pro Ser Phe Arg Arg Val Arg Ser
   305 310 315 320
   Gly Ser Gly Ile Ser Val Ile Ser Ser Thr Ser Val Asp Gln Arg Leu
   325 330 335
   Pro Glu Glu Pro Val Leu Glu Asp Glu Gln Gln Gln Leu Glu Lys Lys
   340 345 350
   Leu Pro Val Thr Phe Glu Asp Lys Lys Arg Glu Asn Phe Glu Arg Gly
   355 360 365
   Asn Leu Glu Leu Glu Lys Arg Arg Gln Ala Leu Leu Glu Gln Gln Arg
   370 375 380
   Lys Glu Gln Glu Arg Leu Ala Gln Leu Glu Arg Ala Glu Gln Glu Arg
   385 390 395 400
   Lys Glu Arg Glu Arg Gln Glu Gln Glu Arg Lys Arg Gln Leu Glu Leu
   405 410 415
   Glu Lys Gln Leu Glu Lys Gln Arg Glu Leu Glu Arg Gln Arg Glu Glu
   420 425 430
   Glu Arg Arg Lys Glu Ile Glu Arg Arg Glu Ala Ala Lys Arg Glu Leu
   435 440 445
   Glu Arg Gln Arg Gln Leu Glu Trp Glu Arg Asn Arg Arg Gln Glu Leu
   450 455 460
   Leu Asn Gln Arg Asn Lys Glu Gln Glu Asp Ile Val Val Leu Lys Ala
   465 470 475 480
   Lys Lys Lys Thr Leu Glu Phe Glu Leu Glu Ala Leu Asn Asp Lys Lys
               485                 490                 495
   His Gln Leu Glu Gly Lys Leu Gln Asp Ile Arg Cys Arg Leu Thr Thr
                   500                 505                 510
   Gln Arg Gln Glu Ile Glu Ser Thr Asn Lys Ser Arg Glu Leu Arg Ile
               515                 520                 525
   Ala Glu Ile Thr His Leu Gln Gln Gln Leu Gln Glu Ser Gln Gln Met
       530                 535                 540
   Leu Gly Arg Leu Ile Pro Glu Lys Gln Ile Leu Asn Asp Gln Leu Lys
   545                 550                 555                 560
   Gln Val Gln Gln Asn Ser Leu His Arg Asp Ser Leu Val Thr Leu Lys
                   565                 570                 575
   Arg Ala Leu Glu Ala Lys Glu Leu Ala Arg Gln His Leu Arg Asp Gln
                   580                 585                 590
   Leu Asp Glu Val Glu Lys Glu Thr Arg Ser Lys Leu Gln Glu Ile Asp
               595                 600                 605
   Ile Phe Asn Asn Gln Leu Lys Glu Leu Arg Glu Ile His Asn Lys Gln
       610                 615                 620
   Gln Leu Gln Lys Gln Lys Ser Met Glu Ala Glu Arg Leu Lys Gln Lys
   625                 630                 635                 640
   Glu Gln Glu Arg Lys Ile Ile Glu Leu Glu Lys Gln Lys Glu Glu Ala
                   645                 650                 655
   Gln Arg Arg Ala Gln Glu Arg Asp Lys Gln Trp Leu Glu His Val Gln
                   660                 665                 670
   Gln Glu Asp Glu His Gln Arg Pro Arg Lys Leu His Glu Glu Glu Lys
               675                 680                 685
   Leu Lys Arg Glu Glu Ser Val Lys Lys Lys Asp Gly Glu Glu Lys Gly
       690                 695                 700
   Lys Gln Glu Ala Gln Asp Lys Leu Gly Arg Leu Phe His Gln His Gln
   705                 710                 715                 720
   Glu Pro Ala Lys Pro Ala Val Gln Ala Pro Trp Ser Thr Ala Glu Lys
                   725                 730                 735
   Gly Pro Leu Thr Ile Ser Ala Gln Glu Asn Val Lys Val Val Tyr Tyr
   740 745 750
   Arg Ala Leu Tyr Pro Phe Glu Ser Arg Ser His Asp Glu Ile Thr Ile
   755 760 765
   Gln Pro Gly Asp Ile Val Met Val Asp Glu Ser Gln Thr Gly Glu Pro
   770 775 780
   Gly Trp Leu Gly Gly Glu Leu Lys Gly Lys Thr Gly Trp Phe Pro Ala
   785 790 795 800
   Asn Tyr Ala Glu Lys Ile Pro Glu Asn Glu Val Pro Ala Pro Val Lys
   805 810 815
   Pro Val Thr Asp Ser Thr Ser Ala Pro Ala Pro Lys Leu Ala Leu Arg
   820 825 830
   Glu Thr Pro Ala Pro Leu Ala Val Thr Ser Ser Glu Pro Ser Thr Thr
   835 840 845
   Pro Asn Asn Trp Ala Asp Phe Ser Ser Thr Trp Pro Thr Ser Thr Asn
   850 855 860
   Glu Lys Pro Glu Thr Asp Asn Trp Asp Ala Trp Ala Ala Gln Pro Ser
   865 870 875 880
   Leu Thr Val Pro Ser Ala Gly Gln Leu Arg Gln Arg Ser Ala Phe Thr
   885 890 895
   Pro Ala Thr Ala Thr Gly Ser Ser Pro Ser Pro Val Leu Gly Gln Gly
   900 905 910
   Glu Lys Val Glu Gly Leu Gln Ala Gln Ala Leu Tyr Pro Trp Arg Ala
   915 920 925
   Lys Lys Asp Asn His Leu Asn Phe Asn Lys Asn Asp Val Ile Thr Val
   930 935 940
   Leu Glu Gln Gln Asp Met Trp Trp Phe Gly Glu Val Gln Gly Gln Lys
   945 950 955 960
   Gly Trp Phe Pro Lys Ser Tyr Val Lys Leu Ile Ser Gly Pro Ile Arg
   965 970 975
   Lys Ser Thr Ser Met Asp Ser Gly Ser Ser Glu Ser Pro Ala Ser Leu
   980 985 990
   Lys Arg Val Ala Ser Pro Ala Ala Lys Pro Val Val Ser Gly Glu Glu
   995 1000 1005
   Phe Ile Ala Met Tyr Thr Tyr Glu Ser Ser Glu Gln Gly Asp Leu Thr
   1010 1015 1020
   Phe Gln Gln Gly Asp Val Ile Leu Val Thr Lys Lys Asp Gly Asp Trp
   1025 1030 1035 1040
   Trp Thr Gly Thr Val Gly Asp Lys Ala Gly Val Phe Pro Ser Asn Tyr
   1045 1050 1055
   Val Arg Leu Lys Asp Ser Glu Gly Ser Gly Thr Ala Gly Lys Thr Gly
   1060 1065 1070
   Ser Leu Gly Lys Lys Pro Glu Ile Ala Gln Val Ile Ala Ser Tyr Thr
   1075 1080 1085
   Ala Thr Gly Pro Glu Gln Leu Thr Leu Ala Pro Gly Gln Leu Ile Leu
   1090 1095 1100
   Ile Arg Lys Lys Asn Pro Gly Gly Trp Trp Glu Gly Glu Leu Gln Ala
   1105 1110 1115 1120
   Arg Gly Lys Lys Arg Gln Ile Gly Trp Phe Pro Ala Asn Tyr Val Lys
   1125 1130 1135
   Leu Leu Ser Pro Gly Thr Ser Lys Ile Thr Pro Thr Glu Pro Pro Lys
   1140 1145 1150
   Ser Thr Ala Leu Ala Ala Val Cys Gln Val Ile Gly Met Tyr Asp Tyr
   1155 1160 1165
   Thr Ala Gln Asn Asp Asp Glu Leu Ala Phe Asn Lys Gly Gln Ile Ile
   1170 1175 1180
   Asn Val Leu Asn Lys Glu Asp Pro Asp Trp Trp Lys Gly Glu Val Asn
   1185 1190 1195 1200
   Gly Gln Val Gly Leu Phe Pro Ser Asn Tyr Val Lys Leu Thr Thr Asp
   1205 1210 1215
   Met Asp Pro Ser Gln Gln
   1220
<210> 45
   <211> 10
   <212> PRT
   <213> Homo sapiens
<400> 45
   Ile Ile Cys Cys Pro Ser Pro Pro Gln Ala
   1 5 10
<210> 46
   <211> 11
   <212> PRT
   <213> Homo sapiens
<400> 46
   Lys Ser Phe Cys Gly Phe Pro Ser Tyr Ser Asn
   1 5 10
<210> 47
   <211> 30
   <212> PRT
   <213> Homo sapiens
<400> 47
   Leu Ser Pro Thr Phe Ala Gln Val Leu Ser Ile Val Leu Lys Leu Phe
   1 5 10 15
   Leu Asn Ile Tyr Phe Ser Phe Leu Ile Asn Lys Ile Asn Lys
   20 25 30
<210> 48
   <211> 20
   <212> PRT
   <213> Homo sapiens
<400> 48
   Leu Leu Cys Tyr Phe Gly Phe Ala Lys Arg Pro Thr Ile Lys Glu Cys
   1 5 10 15
   Cys Met Cys Tyr
   20
<210> 49
   <211> 34
   <212> PRT
   <213> Homo sapiens
<400> 49
   Lys Leu Phe Gln Met Ser Ile Asn Leu Arg Leu Asp Val Phe Phe His
   1 5 10 15
   Phe Val Gln Cys Tyr Gln Leu Asn Cys Ala Val Trp Gly Phe Ser Pro
   20 25 30
   Leu Pro
<210> 50
   <211> 13
   <212> PRT
   <213> Homo sapiens
<400> 50
   Lys Cys Arg Gly Val Gln Tyr Leu Cys Phe Lys Asp Val
   1 5 10
<210> 51
   <211> 4
   <212> PRT
   <213> Homo sapiens
<400> 51
   Asn Glu Pro Asn
   1
<210> 52
   <211> 15
   <212> PRT
   <213> Homo sapiens
<400> 52
   Ser Glu Gly Val Cys Ala Cys Leu Cys Val Ser Ala Val Pro Cys
   1 5 10 15
<210> 53
   <211> 7
   <212> PRT
   <213> Homo sapiens
<400> 53
   Ala Cys Asn Thr Ser Cys Thr
   1 5
<210> 54
   <211> 29
   <212> PRT
   <213> Homo sapiens
<400> 54
   Glu Ile Ser Ser Phe His Gly Lys Ala Ile Thr Leu Tyr Asp Ala Leu
    1               5                  10                  15
   Ile Ile Leu His Leu Ile Leu Phe Cys Thr Val Thr Leu
               20                  25
<210> 55
   <211> 33
   <212> PRT
   <213> Homo sapiens
<400> 55
   Pro His Glu Lys Ala Leu Cys Val Phe Val Arg Ser Gln Ile Tyr Leu
    1               5                  10                  15
   Val Glu Leu Val Phe Cys Leu Gly Phe Leu Ile Leu Arg Val Cys Ile
               20                  25                  30
   Ala
<210> 56
   <211> 2
   <212> PRT
   <213> Homo sapiens
<400> 56
   Asn Gln
    1
<210> 57
   <211> 16
   <212> PRT
   <213> Homo sapiens
<400> 57
   Thr Thr Pro Leu Arg Ser Leu Arg Ser Thr Ile Ser Thr Val Ser Phe
    1               5                  10                  15
<210> 58
   <211> 14
   <212> PRT
   <213> Homo sapiens
<400> 58
   Ser Leu Leu His Glu Val Leu Phe Gln Leu Leu Phe Met Glu
   1 5 10
<210> 59
   <211> 5
   <212> PRT
   <213> Homo sapiens
<400> 59
   Pro Ile Leu Asn Lys
   1 5
<210> 60
   <211> 2
   <212> PRT
   <213> Homo sapiens
<400> 60
   Phe Ser
   1
<210> 61
   <211> 29
   <212> PRT
   <213> Homo sapiens
<400> 61
   Gln Glu Arg Met Tyr Arg Ser Leu Pro Ala Ile Asn Phe Gln Cys Leu
   1 5 10 15
   His Phe Leu Thr Arg Leu Trp Asn Phe Tyr Arg Leu Ile
   20 25
<210> 62
   <211> 9
   <212> PRT
   <213> Homo sapiens
<400> 62
   Asn Gly Ala His Gly Pro Phe Val Cys
   1 5
<210> 63
   <211> 4
   <212> PRT
   <213> Homo sapiens
<400> 63
   Ile Cys Cys Ser
   1
<210> 64
   <211> 33
   <212> PRT
   <213> Homo sapiens
<400> 64
   Ser Pro Val Cys Leu Leu Asn Thr Ser Trp Lys Leu Ser Ile Lys Met
   1 5 10 15
   Pro Ala Ala His Ser Thr Glu Asn Gly Ala Gly Gly Ala Ser Ser Thr
   20 25 30
   Ile
<210> 65
   <211> 3
   <212> PRT
   <213> Homo sapiens
<400> 65
   Leu Ser Ser
   1
<210> 66
   <211> 50
   <212> PRT
   <213> Homo sapiens
<400> 66
   Arg Leu Cys Asn Ala His Ser Pro Arg Val Leu Pro Ala Leu Ser Gly
   1 5 10 15
   Gly Cys Ala Gly Gly Arg Val Glu Val Leu Leu Leu Ser His Gly Ala
   20 25 30
   Glu Ser Glu Asp Leu Ser Ser Ser Phe Ser Cys Thr Ser Val Phe Ser
   35 40 45
   Arg Ile
   50
<210> 67
   <211> 1
   <212> PRT
   <213> Homo sapiens
<400> 67
   Met
   1
<210> 68
   <211> 2
   <212> PRT
   <213> Homo sapiens
<400> 68
   Asn Ile
   1
<210> 69
   <211> 22
   <212> PRT
   <213> Homo sapiens
<400> 69
   Ile Tyr Lys Pro Ala Ala Leu Thr Thr Val Ile Gln Pro Phe Glu Leu
   1 5 10 15
   Val Pro Cys Ile Asp Asn
   20
<210> 70
   <211> 13
   <212> PRT
   <213> Homo sapiens
<400> 70
   Ile Leu His Thr Lys Val Lys Lys Lys Lys Lys Lys Lys
   1 5 10
<210> 71
   <211> 2079
   <212> DNA
   <213> Homo sapiens
<400> 71
   cggggatggt gtgcggggct gcggctcctg cgtccctccc agcggcgcgt gagcggcact 60
   gatttgtccc tggggcggca gcgcggaccc gcccggagat gaggcgtcga ttagcaaggt 120
   aaaagtaaca gaaccatggc tcagtttcca acaccttttg gtggcagcct ggatatctgg 180
   gccataactg tagaggaaag agcgaagcat gatcagcagt tccatagttt aaagccaata 240
   tctggattca ttactggtga tcaagctaga aacttttttt ttcaatctgg gttacctcaa 300
   cctgttttag cacagatatg ggcactagct gacatgaata atgatggaag aatggatcaa 360
   gtggagtttt ccatagctat gaaacttatc aaactgaagc tacaaggata tcagctaccc 420
   tctgcacttc cccctgtcat gaaacagcaa ccagttgcta tttctagcgc accagcattt 480
   ggtatgggag gtatcgccag catgccaccg cttacagctg ttgctccagt gccaatggga 540
   tccattccag ttgttggaat gtctccaacc ctagtatctt ctgttcccac agcagctgtg 600
   ccccccctgg ctaacggggc tccccctgtt atacaacctc tgcctgcatt tgctcatcct 660
   gcagccacat tgccaaagag ttcttccttt agtagatctg gtccagggtc acaactaaac 720
   actaaattac aaaaggcaca gtcatttgat gtggccagtg tcccaccagt ggcagagtgg 780
   gctgttcctc agtcatcaag actgaaatac aggcaattat tcaatagtca tgacaaaact 840
   atgagtggac acttaacagg tccccaagca agaactattc ttatgcagtc aagtttacca 900
   caggctcagc tggcttcaat atggaatctt tctgacattg atcaagatgg aaaacttaca 960
   gcagaggaat ttatcctggc aatgcacctc attgatgtag ctatgtctgg ccaaccactg 1020
   ccacctgtcc tgcctccaga atacattcca ccttctttta gaagagttcg atctggcagt 1080
   ggtatatctg tcataagctc aacatctgta gatcagaggc taccagagga accagtttta 1140
   gaagatgaac aacaacaatt agaaaagaaa ttacctgtaa cgtttgaaga taagaagcgg 1200
   gagaactttg aacgtggcaa cctggaactg gagaaacgaa ggcaagctct cctggaacag 1260
   cagcgcaagg agcaggagcg cctggcccag ctggagcggg cggagcagga gaggaaggag 1320
   cgtgagcgcc aggagcaaga gcgcaaaaga caactggaac tggagaagca actggaaaag 1380
   cagcgggagc tagaacggca gagagaggag gagaggagga aagaaattga gaggcgagag 1440
   gctgcaaaac gggaacttga aaggcaacga caacttgagt gggaacggaa tcgaaggcaa 1500
   gaactactaa atcaaagaaa caaagaacaa gaggacatag ttgtactgaa agcaaagaaa 1560
   aagactttgg aatttgaatt agaagctcta aatgataaaa agcatcaact agaagggaaa 1620
   cttcaagata tcagatgtcg attgaccacc caaaggcaag aaattgagag cacaaacaaa 1680
   tctagagagt tgagaattgc cgaaatcacc catctacagc aacaattaca ggaatctcag 1740
   caaatgcttg gaagacttat tccagaaaaa cagatactca atgaccaatt aaaacaagtt 1800
   cagcagaaca gtttgcacag agattcactt gttacactta aaagagcctt agaagcaaaa 1860
   gaactagctc ggcagcacct acgagaccaa ctggatgaag tggagaaaga aactagatca 1920
   aaactacagg agattgatat tttcaataat cagctgaagg aactaagaga aatacacaat 1980
   aagcaacaac tccagaagca aaagtccatg gaggctgaac gactgaaaca gaaagaacaa 2040
   gaacgaaaga tcatagaatt agaaaaaaaa aaaaaaaaa 2079
<210> 72
   <211> 648
   <212> PRT
   <213> Homo sapiens
<400> 72
   Met Ala Gln Phe Pro Thr Pro Phe Gly Gly Ser Leu Asp Ile Trp Ala
    1               5                  10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Ala Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
       130                 135                 140
   Thr Leu Val Ser Ser Val Pro Thr Ala Ala Val Pro Pro Leu Ala Asn
   145                 150                 155                 160
   Gly Ala Pro Pro Val Ile Gln Pro Leu Pro Ala Phe Ala His Pro Ala
                   165                 170                 175
   Ala Thr Leu Pro Lys Ser Ser Ser Phe Ser Arg Ser Gly Pro Gly Ser
               180                 185                 190
   Gln Leu Asn Thr Lys Leu Gln Lys Ala Gln Ser Phe Asp Val Ala Ser
   195 200 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385 390 395 400
   Gln Glu Arg Lys Arg Gln Leu Glu Leu Glu Lys Gln Leu Glu Lys Gln
   405 410 415
   Arg Glu Leu Glu Arg Gln Arg Glu Glu Glu Arg Arg Lys Glu Ile Glu
   420 425 430
   Arg Arg Glu Ala Ala Lys Arg Glu Leu Glu Arg Gln Arg Gln Leu Glu
   435 440 445
   Trp Glu Arg Asn Arg Arg Gln Glu Leu Leu Asn Gln Arg Asn Lys Glu
                   450                 455                 460
   Gln Glu Asp Ile Val Val Leu Lys Ala Lys Lys Lys Thr Leu Glu Phe
   465                 470                 475                 480
   Glu Leu Glu Ala Leu Asn Asp Lys Lys His Gln Leu Glu Gly Lys Leu
                   485                 490                 495
   Gln Asp Ile Arg Cys Arg Leu Thr Thr Gln Arg Gln Glu Ile Glu Ser
               500                 505                 510
   Thr Asn Lys Ser Arg Glu Leu Arg Ile Ala Glu Ile Thr His Leu Gln
           515                 520                 525
   Gln Gln Leu Gln Glu Ser Gln Gln Met Leu Gly Arg Leu Ile Pro Glu
       530                 535                 540
   Lys Gln Ile Leu Asn Asp Gln Leu Lys Gln Val Gln Gln Asn Ser Leu
   545                 550                 555                 560
   His Arg Asp Ser Leu Val Thr Leu Lys Arg Ala Leu Glu Ala Lys Glu
                   565                 570                 575
   Leu Ala Arg Gln His Leu Arg Asp Gln Leu Asp Glu Val Glu Lys Glu
               580                 585                 590
   Thr Arg Ser Lys Leu Gln Glu Ile Asp Ile Phe Asn Asn Gln Leu Lys
           595                 600                 605
   Glu Leu Arg Glu Ile His Asn Lys Gln Gln Leu Gln Lys Gln Lys Ser
       610                 615                 620
   Met Glu Ala Glu Arg Leu Lys Gln Lys Glu Gln Glu Arg Lys Ile Ile
   625                 630                 635                 640
   Glu Leu Glu Lys Lys Lys Lys Lys
                   645
<210> 73
   <211> 33
   <212> PRT
   <213> Homo sapiens
<220>
   <223> From Seq ID 73 to ID 75, there are 3 pretein sequences translated from Seq ID No. 71. Together, they form the whole protein sequence.
<400> 73
   Arg Gly Trp Cys Ala Gly Leu Arg Leu Leu Arg Pro Ser Gln Arg Arg
   1               5                   10                  15
   Val Ser Gly Thr Asp Leu Ser Leu Gly Arg Gln Arg Gly Pro Ala Arg
               20                  25                  30
   Arg
<210> 74
   <211> 3
   <212> PRT
   <213> Homo sapiens
<400> 74
   Gly Val Asp
   1
<210> 75
   <211> 655
   <212> PRT
   <213> Homo sapiens
<400> 75
   Gln Gly Lys Ser Asn Arg Thr Met Ala Gln Phe Pro Thr Pro Phe Gly
   1               5                   10                  15
   Gly Ser Leu Asp Ile Trp Ala Ile Thr Val Glu Glu Arg Ala Lys His
               20                  25                  30
   Asp Gln Gln Phe His Ser Leu Lys Pro Ile Ser Gly Phe Ile Thr Gly
           35                  40                  45
   Asp Gln Ala Arg Asn Phe Phe Phe Gln Ser Gly Leu Pro Gln Pro Val
       50                  55                  60
   Leu Ala Gln Ile Trp Ala Leu Ala Asp Met Asn Asn Asp Gly Arg Met
   65                  70                  75                  80
   Asp Gln Val Glu Phe Ser Ile Ala Met Lys Leu Ile Lys Leu Lys Leu
                   85                  90                  95
   Gln Gly Tyr Gln Leu Pro Ser Ala Leu Pro Pro Val Met Lys Gln Gln
               100                 105                 110
   Pro Val Ala Ile Ser Ser Ala Pro Ala Phe Gly Met Gly Gly Ile Ala
           115                 120                 125
   Ser Met Pro Pro Leu Thr Ala Val Ala Pro Val Pro Met Gly Ser Ile
       130                 135                 140
   Pro Val Val Gly Met Ser Pro Thr Leu Val Ser Ser Val Pro Thr Ala
   145                 150                 155                 160
   Ala Val Pro Pro Leu Ala Asn Gly Ala Pro Pro Val Ile Gln Pro Leu
                   165                 170                 175
   Pro Ala Phe Ala His Pro Ala Ala Thr Leu Pro Lys Ser Ser Ser Phe
               180                 185                 190
   Ser Arg Ser Gly Pro Gly Ser Gln Leu Asn Thr Lys Leu Gln Lys Ala
           195                 200                 205
   Gln Ser Phe Asp Val Ala Ser Val Pro Pro Val Ala Glu Trp Ala Val
       210                 215                 220
   Pro Gln Ser Ser Arg Leu Lys Tyr Arg Gln Leu Phe Asn Ser His Asp
   225                 230                 235                 240
   Lys Thr Met Ser Gly His Leu Thr Gly Pro Gln Ala Arg Thr Ile Leu
                   245                 250                 255
   Met Gln Ser Ser Leu Pro Gln Ala Gln Leu Ala Ser Ile Trp Asn Leu
               260                 265                 270
   Ser Asp Ile Asp Gln Asp Gly Lys Leu Thr Ala Glu Glu Phe Ile Leu
           275                 280                 285
   Ala Met His Leu Ile Asp Val Ala Met Ser Gly Gln Pro Leu Pro Pro
       290                 295                 300
   Val Leu Pro Pro Glu Tyr Ile Pro Pro Ser Phe Arg Arg Val Arg Ser
   305                 310                 315                 320
   Gly Ser Gly Ile Ser Val Ile Ser Ser Thr Ser Val Asp Gln Arg Leu
                   325                 330                 335
   Pro Glu Glu Pro Val Leu Glu Asp Glu Gln Gln Gln Leu Glu Lys Lys
               340                 345                 350
   Leu Pro Val Thr Phe Glu Asp Lys Lys Arg Glu Asn Phe Glu Arg Gly
           355                 360                 365
   Asn Leu Glu Leu Glu Lys Arg Arg Gln Ala Leu Leu Glu Gln Gln Arg
   370 375 380
   Lys Glu Gln Glu Arg Leu Ala Gln Leu Glu Arg Ala Glu Gln Glu Arg
   385 390 395 400
   Lys Glu Arg Glu Arg Gln Glu Gln Glu Arg Lys Arg Gln Leu Glu Leu
   405 410 415
   Glu Lys Gln Leu Glu Lys Gln Arg Glu Leu Glu Arg Gln Arg Glu Glu
   420 425 430
   Glu Arg Arg Lys Glu Ile Glu Arg Arg Glu Ala Ala Lys Arg Glu Leu
   435 440 445
   Glu Arg Gln Arg Gln Leu Glu Trp Glu Arg Asn Arg Arg Gln Glu Leu
   450 455 460
   Leu Asn Gln Arg Asn Lys Glu Gln Glu Asp Ile Val Val Leu Lys Ala
   465 470 475 480
   Lys Lys Lys Thr Leu Glu Phe Glu Leu Glu Ala Leu Asn Asp Lys Lys
   485 490 495
   His Gln Leu Glu Gly Lys Leu Gln Asp Ile Arg Cys Arg Leu Thr Thr
   500 505 510
   Gln Arg Gln Glu Ile Glu Ser Thr Asn Lys Ser Arg Glu Leu Arg Ile
   515 520 525
   Ala Glu Ile Thr His Leu Gln Gln Gln Leu Gln Glu Ser Gln Gln Met
   530 535 540
   Leu Gly Arg Leu Ile Pro Glu Lys Gln Ile Leu Asn Asp Gln Leu Lys
   545 550 555 560
   Gln Val Gln Gln Asn Ser Leu His Arg Asp Ser Leu Val Thr Leu Lys
   565 570 575
   Arg Ala Leu Glu Ala Lys Glu Leu Ala Arg Gln His Leu Arg Asp Gln
   580 585 590
   Leu Asp Glu Val Glu Lys Glu Thr Arg Ser Lys Leu Gln Glu Ile Asp
   595 600 605
   Ile Phe Asn Asn Gln Leu Lys Glu Leu Arg Glu Ile His Asn Lys Gln
   610 615 620
   Gln Leu Gln Lys Gln Lys Ser Met Glu Ala Glu Arg Leu Lys Gln Lys
   625 630 635 640
   Glu Gln Glu Arg Lys Ile Ile Glu Leu Glu Lys Lys Lys Lys Lys
   645 650 655
<210> 76
   <211> 3231
   <212> DNA
   <213> Homo sapiens
<400> 76
   gaccacccaa aggcaagaaa ttgagagcac aaacaaatct agagagttga gaattgccga 60
   aatcacccat ctacagcaac aattacagga atctcagcaa atgcttggaa gacttattcc 120
   agaaaaacag atactcaatg accaattaaa acaagttcag cagaacagtt tgcacagaga 180
   ttcacttgtt acacttaaaa gagccttaga agcaaaagaa ctagctcggc agcacctacg 240
   agaccaactg gatgaagtgg agaaagaaac tagatcaaaa ctacaggaga ttgatatttt 300
   caataatcag ctgaaggaac taagagaaat acacaataag caacaactcc agaagcaaaa 360
   gtccatggag gctgaacgac tgaaacagaa agaacaagaa cgaaagatca tagaattaga 420
   aaaacaaaaa gaagaagccc aaagacgagc tcaggaaagg gacaagcagt ggctggagca 480
   tgtgcagcag gaggacgagc atcagagacc aagaaaactc cacgaagagg aaaaactgaa 540
   aagggaggag agtgtcaaaa agaaggatgg cgaggaaaaa ggcaaacagg aagcacaaga 600
   caagctgggt cggcttttcc atcaacacca agaaccagct aagccagctg tccaggcacc 660
   ctggtccact gcagaaaaag gtccacttac catttctgca caggaaaatg taaaagtggt 720
   gtattaccgg gcactgtacc cctttgaatc cagaagccat gatgaaatca ctatccagcc 780
   aggagacata gtcatggtgg atgaaagcca aactggagaa cccggctggc ttggaggaga 840
   attaaaagga aagacagggt ggttccctgc aaactatgca gagaaaatcc cagaaaatga 900
   ggttcccgct ccagtgaaac cagtgactga ttcaacatct gcccctgccc ccaaactggc 960
   cttgcgtgag acccccgccc ctttggcagt aacctcttca gagccctcca cgacccctaa 1020
   taactgggcc gacttcagct ccacgtggcc caccagcacg aatgagaaac cagaaacgga 1080
   taactgggat gcatgggcag cccagccctc tctcaccgtt ccaagtgccg gccagttaag 1140
   gcagaggtcc gcctttactc cagccacggc cactggctcc tccccgtctc ctgtgctagg 1200
   ccagggtgaa aaggtggagg ggctacaagc tcaagcccta tatccttgga gagccaaaaa 1260
   agacaaccac ttaaatttta acaaaaatga tgtcatcacc gtcctggaac agcaagacat 1320
   gtggtggttt ggagaagttc aaggtcagaa gggttggttc cccaagtctt acgtgaaact 1380
   catttcaggg cccataagga agtctacaag catggattct ggttcttcag agagtcctgc 1440
   tagtctaaag cgagtagcct ctccagcagc caagccggtc gtttcgggag aagaaattgc 1500
   ccaggttatt gcctcataca ccgccaccgg ccccgagcag ctcactctcg cccctggtca 1560
   gctgattttg atccgaaaaa agaacccagg tggatggtgg gaaggagagc tgcaagcacg 1620
   tgggaaaaag cgccagatag gctggttccc agctaattat gtaaagcttc taagccctgg 1680
   gacgagcaaa atcactccaa cagagccacc taagtcaaca gcattagcgg cagtgtgcca 1740
   ggtgattggg atgtacgact acaccgcgca gaatgacgat gagctggcct tcaacaaggg 1800
   ccagatcatc aacgtcctca acaaggagga ccctgactgg tggaaaggag aagtcaatgg 1860
   acaagtgggg ctcttcccat ccaattatgt gaagctgacc acagacatgg acccaagcca 1920
   gcaatgaatc atatgttgtc catccccccc tcaggcttga aagtcctttt gtggctttcc 1980
   tagttactca aattgacttt cccccacctt tgcacaggtg ctttcaatag ttttaaaatt 2040
   atttttaaat atatatttta gctttttaat aaacaaaata aataaatgac ttctttgcta 2100
   ttttggtttt gcaaaaagac ccactatcaa ggaatgctgc atgtgctatt aaaaattgtt 2160
   ccaaatgtcc ataaatctga gacttgatgt attttttcat tttgtccagt gttaccaact 2220
   aaattgtgca gtttggggct tttccccctt accatagaag tgcagaggag ttcagtatct 2280
   ctgttttaaa gacgtataga atgagcccaa ttaaagcgaa ggtgtttgtg cttgtttgtg 2340
   tgtatcagct gtaccttgtt gagcatgtaa tacatcctgt acataagaaa ttagttcttt 2400
   ccatggcaaa gctattacct tgtacgatgc tctaatcata ttgcatttaa ttttattttg 2460
   cacagtgacc ttgtagccac atgagaaagc actctgtgtt tttgttcggt ctcagattta 2520
   tctggttgag ttggtgtttt gtttggggtt tttaattttg cgtgtttgca tagcataaaa 2580
   tcagtagaca acaccactga ggtcgttacg atcaacgata tccacagtct ctttttagtc 2640
   tctgttacat gaagttttat tccagttact tttcatggaa tgacctattt tgaacaagta 2700
   attttcttga caagaaagaa tgtatagaag tctccctgca attaatttcc aatgtttaca 2760
   ttttttaact agactgtgga atttctacag attaatatga aatggagctc atggtccgtt 2820
   tgtgtgttag atatgctgta gctgaagccc tgtttgtctt ttaaacacta gttggaagct 2880
   ctcaataaaa atgcctgctg ctcacagcac agaaaatggg gcagggggag cctcaagcac 2940
   aatctagctg tcctcctaaa gactctgtaa tgctcactcc cctcgcgttc tcccggcgct 3000
   gtcgggaggc tgtgctggtg gtcgtgtaag gtccttctcc tttcacatgg tgcagagagc 3060
   gaggacctct cctcctcgtt cagttgcact tcagtatttt cacggatatg aatgtaaaat 3120
   atataaatat ataaacctgc ggctttaaca actgtaatac aaccttttga attagttccg 3180
   tgtatagata attaaattct tcatacaaaa gttaaaaaaa aaaaaaaaaa a          3231
<210> 77
   <211> 641
   <212> PRT
   <213> Homo sapiens
<400> 77
   Thr Thr Gln Arg Gln Glu Ile Glu Ser Thr Asn Lys Ser Arg Glu Leu
   1               5                   10                  15
   Arg Ile Ala Glu Ile Thr His Leu Gln Gln Gln Leu Gln Glu Ser Gln
               20                  25                  30
   Gln Met Leu Gly Arg Leu Ile Pro Glu Lys Gln Ile Leu Asn Asp Gln
           35                  40                  45
   Leu Lys Gln Val Gln Gln Asn Ser Leu His Arg Asp Ser Leu Val Thr
       50                  55                  60
   Leu Lys Arg Ala Leu Glu Ala Lys Glu Leu Ala Arg Gln His Leu Arg
   65                  70                  75                  80
   Asp Gln Leu Asp Glu Val Glu Lys Glu Thr Arg Ser Lys Leu Gln Glu
                   85                  90                  95
   Ile Asp Ile Phe Asn Asn Gln Leu Lys Glu Leu Arg Glu Ile His Asn
               100                 105                 110
   Lys Gln Gln Leu Gln Lys Gln Lys Ser Met Glu Ala Glu Arg Leu Lys
           115                 120                 125
   Gln Lys Glu Gln Glu Arg Lys Ile Ile Glu Leu Glu Lys Gln Lys Glu
       130                 135                 140
   Glu Ala Gln Arg Arg Ala Gln Glu Arg Asp Lys Gln Trp Leu Glu His
   145                 150                 155                 160
   Val Gln Gln Glu Asp Glu His Gln Arg Pro Arg Lys Leu His Glu Glu
                   165                 170                 175
   Glu Lys Leu Lys Arg Glu Glu Ser Val Lys Lys Lys Asp Gly Glu Glu
               180                 185                 190
   Lys Gly Lys Gln Glu Ala Gln Asp Lys Leu Gly Arg Leu Phe His Gln
           195                 200                 205
   His Gln Glu Pro Ala Lys Pro Ala Val Gln Ala Pro Trp Ser Thr Ala
       210                 215                 220
   Glu Lys Gly Pro Leu Thr Ile Ser Ala Gln Glu Asn Val Lys Val Val
   225                 230                 235                 240
   Tyr Tyr Arg Ala Leu Tyr Pro Phe Glu Ser Arg Ser His Asp Glu Ile
                   245                 250                 255
   Thr Ile Gln Pro Gly Asp Ile Val Met Val Asp Glu Ser Gln Thr Gly
               260                 265                 270
   Glu Pro Gly Trp Leu Gly Gly Glu Leu Lys Gly Lys Thr Gly Trp Phe
           275                 280                 285
   Pro Ala Asn Tyr Ala Glu Lys Ile Pro Glu Asn Glu Val Pro Ala Pro
       290                 295                 300
   Val Lys Pro Val Thr Asp Ser Thr Ser Ala Pro Ala Pro Lys Leu Ala
   305                 310                 315                 320
   Leu Arg Glu Thr Pro Ala Pro Leu Ala Val Thr Ser Ser Glu Pro Ser
                   325                 330                 335
   Thr Thr Pro Asn Asn Trp Ala Asp Phe Ser Ser Thr Trp Pro Thr Ser
               340                 345                 350
   Thr Asn Glu Lys Pro Glu Thr Asp Asn Trp Asp Ala Trp Ala Ala Gln
           355                 360                 365
   Pro Ser Leu Thr Val Pro Ser Ala Gly Gln Leu Arg Gln Arg Ser Ala
       370                 375                 380
   Phe Thr Pro Ala Thr Ala Thr Gly Ser Ser Pro Ser Pro Val Leu Gly
   385                 390                 395                 400
   Gln Gly Glu Lys Val Glu Gly Leu Gln Ala Gln Ala Leu Tyr Pro Trp
                   405                 410                 415
   Arg Ala Lys Lys Asp Asn His Leu Asn Phe Asn Lys Asn Asp Val Ile
               420                 425                 430
   Thr Val Leu Glu Gln Gln Asp Met Trp Trp Phe Gly Glu Val Gln Gly
           435                 440                 445
   Gln Lys Gly Trp Phe Pro Lys Ser Tyr Val Lys Leu Ile Ser Gly Pro
       450                 455                 460
   Ile Arg Lys Ser Thr Ser Met Asp Ser Gly Ser Ser Glu Ser Pro Ala
   465                 470                 475                 480
   Ser Leu Lys Arg Val Ala Ser Pro Ala Ala Lys Pro Val Val Ser Gly
                   485                 490                 495
   Glu Glu Ile Ala Gln Val Ile Ala Ser Tyr Thr Ala Thr Gly Pro Glu
               500                 505                 510
   Gln Leu Thr Leu Ala Pro Gly Gln Leu Ile Leu Ile Arg Lys Lys Asn
           515                 520                 525
   Pro Gly Gly Trp Trp Glu Gly Glu Leu Gln Ala Arg Gly Lys Lys Arg
       530                 535                 540
   Gln Ile Gly Trp Phe Pro Ala Asn Tyr Val Lys Leu Leu Ser Pro Gly
   545                 550                 555                 560
   Thr Ser Lys Ile Thr Pro Thr Glu Pro Pro Lys Ser Thr Ala Leu Ala
                   565                 570                 575
   Ala Val Cys Gln Val Ile Gly Met Tyr Asp Tyr Thr Ala Gln Asn Asp
               580                 585                 590
   Asp Glu Leu Ala Phe Asn Lys Gly Gln Ile Ile Asn Val Leu Asn Lys
           595                 600                 605
   Glu Asp Pro Asp Trp Trp Lys Gly Glu Val Asn Gly Gln Val Gly Leu
       610                 615                 620
   Phe Pro Ser Asn Tyr Val Lys Leu Thr Thr Asp Met Asp Pro Ser Gln
   625 630 635 640
   Gln
<210> 78
   <211> 641
   <212> PRT
   <213> Homo sapiens
<400> 78
   Thr Thr Gln Arg Gln Glu Ile Glu Ser Thr Asn Lys Ser Arg Glu Leu
   1 5 10 15
   Arg Ile Ala Glu Ile Thr His Leu Gln Gln Gln Leu Gln Glu Ser Gln
   20 25 30
   Gln Met Leu Gly Arg Leu Ile Pro Glu Lys Gln Ile Leu Asn Asp Gln
   35 40 45
   Leu Lys Gln Val Gln Gln Asn Ser Leu His Arg Asp Ser Leu Val Thr
   50 55 60
   Leu Lys Arg Ala Leu Glu Ala Lys Glu Leu Ala Arg Gln His Leu Arg
   65 70 75 80
   Asp Gln Leu Asp Glu Val Glu Lys Glu Thr Arg Ser Lys Leu Gln Glu
   85 90 95
   Ile Asp Ile Phe Asn Asn Gln Leu Lys Glu Leu Arg Glu Ile His Asn
   100 105 110
   Lys Gln Gln Leu Gln Lys Gln Lys Ser Met Glu Ala Glu Arg Leu Lys
   115 120 125
   Gln Lys Glu Gln Glu Arg Lys Ile Ile Glu Leu Glu Lys Gln Lys Glu
   130 135 140
   Glu Ala Gln Arg Arg Ala Gln Glu Arg Asp Lys Gln Trp Leu Glu His
   145 150 155 160
   Val Gln Gln Glu Asp Glu His Gln Arg Pro Arg Lys Leu His Glu Glu
   165 170 175
   Glu Lys Leu Lys Arg Glu Glu Ser Val Lys Lys Lys Asp Gly Glu Glu
   180 185 190
   Lys Gly Lys Gln Glu Ala Gln Asp Lys Leu Gly Arg Leu Phe His Gln
   195 200 205
   His Gln Glu Pro Ala Lys Pro Ala Val Gln Ala Pro Trp Ser Thr Ala
   210 215 220
   Glu Lys Gly Pro Leu Thr Ile Ser Ala Gln Glu Asn Val Lys Val Val
   225 230 235 240
   Tyr Tyr Arg Ala Leu Tyr Pro Phe Glu Ser Arg Ser His Asp Glu Ile
   245 250 255
   Thr Ile Gln Pro Gly Asp Ile Val Met Val Asp Glu Ser Gln Thr Gly
   260 265 270
   Glu Pro Gly Trp Leu Gly Gly Glu Leu Lys Gly Lys Thr Gly Trp Phe
   275 280 285
   Pro Ala Asn Tyr Ala Glu Lys Ile Pro Glu Asn Glu Val Pro Ala Pro
   290 295 300
   Val Lys Pro Val Thr Asp Ser Thr Ser Ala Pro Ala Pro Lys Leu Ala
   305 310 315 320
   Leu Arg Glu Thr Pro Ala Pro Leu Ala Val Thr Ser Ser Glu Pro Ser
   325 330 335
   Thr Thr Pro Asn Asn Trp Ala Asp Phe Ser Ser Thr Trp Pro Thr Ser
   340 345 350
   Thr Asn Glu Lys Pro Glu Thr Asp Asn Trp Asp Ala Trp Ala Ala Gln
   355 360 365
   Pro Ser Leu Thr Val Pro Ser Ala Gly Gln Leu Arg Gln Arg Ser Ala
   370 375 380
   Phe Thr Pro Ala Thr Ala Thr Gly Ser Ser Pro Ser Pro Val Leu Gly
   385 390 395 400
   Gln Gly Glu Lys Val Glu Gly Leu Gln Ala Gln Ala Leu Tyr Pro Trp
   405 410 415
   Arg Ala Lys Lys Asp Asn His Leu Asn Phe Asn Lys Asn Asp Val Ile
   420 425 430
   Thr Val Leu Glu Gln Gln Asp Met Trp Trp Phe Gly Glu Val Gln Gly
   435 440 445
   Gln Lys Gly Trp Phe Pro Lys Ser Tyr Val Lys Leu Ile Ser Gly Pro
       450                 455                 460
   Ile Arg Lys Ser Thr Ser Met Asp Ser Gly Ser Ser Glu Ser Pro Ala
   465                 470                 475                 480
   Ser Leu Lys Arg Val Ala Ser Pro Ala Ala Lys Pro Val Val Ser Gly
                   485                 490                 495
   Glu Glu Ile Ala Gln Val Ile Ala Ser Tyr Thr Ala Thr Gly Pro Glu
               500                 505                 510
   Gln Leu Thr Leu Ala Pro Gly Gln Leu Ile Leu Ile Arg Lys Lys Asn
           515                 520                 525
   Pro Gly Gly Trp Trp Glu Gly Glu Leu Gln Ala Arg Gly Lys Lys Arg
       530                 535                 540
   Gln Ile Gly Trp Phe Pro Ala Asn Tyr Val Lys Leu Leu Ser Pro Gly
   545                 550                 555                 560
   Thr Ser Lys Ile Thr Pro Thr Glu Pro Pro Lys Ser Thr Ala Leu Ala
                   565                 570                 575
   Ala Val Cys Gln Val Ile Gly Met Tyr Asp Tyr Thr Ala Gln Asn Asp
               580                 585                 590
   Asp Glu Leu Ala Phe Asn Lys Gly Gln Ile Ile Asn Val Leu Asn Lys
           595                 600                 605
   Glu Asp Pro Asp Trp Trp Lys Gly Glu Val Asn Gly Gln Val Gly Leu
       610                 615                 620
   Phe Pro Ser Asn Tyr Val Lys Leu Thr Thr Asp Met Asp Pro Ser Gln
   625                 630                 635                 640
   Gln
<210> 79
   <211> 10
   <212> PRT
   <213> Homo sapiens
<400> 79
   Ile Ile Cys Cys Pro Ser Pro Pro Gln Ala
   1               5                   10
<210> 80
   <211> 11
   <212> PRT
   <213> Homo sapiens
<400> 80
   Lys Ser Phe Cys Gly Phe Pro Ser Tyr Ser Asn
    1               5                   10
<210> 81
   <211> 30
   <212> PRT
   <213> Homo sapiens
<400> 81
   Leu Ser Pro Thr Phe Ala Gln Val Leu Ser Ile Val Leu Lys Leu Phe
    1               5                   10                  15
   Leu Asn Ile Tyr Phe Ser Phe Leu Ile Asn Lys Ile Asn Lys
               20                  25                  30
<210> 82
   <211> 20
   <212> PRT
   <213> Homo sapiens
<400> 82
   Leu Leu Cys Tyr Phe Gly Phe Ala Lys Arg Pro Thr Ile Lys Glu Cys
    1               5                   10                  15
   Cys Met Cys Tyr
               20
<210> 83
   <211> 34
   <212> PRT
   <213> Homo sapiens
<400> 83
   Lys Leu Phe Gln Met Ser Ile Asn Leu Arg Leu Asp Val Phe Phe His
    1               5                   10                  15
   Phe Val Gln Cys Tyr Gln Leu Asn Cys Ala Val Trp Gly Phe Ser Pro
               20                  25                  30
   Leu Pro
<210> 84
   <211> 13
   <212> PRT
   <213> Homo sapiens
<400> 84
   Lys Cys Arg Gly Val Gln Tyr Leu Cys Phe Lys Asp Val
   1 5 10
<210> 85
   <211> 4
   <212> PRT
   <213> Homo sapiens
<400> 85
   Asn Glu Pro Asn
   1
<210> 86
   <211> 15
   <212> PRT
   <213> Homo sapiens
<400> 86
   Ser Glu Gly Val Cys Ala Cys Leu Cys Val Ser Ala Val Pro Cys
   1 5 10 15
<210> 87
   <211> 7
   <212> PRT
   <213> Homo sapiens
<400> 87
   Ala Cys Asn Thr Ser Cys Thr
   1 5
<210> 88
   <211> 29
   <212> PRT
   <213> Homo sapiens
<400> 88
   Glu Ile Ser Ser Phe His Gly Lys Ala Ile Thr Leu Tyr Asp Ala Leu
   1 5 10 15
   Ile Ile Leu His Leu Ile Leu Phe Cys Thr Val Thr Leu
   20 25
<210> 89
   <211> 33
   <212> PRT
   <213> Homo sapiens
<400> 89
   Pro His Glu Lys Ala Leu Cys Val Phe Val Arg Ser Gln Ile Tyr Leu
   1 5 10 15
   Val Glu Leu Val Phe Cys Leu Gly Phe Leu Ile Leu Arg Val Cys Ile
   20 25 30
   Ala
<210> 90
   <211> 2
   <212> PRT
   <213> Homo sapiens
<400> 90
   Asn Gln
   1
<210> 91
   <211> 16
   <212> PRT
   <213> Homo sapiens
<400> 91
   Thr Thr Pro Leu Arg Ser Leu Arg Ser Thr Ile Ser Thr Val Ser Phe
   1 5 10 15
<210> 92
   <211> 14
   <212> PRT
   <213> Homo sapiens
<400> 92
   Ser Leu Leu His Glu Val Leu Phe Gln Leu Leu Phe Met Glu
   1 5 10
<210> 93
   <211> 5
   <212> PRT
   <213> Homo sapiens
<400> 93
   Pro Ile Leu Asn Lys
   1 5
<210> 94
   <211> 2
   <212> PRT
   <213> Homo sapiens
<400> 94
   Phe Ser
   1
<210> 95
   <211> 29
   <212> PRT
   <213> Homo sapiens
<400> 95
   Gln Glu Arg Met Tyr Arg Ser Leu Pro Ala Ile Asn Phe Gln Cys Leu
   1 5 10 15
   His Phe Leu Thr Arg Leu Trp Asn Phe Tyr Arg Leu Ile
   20 25
<210> 96
   <211> 9
   <212> PRT
   <213> Homo sapiens
<400> 96
   Asn Gly Ala His Gly Pro Phe Val Cys
   1 5
<210> 97
   <211> 4
   <212> PRT
   <213> Homo sapiens
<400> 97
   Ile Cys Cys Ser
   1
<210> 98
   <211> 33
   <212> PRT
   <213> Homo sapiens
<400> 98
   Ser Pro Val Cys Leu Leu Asn Thr Ser Trp Lys Leu Ser Ile Lys Met
   1 5 10 15
   Pro Ala Ala His Ser Thr Glu Asn Gly Ala Gly Gly Ala Ser Ser Thr
   20 25 30
   Ile
<210> 99
   <211> 3
   <212> PRT
   <213> Homo sapiens
<400> 99
   Leu Ser Ser
   1
<210> 100
   <211> 62
   <212> PRT
   <213> Homo sapiens
<400> 100
   Arg Leu Cys Asn Ala His Ser Pro Arg Val Leu Pro Ala Leu Ser Gly
   1 5 10 15
   Gly Cys Ala Gly Gly Arg Val Arg Ser Phe Ser Phe His Met Val Gln
   20 25 30
   Arg Ala Arg Thr Ser Pro Pro Arg Ser Val Ala Leu Gln Tyr Phe His
   35 40 45
   Gly Tyr Glu Cys Lys Ile Tyr Lys Tyr Ile Asn Leu Arg Leu
   50 55 60
<210> 101
   <211> 2
   <212> PRT
   <213> Homo sapiens
<400> 101
   Gln Leu
   1
<210> 102
   <211> 5
   <212> PRT
   <213> Homo sapiens
<400> 102
   Tyr Asn Leu Leu Asn
   1 5
<210> 103
   <211> 3
   <212> PRT
   <213> Homo sapiens
<400> 103
   Phe Arg Val
   1
<210> 104
   <211> 14
   <212> PRT
   <213> Homo sapiens
<220>
   <223> From Seq ID 78 to ID 104, there are 27 pretein sequences translated from Seq ID No. 76. Together, they form the whole protein sequence.
<400> 104
   Ile Ile Lys Phe Phe Ile Gln Lys Leu Lys Lys Lys Lys Lys
   1 5 10
<210> 105
   <211> 1721
   <212> PRT
   <213> Homo sapiens
<400> 105
   Met Ala Gln Phe Pro Thr Pro Phe Gly Gly Ser Leu Asp Ile Trp Ala
    1               5                  10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Pro Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
       130                 135                 140
   Thr Leu Val Ser Ser Val Pro Thr Ala Ala Val Pro Pro Leu Ala Asn
   145                 150                 155                 160
   Gly Ala Pro Pro Val Ile Gln Pro Leu Pro Ala Phe Ala His Pro Ala
                   165                 170                 175
   Ala Thr Leu Pro Lys Ser Ser Ser Phe Ser Arg Ser Gly Pro Gly Ser
   180 185 190
   Gln Leu Asn Thr Lys Leu Gln Lys Ala Gln Ser Phe Asp Val Ala Ser
   195 200 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385 390 395 400
   Gln Glu Arg Lys Arg Gln Leu Glu Leu Glu Lys Gln Leu Glu Lys Gln
   405 410 415
   Arg Glu Leu Glu Arg Gln Arg Glu Glu Glu Arg Arg Lys Glu Ile Glu
   420 425 430
   Arg Arg Glu Ala Ala Lys Arg Glu Leu Glu Arg Gln Arg Gln Leu Glu
   435 440 445
   Trp Glu Arg Asn Arg Arg Gln Glu Leu Leu Asn Gln Arg Asn Lys Glu
   450 455 460
   Gln Glu Asp Ile Val Val Leu Lys Ala Lys Lys Lys Thr Leu Glu Phe
   465 470 475 480
   Glu Leu Glu Ala Leu Asn Asp Lys Lys His Gln Leu Glu Gly Lys Leu
   485 490 495
   Gln Asp Ile Arg Cys Arg Leu Thr Thr Gln Arg Gln Glu Ile Glu Ser
   500 505 510
   Thr Asn Lys Ser Arg Glu Leu Arg Ile Ala Glu Ile Thr His Leu Gln
   515 520 525
   Gln Gln Leu Gln Glu Ser Gln Gln Met Leu Gly Arg Leu Ile Pro Glu
   530 535 540
   Lys Gln Ile Leu Asn Asp Gln Leu Lys Gln Val Gln Gln Asn Ser Leu
   545 550 555 560
   His Arg Asp Ser Leu Val Thr Leu Lys Arg Ala Leu Glu Ala Lys Glu
   565 570 575
   Leu Ala Arg Gln His Leu Arg Asp Gln Leu Asp Glu Val Glu Lys Glu
   580 585 590
   Thr Arg Ser Lys Leu Gln Glu Ile Asp Ile Phe Asn Asn Gln Leu Lys
   595 600 605
   Glu Leu Arg Glu Ile His Asn Lys Gln Gln Leu Gln Lys Gln Lys Ser
   610 615 620
   Met Glu Ala Glu Arg Leu Lys Gln Lys Glu Gln Glu Arg Lys Ile Ile
   625 630 635 640
   Glu Leu Glu Lys Gln Lys Glu Glu Ala Gln Arg Arg Ala Gln Glu Arg
   645 650 655
   Asp Lys Gln Trp Leu Glu His Val Gln Gln Glu Asp Glu His Gln Arg
   660 665 670
   Pro Arg Lys Leu His Glu Glu Glu Lys Leu Lys Arg Glu Glu Ser Val
   675 680 685
   Lys Lys Lys Asp Gly Glu Glu Lys Gly Lys Gln Glu Ala Gln Asp Lys
       690                 695                 700
   Leu Gly Arg Leu Phe His Gln His Gln Glu Pro Ala Lys Pro Ala Val
   705                 710                 715                 720
   Gln Ala Pro Trp Ser Thr Ala Glu Lys Gly Pro Leu Thr Ile Ser Ala
                   725                 730                 735
   Gln Glu Asn Val Lys Val Val Tyr Tyr Arg Ala Leu Tyr Pro Phe Glu
               740                 745                 750
   Ser Arg Ser His Asp Glu Ile Thr Ile Gln Pro Gly Asp Ile Val Met
           755                 760                 765
   Val Lys Gly Glu Trp Val Asp Glu Ser Gln Thr Gly Glu Pro Gly Trp
       770                 775                 780
   Leu Gly Gly Glu Leu Lys Gly Lys Thr Gly Trp Phe Pro Ala Asn Tyr
   785                 790                 795                 800
   Ala Glu Lys Ile Pro Glu Asn Glu Val Pro Ala Pro Val Lys Pro Val
                   805                 810                 815
   Thr Asp Ser Thr Ser Ala Pro Ala Pro Lys Leu Ala Leu Arg Glu Thr
               820                 825                 830
   Pro Ala Pro Leu Ala Val Thr Ser Ser Glu Pro Ser Thr Thr Pro Asn
           835                 840                 845
   Asn Trp Ala Asp Phe Ser Ser Thr Trp Pro Thr Ser Thr Asn Glu Lys
       850                 855                 860
   Pro Glu Thr Asp Asn Trp Asp Ala Trp Ala Ala Gln Pro Ser Leu Thr
   865                 870                 875                 880
   Val Pro Ser Ala Gly Gln Leu Arg Gln Arg Ser Ala Phe Thr Pro Ala
                   885                 890                 895
   Thr Ala Thr Gly Ser Ser Pro Ser Pro Val Leu Gly Gln Gly Glu Lys
               900                 905                 910
   Val Glu Gly Leu Gln Ala Gln Ala Leu Tyr Pro Trp Arg Ala Lys Lys
           915                 920                 925
   Asp Asn His Leu Asn Phe Asn Lys Asn Asp Val Ile Thr Val Leu Glu
       930                 935                 940
   Gln Gln Asp Met Trp Trp Phe Gly Glu Val Gln Gly Gln Lys Gly Trp
   945 950 955 960
   Phe Pro Lys Ser Tyr Val Lys Leu Ile Ser Gly Pro Ile Arg Lys Ser
   965 970 975
   Thr Ser Met Asp Ser Gly Ser Ser Glu Ser Pro Ala Ser Leu Lys Arg
   980 985 990
   Val Ala Ser Pro Ala Ala Lys Pro Val Val Ser Gly Glu Glu Phe Ile
   995 1000 1005
   Ala Met Tyr Thr Tyr Glu Ser Ser Glu Gln Gly Asp Leu Thr Phe Gln
   1010 1015 1020
   Gln Gly Asp Val Ile Leu Val Thr Lys Lys Asp Gly Asp Trp Trp Thr
   1025 1030 1035 1040
   Gly Thr Val Gly Asp Lys Ala Gly Val Phe Pro Ser Asn Tyr Val Arg
   1045 1050 1055
   Leu Lys Asp Ser Glu Gly Ser Gly Thr Ala Gly Lys Thr Gly Ser Leu
   1060 1065 1070
   Gly Lys Lys Pro Glu Ile Ala Gln Val Ile Ala Ser Tyr Thr Ala Thr
   1075 1080 1085
   Gly Pro Glu Gln Leu Thr Leu Ala Pro Gly Gln Leu Ile Leu Ile Arg
   1090 1095 1100
   Lys Lys Asn Pro Gly Gly Trp Trp Glu Gly Glu Leu Gln Ala Arg Gly
   1105 1110 1115 1120
   Lys Lys Arg Gln Ile Gly Trp Phe Pro Ala Asn Tyr Val Lys Leu Leu
   1125 1130 1135
   Asn Pro Gly Thr Ser Lys Ile Thr Pro Thr Glu Pro Pro Lys Ser Thr
   1140 1145 1150
   Ala Leu Ala Ala Val Cys Gln Val Ile Gly Met Tyr Asp Tyr Thr Ala
   1155 1160 1165
   Gln Asn Asp Asp Glu Leu Ala Phe Asn Lys Gly Gln Ile Ile Asn Val
   1170 1175 1180
   Leu Asn Lys Glu Asp Pro Asp Trp Trp Lys Gly Glu Val Asn Gly Gln
   1185 1190 1195 1200
   Val Gly Leu Phe Pro Ser Asn Tyr Val Lys Leu Thr Thr Asp Met Asp
   1205 1210 1215
   Pro Ser Gln Gln Trp Cys Ser Asp Leu His Leu Leu Asp Met Leu Thr
   1220 1225 1230
   Pro Thr Glu Arg Lys Arg Gln Gly Tyr Ile His Glu Leu Ile Val Thr
   1235 1240 1245
   Glu Glu Asn Tyr Val Asn Asp Leu Gln Leu Val Thr Glu Ile Phe Gln
   1250 1255 1260
   Lys Pro Leu Met Glu Ser Glu Leu Leu Thr Glu Lys Glu Val Ala Met
   1265 1270 1275 1280
   Ile Phe Val Asn Trp Lys Glu Leu Ile Met Cys Asn Ile Lys Leu Leu
   1285 1290 1295
   Lys Ala Leu Arg Val Arg Lys Lys Met Ser Gly Glu Lys Met Pro Val
   1300 1305 1310
   Lys Met Ile Gly Asp Ile Leu Ser Ala Gln Leu Pro His Met Gln Pro
   1315 1320 1325
   Tyr Ile Arg Phe Cys Ser Arg Gln Leu Asn Gly Ala Ala Leu Ile Gln
   1330 1335 1340
   Gln Lys Thr Asp Glu Ala Pro Asp Phe Lys Glu Phe Val Lys Arg Leu
   1345 1350 1355 1360
   Glu Met Asp Pro Arg Cys Lys Gly Met Pro Leu Ser Ser Phe Ile Leu
   1365 1370 1375
   Lys Pro Met Gln Arg Val Thr Arg Tyr Pro Leu Ile Ile Lys Asn Ile
   1380 1385 1390
   Leu Glu Asn Thr Pro Glu Asn His Pro Asp His Ser His Leu Lys His
   1395 1400 1405
   Ala Leu Glu Lys Ala Glu Glu Leu Cys Ser Gln Val Asn Glu Gly Val
   1410 1415 1420
   Arg Glu Lys Glu Asn Ser Asp Arg Leu Glu Trp Ile Gln Ala His Val
   1425 1430 1435 1440
   Gln Cys Glu Gly Leu Ser Glu Gln Leu Val Phe Asn Ser Val Thr Asn
   1445 1450 1455
   Cys Leu Gly Pro Arg Lys Phe Leu His Ser Gly Lys Leu Tyr Lys Ala
   1460 1465 1470
   Lys Asn Asn Lys Glu Leu Tyr Gly Phe Leu Phe Asn Asp Phe Leu Leu
   1475 1480 1485
   Leu Thr Gln Ile Thr Lys Pro Leu Gly Ser Ser Gly Thr Asp Lys Val
   1490 1495 1500
   Phe Ser Pro Lys Ser Asn Leu Gln Tyr Lys Met Tyr Lys Thr Pro Ile
   1505 1510 1515 1520
   Phe Leu Asn Glu Val Leu Val Lys Leu Pro Thr Asp Pro Ser Gly Asp
   1525 1530 1535
   Glu Pro Ile Phe His Ile Ser His Ile Asp Arg Val Tyr Thr Leu Arg
   1540 1545 1550
   Ala Glu Ser Ile Asn Glu Arg Thr Ala Trp Val Gln Lys Ile Lys Ala
   1555 1560 1565
   Ala Ser Glu Leu Tyr Ile Glu Thr Glu Lys Lys Lys Arg Glu Lys Ala
   1570 1575 1580
   Tyr Leu Val Arg Ser Gln Arg Ala Thr Gly Ile Gly Arg Leu Met Val
   1585 1590 1595 1600
   Asn Val Val Glu Gly Ile Glu Leu Lys Pro Cys Arg Ser His Gly Lys
   1605 1610 1615
   Ser Asn Pro Tyr Cys Glu Val Thr Met Gly Ser Gln Cys His Ile Thr
   1620 1625 1630
   Lys Thr Ile Gln Asp Thr Leu Asn Pro Lys Trp Asn Ser Asn Cys Gln
   1635 1640 1645
   Phe Phe Ile Arg Asp Leu Glu Gln Glu Val Leu Cys Ile Thr Val Phe
   1650 1655 1660
   Glu Arg Asp Gln Phe Ser Pro Asp Asp Phe Leu Gly Arg Thr Glu Ile
   1665 1670 1675 1680
   Arg Val Ala Asp Ile Lys Lys Asp Gln Gly Ser Lys Gly Pro Val Thr
   1685 1690 1695
   Lys Cys Leu Leu Leu His Glu Val Pro Thr Gly Glu Ile Val Val Arg
   1700 1705 1710
   Leu Asp Leu Gln Leu Phe Asp Glu Pro
   1715 1720
<210> 106
   <211> 1220
   <212> PRT
   <213> Homo sapiens
<400> 106
   Met Ala Gln Phe Pro Thr Pro Phe Gly Gly Ser Leu Asp Ile Trp Ala
   1               5                   10                  15
   Ile Thr Val Glu Glu Arg Ala Lys His Asp Gln Gln Phe His Ser Leu
               20                  25                  30
   Lys Pro Ile Ser Gly Phe Ile Thr Gly Asp Gln Ala Arg Asn Phe Phe
           35                  40                  45
   Phe Gln Ser Gly Leu Pro Gln Pro Val Leu Ala Gln Ile Trp Ala Leu
       50                  55                  60
   Ala Asp Met Asn Asn Asp Gly Arg Met Asp Gln Val Glu Phe Ser Ile
   65                  70                  75                  80
   Ala Met Lys Leu Ile Lys Leu Lys Leu Gln Gly Tyr Gln Leu Pro Ser
                   85                  90                  95
   Ala Leu Pro Pro Val Met Lys Gln Gln Pro Val Ala Ile Ser Ser Ala
               100                 105                 110
   Pro Pro Phe Gly Met Gly Gly Ile Ala Ser Met Pro Pro Leu Thr Ala
           115                 120                 125
   Val Ala Pro Val Pro Met Gly Ser Ile Pro Val Val Gly Met Ser Pro
       130                 135                 140
   Thr Leu Val Ser Ser Val Pro Thr Ala Ala Val Pro Pro Leu Ala Asn
   145                 150                 155                 160
   Gly Ala Pro Pro Val Ile Gln Pro Leu Pro Ala Phe Ala His Pro Ala
                   165                 170                 175
   Ala Thr Leu Pro Lys Ser Ser Ser Phe Ser Arg Ser Gly Pro Gly Ser
               180                 185                 190
   Gln Leu Asn Thr Lys Leu Gln Lys Ala Gln Ser Phe Asp Val Ala Ser
           195                 200                 205
   Val Pro Pro Val Ala Glu Trp Ala Val Pro Gln Ser Ser Arg Leu Lys
   210 215 220
   Tyr Arg Gln Leu Phe Asn Ser His Asp Lys Thr Met Ser Gly His Leu
   225 230 235 240
   Thr Gly Pro Gln Ala Arg Thr Ile Leu Met Gln Ser Ser Leu Pro Gln
   245 250 255
   Ala Gln Leu Ala Ser Ile Trp Asn Leu Ser Asp Ile Asp Gln Asp Gly
   260 265 270
   Lys Leu Thr Ala Glu Glu Phe Ile Leu Ala Met His Leu Ile Asp Val
   275 280 285
   Ala Met Ser Gly Gln Pro Leu Pro Pro Val Leu Pro Pro Glu Tyr Ile
   290 295 300
   Pro Pro Ser Phe Arg Arg Val Arg Ser Gly Ser Gly Ile Ser Val Ile
   305 310 315 320
   Ser Ser Thr Ser Val Asp Gln Arg Leu Pro Glu Glu Pro Val Leu Glu
   325 330 335
   Asp Glu Gln Gln Gln Leu Glu Lys Lys Leu Pro Val Thr Phe Glu Asp
   340 345 350
   Lys Lys Arg Glu Asn Phe Glu Arg Gly Asn Leu Glu Leu Glu Lys Arg
   355 360 365
   Arg Gln Ala Leu Leu Glu Gln Gln Arg Lys Glu Gln Glu Arg Leu Ala
   370 375 380
   Gln Leu Glu Arg Ala Glu Gln Glu Arg Lys Glu Arg Glu Arg Gln Glu
   385 390 395 400
   Gln Glu Arg Lys Arg Gln Leu Glu Leu Glu Lys Gln Leu Glu Lys Gln
   405 410 415
   Arg Glu Leu Glu Arg Gln Arg Glu Glu Glu Arg Arg Lys Glu Ile Glu
   420 425 430
   Arg Arg Glu Ala Ala Lys Arg Glu Leu Glu Arg Gln Arg Gln Leu Glu
   435 440 445
   Trp Glu Arg Asn Arg Arg Gln Glu Leu Leu Asn Gln Arg Asn Lys Glu
   450 455 460
   Gln Glu Asp Ile Val Val Leu Lys Ala Lys Lys Lys Thr Leu Glu Phe
   465 470 475 480
   Glu Leu Glu Ala Leu Asn Asp Lys Lys His Gln Leu Glu Gly Lys Leu
   485 490 495
   Gln Asp Ile Arg Cys Arg Leu Thr Thr Gln Arg Gln Glu Ile Glu Ser
   500 505 510
   Thr Asn Lys Ser Arg Glu Leu Arg Ile Ala Glu Ile Thr His Leu Gln
   515 520 525
   Gln Gln Leu Gln Glu Ser Gln Gln Met Leu Gly Arg Leu Ile Pro Glu
   530 535 540
   Lys Gln Ile Leu Asn Asp Gln Leu Lys Gln Val Gln Gln Asn Ser Leu
   545 550 555   560
   His Arg Asp Ser Leu Val Thr Leu Lys Arg Ala Leu Glu Ala Lys Glu
   565 570 575
   Leu Ala Arg Gln His Leu Arg Asp Gln Leu Asp Glu Val Glu Lys Glu
   580 585 590
   Thr Arg Ser Lys Leu Gln Glu Ile Asp Ile Phe Asn Asn Gln Leu Lys
   595 600 605
   Glu Leu Arg Glu Ile His Asn Lys Gln Gln Leu Gln Lys Gln Lys Ser
   610 615 620
   Met Glu Ala Glu Arg Leu Lys Gln Lys Glu Gln Glu Arg Lys Ile Ile
   625 630 635 640
   Glu Leu Glu Lys Gln Lys Glu Glu Ala Gln Arg Arg Ala Gln Glu Arg
   645 650 655
   Asp Lys Gln Trp Leu Glu His Val Gln Gln Glu Asp Glu His Gln Arg
   660 665 670
   Pro Arg Lys Leu His Glu Glu Glu Lys Leu Lys Arg Glu Glu Ser Val
   675 680 685
   Lys Lys Lys Asp Gly Glu Glu Lys Gly Lys Gln Glu Ala Gln Asp Lys
   690 695 700
   Leu Gly Arg Leu Phe His Gln His Gln Glu Pro Ala Lys Pro Ala Val
   705 710 715 720
   Gln Ala Pro Trp Ser Thr Ala Glu Lys Gly Pro Leu Thr Ile Ser Ala
   725 730 735
   Gln Glu Asn Val Lys Val Val Tyr Tyr Arg Ala Leu Tyr Pro Phe Glu
   740 745 750
   Ser Arg Ser His Asp Glu Ile Thr Ile Gln Pro Gly Asp Ile Val Met
   755 760 765
   Val Lys Gly Glu Trp Val Asp Glu Ser Gln Thr Gly Glu Pro Gly Trp
   770 775 780
   Leu Gly Gly Glu Leu Lys Gly Lys Thr Gly Trp Phe Pro Ala Asn Tyr
   785 790 795 800
   Ala Glu Lys Ile Pro Glu Asn Glu Val Pro Ala Pro Val Lys Pro Val
   805 810 815
   Thr Asp Ser Thr Ser Ala Pro Ala Pro Lys Leu Ala Leu Arg Glu Thr
   820 825 830
   Pro Ala Pro Leu Ala Val Thr Ser Ser Glu Pro Ser Thr Thr Pro Asn
   835 840 845
   Asn Trp Ala Asp Phe Ser Ser Thr Trp Pro Thr Ser Thr Asn Glu Lys
   850 855 860
   Pro Glu Thr Asp Asn Trp Asp Ala Trp Ala Ala Gln Pro Ser Leu Thr
   865 870 875 880
   Val Pro Ser Ala Gly Gln Leu Arg Gln Arg Ser Ala Phe Thr Pro Ala
   885 890 895
   Thr Ala Thr Gly Ser Ser Pro Ser Pro Val Leu Gly Gln Gly Glu Lys
   900 905 910
   Val Glu Gly Leu Gln Ala Gln Ala Leu Tyr Pro Trp Arg Ala Lys Lys
   915 920 925
   Asp Asn His Leu Asn Phe Asn Lys Asn Asp Val Ile Thr Val Leu Glu
   930 935 940
   Gln Gln Asp Met Trp Trp Phe Gly Glu Val Gln Gly Gln Lys Gly Trp
   945 950 955 960
   Phe Pro Lys Ser Tyr Val Lys Leu Ile Ser Gly Pro Ile Arg Lys Ser
   965 970 975
   Thr Ser Met Asp Ser Gly Ser Ser Glu Ser Pro Ala Ser Leu Lys Arg
               980                 985                 990
   Val Ala Ser Pro Ala Ala Lys Pro Val Val Ser Gly Glu Glu Phe Ile
           995                 1000                1005
   Ala Met Tyr Thr Tyr Glu Ser Ser Glu Gln Gly Asp Leu Thr Phe Gln
       1010                1015                1020
   Gln Gly Asp Val Ile Leu Val Thr Lys Lys Asp Gly Asp Trp Trp Thr
   1025                1030                1035                1040
   Gly Thr Val Gly Asp Lys Ala Gly Val Phe Pro Ser Asn Tyr Val Arg
                   1045                1050                1055
   Leu Lys Asp Ser Glu Gly Ser Gly Thr Ala Gly Lys Thr Gly Ser Leu
               1060                1065                1070
   Gly Lys Lys Pro Glu Ile Ala Gln Val Ile Ala Ser Tyr Thr Ala Thr
           1075                1080                1085
   Gly Pro Glu Gln Leu Thr Leu Ala Pro Gly Gln Leu Ile Leu Ile Arg
       1090                1095                1100
   Lys Lys Asn Pro Gly Gly Trp Trp Glu Gly Glu Leu Gln Ala Arg Gly
   1105                1110                1115                1120
   Lys Lys Arg Gln Ile Gly Trp Phe Pro Ala Asn Tyr Val Lys Leu Leu
                   1125                1130                1135
   Asn Pro Gly Thr Ser Lys Ile Thr Pro Thr Glu Pro Pro Lys Ser Thr
               1140                1145                1150
   Ala Leu Ala Ala Val Cys Gln Val Ile Gly Met Tyr Asp Tyr Thr Ala
           1155                1160                1165
   Gln Asn Asp Asp Glu Leu Ala Phe Asn Lys Gly Gln Ile Ile Asn Val
       1170                1175                1180
   Leu Asn Lys Glu Asp Pro Asp Trp Trp Lys Gly Glu Val Asn Gly Gln
   1185                1190                1195                1200
   Val Gly Leu Phe Pro Ser Asn Tyr Val Lys Leu Thr Thr Asp Met Asp
                   1205                1210                1215
   Pro Ser Gln Gln
               1220
<210> 107
   <211> 1270
   <212> PRT
   <213> Xenopus laevis
<400> 107
   Met Ala Gln Phe Gly Thr Pro Phe Gly Gly Asn Leu Asp Ile Trp Ala
   1               5                   10                  15
   Ile Thr Val Glu Glu Arg Ala Lys His Asp Gln Gln Phe His Gly Leu
               20                  25                  30
   Lys Pro Thr Ala Gly Tyr Ile Thr Gly Asp Gln Ala Arg Asn Phe Phe
           35                  40                  45
   Leu Gln Ser Gly Leu Pro Gln Pro Val Leu Ala Gln Ile Trp Ala Leu
       50                  55                  60
   Ala Asp Met Asn Asn Asp Gly Arg Met Asp Gln Leu Glu Phe Ser Ile
   65                  70                  75                  80
   Ala Met Lys Leu Ile Lys Leu Lys Leu Gln Gly Tyr Pro Leu Pro Ser
                   85                  90                  95
   Ile Leu Pro Ser Asn Met Leu Lys Gln Pro Val Ala Met Pro Ala Ala
               100                 105                 110
   Ala Val Ala Gly Phe Gly Met Ser Gly Ile Val Gly Ile Pro Pro Leu
           115                 120                 125
   Ala Ala Val Ala Pro Val Pro Met Pro Ser Ile Pro Val Val Gly Met
       130                 135                 140
   Ser Pro Pro Leu Val Ser Ser Val Pro Thr Val Pro Pro Leu Ser Asn
   145                 150                 155                 160
   Gly Ala Pro Ala Val Ile Gln Ser His Pro Ala Phe Ala His Ser Ala
                   165                 170                 175
   Thr Leu Pro Lys Ser Ser Ser Phe Gly Arg Ser Val Ala Gly Ser Gln
               180                 185                 190
   Ile Asn Thr Lys Leu Gln Lys Ala Gln Ser Phe Asp Val Pro Ala Pro
           195                 200                 205
   Pro Leu Val Val Glu Trp Ala Val Pro Ser Ser Ser Arg Leu Lys Tyr
           210                 215                 220
   Arg Gln Leu Phe Asn Ser Gln Asp Lys Thr Met Ser Gly Asn Leu Thr
   225                 230                 235                 240
   Gly Pro Gln Ala Arg Thr Ile Leu Met Gln Ser Ser Leu Pro Gln Ser
                   245                 250                 255
   Gln Leu Ala Thr Ile Trp Asn Leu Ser Asp Ile Asp Gln Asp Gly Lys
               260                 265                 270
   Leu Thr Ala Glu Glu Phe Ile Leu Ala Met His Leu Ile Asp Val Ala
           275                 280                 285
   Met Ser Gly Gln Pro Leu Pro Pro Ile Leu Pro Pro Glu Tyr Ile Pro
       290                 295                 300
   Pro Ser Phe Arg Arg Val Arg Ser Gly Ser Gly Leu Ser Ile Met Ser
   305                 310                 315                 320
   Ser Val Ser Val Asp Gln Arg Leu Pro Glu Glu Pro Glu Glu Glu Glu
                   325                 330                 335
   Pro Gln Asn Ala Asp Lys Lys Leu Pro Val Thr Phe Glu Asp Lys Lys
               340                 345                 350
   Arg Glu Asn Phe Glu Arg Gly Asn Leu Glu Leu Glu Lys Arg Arg Gln
           355                 360                 365
   Ala Leu Leu Glu Gln Gln Arg Lys Glu Gln Glu Arg Leu Ala Gln Leu
       370                 375                 380
   Glu Arg Ala Glu Gln Glu Arg Lys Glu Arg Glu Arg Gln Asp Gln Glu
   385                 390                 395                 400
   Arg Lys Arg Gln Gln Asp Leu Glu Lys Gln Leu Glu Lys Gln Arg Glu
                   405                 410                 415
   Leu Glu Arg Gln Arg Glu Glu Glu Arg Arg Lys Glu Ile Glu Arg Arg
               420                 425                 430
   Glu Ala Ala Lys Arg Glu Leu Glu Arg Gln Arg Gln Leu Glu Trp Glu
           435                 440                 445
   Arg Asn Arg Arg Gln Glu Leu Leu Asn Gln Arg Asn Arg Glu Gln Glu
       450                 455                 460
       Asp Ile Val Val Leu Lys Ala Lys Lys Lys Thr Leu Glu Phe Glu Leu
   465 470 475 480
   Glu Ala Leu Asn Asp Lys Lys His Gln Leu Glu Gly Lys Leu Gln Asp
   485 490 495
   Ile Arg Cys Arg Leu Thr Thr Gln Arg His Glu Ile Glu Ser Thr Asn
   500 505 510
   Lys Ser Arg Glu Leu Arg Ile Ala Glu Ile Thr His Leu Gln Gln Gln
   515 520 525
   Leu Gln Glu Ser Gln Gln Leu Leu Gly Lys Met Ile Pro Glu Lys Gln
   530 535 540
   Ser Leu Ile Asp Gln Leu Lys Gln Val Gln Gln Asn Ser Leu His Arg
   545 550 555 560
   Asp Ser Leu Leu Thr Leu Lys Arg Ala Leu Glu Thr Lys Glu Ile Gly
   565 570 575
   Arg Gln Gln Leu Arg Asp Gln Leu Asp Glu Val Glu Lys Glu Thr Arg
   580 585 590
   Ala Lys Leu Gln Glu Ile Asp Val Phe Asn Asn Gln Leu Lys Glu Leu
   595 600 605
   Arg Glu Leu Tyr Asn Lys Gln Gln Phe Gln Lys Gln Gln Asp Phe Glu
   610 615 620
   Thr Glu Lys Ile Lys Gln Lys Glu Leu Glu Arg Lys Thr Ser Glu Leu
   625 630 635 640
   Asp Lys Leu Lys Glu Glu Asp Lys Arg Arg Met Leu Glu Gln Asp Lys
   645 650 655
   Leu Trp Gln Asp Arg Val Lys Gln Glu Glu Glu Arg Tyr Lys Phe Gln
   660 665 670
   Asp Glu Glu Lys Glu Lys Arg Glu Glu Ser Val Gln Lys Cys Glu Val
   675 680 685
   Glu Lys Lys Pro Glu Ile Gln Glu Lys Pro Asn Lys Pro Phe His Gln
   690 695 700
   Pro Pro Glu Pro Gly Lys Leu Gly Gly Gln Ile Pro Trp Met Asn Thr
   705 710 715 720
   Glu Lys Ala Pro Leu Thr Ile Asn Gln Gly Asp Val Lys Val Val Tyr
   725 730 735
   Tyr Arg Ala Leu Tyr Pro Phe Asp Ala Arg Ser His Asp Glu Ile Thr
   740 745 750
   Ile Glu Pro Gly Asp Ile Ile Met Val Asp Glu Ser Gln Thr Gly Glu
   755 760 765
   Pro Gly Trp Leu Gly Gly Glu Leu Lys Gly Lys Thr Gly Trp Phe Pro
   770 775 780
   Ala Asn Tyr Ala Glu Arg Met Pro Glu Ser Glu Phe Pro Ser Thr Thr
   785 790 795 800
   Lys Pro Ala Ala Glu Thr Thr Ala Lys Pro Thr Val His Val Ala Pro
   805 810 815
   Ser Pro Val Ala Pro Ala Ala Phe Thr Asn Thr Ser Thr Asn Ser Asn
   820 825 830
   Asn Trp Ala Asp Phe Ser Ser Thr Trp Pro Thr Asn Asn Thr Asp Lys
   835 840 845
   Val Glu Ser Asp Asn Trp Asp Thr Trp Ala Ala Gln Pro Ser Leu Thr
   850 855 860
   Val Pro Ser Ala Gly Gln His Arg Gln Arg Ser Ala Phe Thr Pro Ala
   865 870 875 880
   Thr Val Thr Gly Ser Ser Pro Ser Pro Val Leu Gly Gln Gly Glu Lys
   885 890 895
   Val Glu Gly Leu Gln Ala Gln Ala Leu Tyr Pro Trp Arg Ala Lys Lys
   900 905 910
   Asp Asn His Leu Asn Phe Asn Lys Asn Asp Val Ile Thr Val Leu Glu
   915 920 925
   Gln Gln Asp Met Trp Trp Phe Gly Glu Val Gln Gly Gln Lys Gly Trp
   930 935 940
   Phe Pro Lys Ser Tyr Val Lys Leu Ile Ser Gly Pro Leu Arg Lys Ser
   945 950 955 960
   Thr Ser Ile Asp Ser Thr Ser Ser Glu Ser Pro Ala Ser Leu Lys Arg
   965 970 975
   Val Ser Ser Pro Ala Phe Lys Pro Ala Ile Gln Gly Glu Glu Tyr Ile
   980 985 990
   Ser Met Tyr Thr Tyr Glu Ser Asn Glu Gln Gly Asp Leu Thr Phe Gln
   995 1000 1005
   Gln Gly Asp Leu Ile Val Val Ile Lys Lys Asp Gly Asp Trp Trp Thr
   1010 1015 1020
   Gly Thr Val Gly Glu Lys Thr Gly Val Phe Pro Ser Asn Tyr Val Arg
   1025 1030 1035 1040
   Pro Lys Asp Ser Glu Ala Ala Gly Ser Gly Gly Lys Thr Gly Ser Leu
   1045 1050 1055
   Gly Lys Lys Pro Glu Ile Ala Gln Val Ile Ala Ser Tyr Ala Ala Thr
   1060 1065 1070
   Ala Pro Glu Gln Leu Thr Leu Ala Pro Gly Gln Leu Ile Leu Ile Arg
   1075 1080 1085
   Lys Lys Asn Pro Gly Gly Trp Trp Glu Gly Glu Leu Gln Ala Arg Gly
   1090 1095 1100
   Lys Lys Arg Gln Ile Gly Trp Phe Pro Ala Asn Tyr Val Lys Leu Leu
   1105 1110 1115 1120
   Ser Pro Gly Thr Asn Lys Ser Thr Pro Thr Glu Pro Pro Lys Pro Thr
   1125 1130 1135
   Ser Leu Pro Pro Thr Cys Gln Val Ile Gly Met Tyr Asp Tyr Ile Ala
   1140 1145 1150
   Gln Asn Asp Asp Glu Leu Ala Phe Ser Lys Gly Gln Val Ile Asn Val
   1155 1160 1165
   Leu Asn Lys Glu Asp Pro Asp Trp Trp Lys Gly Glu Leu Asn Gly His
   1170 1175 1180
   Val Gly Leu Phe Pro Ser Asn Tyr Val Lys Leu Thr Thr Asp Met Asp
   1185 1190 1195 1200
   Pro Ser Gln Gln Phe Arg Leu Gly Val Lys Pro Ala Gly Gly Ile Pro
   1205 1210 1215
   Ala Thr Gly Asp Arg Pro Phe Ile Leu Phe Pro Phe Arg Asp Gly Pro
   1220 1225 1230
   Ser Leu Leu Pro Asn Ala Phe Gln Ala Pro Pro Leu Ser Val Val Met
   1235 1240 1245
   Ile Lys Phe Arg Cys Phe Thr Ala Pro Arg Phe Cys Pro Asp Met Asn
   1250 1255 1260
   Val Lys Tyr Ile Asn Ile
   1265 1270
<210> 108
   <211> 1094
   <212> PRT
   <213> Drosophila sp.
<400> 108
   Met Asn Ser Ala Val Asp Ala Trp Ala Val Thr Pro Arg Glu Arg Leu
   1 5 10 15
   Lys Tyr Gln Glu Gln Phe Arg Ala Leu Gln Pro Gln Ala Gly Phe Val
   20 25 30
   Thr Gly Ala Gln Ala Lys Gly Phe Phe Leu Gln Ser Gln Leu Pro Pro
   35 40 45
   Leu Ile Leu Gly Gln Ile Trp Ala Leu Ala Asp Thr Asp Ser Asp Gly
   50 55 60
   Lys Met Asn Ile Asn Glu Phe Ser Ile Ala Cys Lys Leu Ile Asn Leu
   65 70 75 80
   Lys Leu Arg Gly Met Asp Val Pro Lys Val Leu Pro Pro Ser Leu Leu
   85 90 95
   Ser Ser Leu Thr Gly Asp Val Pro Ser Met Thr Pro Arg Gly Ser Thr
   100 105 110
   Ser Ser Leu Ser Pro Leu Asp Pro Leu Lys Gly Ile Val Pro Ala Val
   115 120 125
   Ala Pro Val Val Pro Val Val Ala Pro Pro Val Ala Val Ala Thr Val
   130 135 140
   Ile Ser Pro Pro Gly Val Ser Val Pro Ser Gly Pro Thr Pro Pro Thr
   145 150 155 160
   Ser Asn Pro Pro Ser Arg His Thr Ser Ile Ser Glu Arg Ala Pro Ser
   165 170 175
   Ile Glu Ser Val Asn Gln Gly Glu Trp Ala Val Gln Ala Ala Gln Lys
   180 185 190
   Arg Lys Tyr Thr Gln Val Phe Asn Ala Asn Asp Arg Thr Arg Ser Gly
   195 200 205
   Tyr Leu Thr Gly Ser Gln Ala Arg Gly Val Leu Val Gln Ser Lys Leu
   210 215 220
   Pro Gln Val Thr Leu Ala Gln Ile Trp Thr Leu Ser Asp Ile Asp Gly
   225 230 235 240
   Asp Gly Arg Leu Asn Cys Asp Glu Phe Ile Leu Ala Met Phe Leu Cys
   245 250 255
   Glu Lys Ala Met Ala Gly Glu Lys Ile Pro Val Thr Leu Pro Gln Glu
   260 265 270
   Trp Val Pro Pro Asn Leu Arg Lys Ile Lys Ser Arg Pro Gly Ser Val
   275 280 285
   Ser Gly Val Val Ser Arg Pro Gly Ser Gln Pro Ala Ser Arg His Ala
   290 295 300
   Ser Val Ser Ser Gln Ser Gly Val Gly Val Val Asp Ala Asp Pro Thr
   305 310 315 320
   Ala Gly Leu Pro Gly Gln Thr Ser Phe Glu Asp Lys Arg Lys Glu Asn
   325 330 335
   Tyr Val Lys Gly Gln Ala Glu Leu Asp Arg Arg Arg Lys Ile Met Glu
   340 345 350
   Asp Gln Gln Arg Lys Glu Arg Glu Glu Arg Glu Arg Lys Glu Arg Glu
   355 360 365
   Glu Ala Asp Lys Arg Glu Lys Ala Arg Leu Glu Ala Glu Arg Lys Gln
   370 375 380
   Gln Glu Glu Leu Glu Arg Gln Leu Gln Arg Gln Arg Glu Ile Glu Met
   385 390 395 400
   Glu Lys Glu Glu Gln Arg Lys Arg Glu Leu Glu Ala Lys Glu Ala Ala
   405 410 415
   Arg Lys Glu Leu Glu Lys Gln Arg Gln Gln Glu Trp Glu Gln Ala Arg
   420 425 430
   Ile Ala Glu Met Asn Ala Gln Lys Glu Arg Glu Gln Glu Arg Val Leu
           435                 440                 445
   Lys Gln Lys Ala His Asn Thr Gln Leu Asn Val Glu Leu Ser Thr Leu
       450                 455                 460
   Asn Glu Lys Ile Lys Glu Leu Ser Gln Arg Ile Cys Asp Thr Arg Ala
   465                 470                 475                 480
   Gly Val Thr Asn Val Lys Thr Val Ile Asp Gly Met Arg Thr Gln Arg
                   485                 490                 495
   Asp Thr Ser Met Ser Glu Met Ser Gln Leu Lys Ala Arg Ile Lys Glu
               500                 505                 510
   Gln Asn Ala Lys Leu Leu Gln Leu Thr Gln Glu Arg Ala Lys Trp Glu
           515                 520                 525
   Ala Lys Ser Lys Ala Ser Gly Ala Ala Leu Gly Gly Glu Asn Ala Gln
       530                 535                 540
   Gln Glu Gln Leu Asn Ala Ala Phe Ala His Lys Gln Leu Ile Ile Asn
   545                 550                 555                 560
   Gln Ile Lys Asp Lys Val Glu Asn Ile Ser Lys Glu Ile Glu Ser Lys
                   565                 570                 575
   Lys Glu Asp Ile Asn Thr Asn Asp Val Gln Met Ser Glu Leu Lys Ala
               580                 585                 590
   Glu Leu Ser Ala Leu Ile Thr Lys Cys Glu Asp Leu Tyr Lys Glu Tyr
           595                 600                 605
   Asp Val Gln Arg Thr Ser Val Leu Glu Leu Lys Tyr Asn Arg Lys Asn
       610                 615                 620
   Glu Thr Ser Val Ser Ser Ala Trp Asp Thr Gly Ser Ser Ser Ala Trp
   625                 630                 635                 640
   Glu Glu Thr Gly Thr Thr Val Thr Asp Pro Tyr Ala Val Ala Ser Asn
                   645                 650                 655
   Asp Ile Ser Ala Leu Ala Ala Pro Ala Val Asp Leu Gly Gly Pro Ala
               660                 665                 670
   Pro Glu Gly Phe Val Lys Tyr Gln Ala Val Tyr Glu Phe Asn Ala Arg
           675                 680                 685
   Asn Ala Glu Glu Ile Thr Phe Val Pro Gly Asp Ile Ile Leu Val Pro
   690 695 700
   Leu Glu Gln Asn Ala Glu Pro Gly Trp Leu Ala Gly Glu Ile Asn Gly
   705 710 715 720
   His Thr Gly Trp Phe Pro Glu Ser Tyr Val Glu Lys Leu Glu Val Gly
   725 730 735
   Glu Val Ala Pro Val Ala Ala Val Glu Ala Pro Val Asp Ala Gln Val
   740 745 750
   Ala Asp Thr Tyr Asn Asp Asn Ile Asn Thr Ser Ser Ile Pro Ala Ala
   755 760 765
   Ser Ala Asp Leu Thr Ala Ala Gly Asp Val Glu Tyr Tyr Ile Ala Ala
   770 775 780
   Tyr Pro Tyr Glu Ser Ala Glu Glu Gly Asp Leu Ser Phe Ser Ala Gly
   785 790 795 800
   Glu Met Val Met Val Ile Lys Lys Glu Gly Glu Trp Trp Thr Gly Thr
   805 810 815
   Ile Gly Ser Arg Thr Gly Met Phe Pro Ser Asn Tyr Val Gln Lys Ala
   820 825 830
   Asp Val Gly Thr Ala Ser Thr Ala Ala Ala Glu Pro Val Glu Ser Leu
   835 840 845
   Asp Gln Glu Thr Thr Leu Asn Gly Asn Ala Ala Tyr Thr Ala Ala Pro
   850 855 860
   Val Glu Ala Gln Glu Gln Val Tyr Gln Pro Leu Pro Val Gln Glu Pro
   865 870 875 880
   Ser Glu Gln Pro Ile Ser Ser Pro Gly Val Gly Ala Glu Glu Ala His
   885 890 895
   Glu Asp Leu Asp Thr Glu Val Ser Gln Ile Asn Thr Gln Ser Lys Thr
   900 905 910
   Gln Ser Ser Glu Pro Ala Glu Ser Tyr Ser Arg Pro Met Ser Arg Thr
   915 920 925
   Ser Ser Met Thr Pro Gly Met Arg Ala Lys Arg Ser Glu Ile Ala Gln
   930 935 940
   Val Ile Ala Pro Tyr Glu Ala Thr Ser Thr Glu Gln Leu Ser Leu Thr
   945 950 955 960
   Arg Gly Gln Leu Ile Met Ile Arg Lys Lys Thr Asp Ser Gly Trp Trp
   965 970 975
   Glu Gly Glu Leu Gln Ala Lys Gly Arg Arg Arg Gln Ile Gly Trp Phe
   980 985 990
   Pro Ala Thr Tyr Val Lys Val Leu Gln Gly Gly Arg Asn Ser Gly Arg
   995 1000 1005
   Asn Thr Pro Val Ser Gly Ser Arg Ile Glu Met Thr Glu Gln Ile Leu
   1010 1015 1020
   Asp Lys Val Ile Ala Leu Tyr Pro Tyr Lys Ala Gln Asn Asp Asp Glu
   1025 1030 1035 1040
   Leu Ser Phe Asp Lys Asp Asp Ile Ile Ser Val Leu Gly Arg Asp Glu
   1045 1050 1055
   Pro Glu Trp Trp Arg Gly Glu Leu Asn Gly Leu Ser Gly Leu Phe Pro
   1060 1065 1070
   Ser Asn Tyr Val Gly Pro Phe Val Thr Ser Gly Lys Pro Ala Lys Ala
   1075 1080 1085
   Asn Gly Thr Thr Lys Lys
   1090
<210> 109
   <211> 520
   <212> PRT
   <213> Homo sapiens
<400> 109
   Met Glu Ala Glu Arg Leu Lys Gln Lys Glu Gln Glu Arg Lys Ile Ile
   1 5 10 15
   Glu Leu Glu Lys Gln Lys Glu Glu Ala Gln Arg Arg Ala Gln Glu Arg
   20 25 30
   Asp Lys Gln Trp Leu Glu His Val Gln Gln Glu Asp Glu His Gln Arg
   35 40 45
   Pro Arg Lys Leu His Glu Glu Glu Lys Leu Lys Arg Glu Glu Ser Val
   50 55 60
   Lys Lys Lys Asp Gly Glu Glu Lys Gly Lys Gln Glu Ala Gln Asp Lys
   65 70 75 80
   Leu Gly Arg Leu Phe His Gln His Gln Glu Pro Ala Lys Pro Ala Val
   85 90 95
   Gln Ala Pro Trp Ser Thr Ala Glu Lys Gly Pro Leu Thr Ile Ser Ala
   100 105 110
   Gln Glu Asn Val Lys Val Val Tyr Tyr Arg Ala Leu Tyr Pro Phe Glu
   115 120 125
   Ser Arg Ser His Asp Glu Ile Thr Ile Gln Pro Gly Asp Ile Val Met
   130 135 140
   Val Asp Glu Ser Gln Thr Gly Glu Pro Gly Trp Leu Gly Gly Glu Leu
   145 150 155 160
   Lys Gly Lys Thr Gly Trp Phe Pro Ala Asn Tyr Ala Glu Lys Ile Pro
   165 170 175
   Glu Asn Glu Val Pro Ala Pro Val Lys Pro Val Thr Asp Ser Thr Ser
   180 185 190
   Ala Pro Ala Pro Lys Leu Ala Leu Arg Glu Thr Pro Ala Pro Leu Ala
   195 200 205
   Val Thr Ser Ser Glu Pro Ser Thr Thr Pro Asn Asn Trp Ala Asp Phe
   210 215 220
   Ser Ser Thr Trp Pro Thr Ser Thr Asn Glu Lys Pro Glu Thr Asp Asn
   225 230 235 240
   Trp Asp Ala Trp Ala Ala Gln Pro Ser Leu Thr Val Pro Ser Ala Gly
   245 250 255
   Gln Leu Arg Gln Arg Ser Ala Phe Thr Pro Ala Thr Ala Thr Gly Ser
   260 265 270
   Ser Pro Ser Pro Val Leu Gly Gln Gly Glu Lys Val Glu Gly Leu Gln
   275 280 285
   Ala Gln Ala Leu Tyr Pro Trp Arg Ala Lys Lys Asp Asn His Leu Asn
   290 295 300
   Phe Asn Lys Asn Asp Val Ile Thr Val Leu Glu Gln Gln Asp Met Trp
   305 310 315 320
   Trp Phe Gly Glu Val Gln Gly Gln Lys Gly Trp Phe Pro Lys Ser Tyr
                   325                 330                 335
   Val Lys Leu Ile Ser Gly Pro Ile Arg Lys Ser Thr Ser Met Asp Ser
               340                 345                 350
   Gly Ser Ser Glu Ser Pro Ala Ser Leu Lys Arg Val Ala Ser Pro Ala
           355                 360                 365
   Ala Lys Pro Val Val Ser Gly Glu Glu Ile Ala Gln Val Ile Ala Ser
       370                 375                 380
   Tyr Thr Ala Thr Gly Pro Glu Gln Leu Thr Leu Ala Pro Gly Gln Leu
   385                 390                 395                 400
   Ile Leu Ile Arg Lys Lys Asn Pro Gly Gly Trp Trp Glu Gly Glu Leu
                   405                 410                 415
   Gln Ala Arg Gly Lys Lys Arg Gln Ile Gly Trp Phe Pro Ala Asn Tyr
               420                 425                 430
   Val Lys Leu Leu Ser Pro Gly Thr Ser Lys Ile Thr Pro Thr Glu Pro
           435                 440                 445
   Pro Lys Ser Thr Ala Leu Ala Ala Val Cys Gln Val Ile Gly Met Tyr
       450                 455                 460
   Asp Tyr Thr Ala Gln Asn Asp Asp Glu Leu Ala Phe Asn Lys Gly Gln
   465                 470                 475                 480
   Ile Ile Asn Val Leu Asn Lys Glu Asp Pro Asp Trp Trp Lys Gly Glu
                   485                 490                 495
   Val Asn Gly Gln Val Gly Leu Phe Pro Ser Asn Tyr Val Lys Leu Thr
               500                 505                 510
   Thr Asp Met Asp Pro Ser Gln Gln
           515                 520

## Description

### RESEARCH SUPPORT

The research leading to the present invention was supported in part by the Clinical Molecular. Core grant NICHD P01HD17449 from the National Institutes of Health. The government may have certain rights in the present invention.

### FIELD OF THE INVENTION

The present invention relates to the isolated nucleic acids and corresponding amino acids of an SH3 gene. The invention provides polypeptides, fusion proteins, chimerics, antisense molecules, and uses thereof. Also, this invention is directed to diagnostic methods of determining whether a subject has a megakaryocytic abnormality, myeloproliferative disorder, platelet disorder, hematopoietic disorder, or leukemia, or disorders associated with abnormal neural development, and therapeutic treatments thereof.

### BACKGROUND OF THE INVENTION

Down syndrome, caused by trisomy of human chromosome 21 (HSA21), is the most common autosomal form of mental retardation. The first report describing an association between Down syndrome (DS) and leukemia, which are an important cause of morbidity and mortality worldwide, was presented in 1930. Since that time, the increased incidence of acute leukemia in patients with DS has been clearly established. However, the M7 subtype. AMKL. acute megakaryoblastic leukemia has been found to be common in DS but relatively rare in non-DS. An instability in the control of bone marrow proliferation has been hypothesized as a predisposing factor. The incidence of acute myelogenous leukemia patients with DS has been noted by some to be similar to that in children without mongolism. Chromosome 21 is a model for the study of human chromosomal aneuploidy, and the construction of its physical and transcriptional maps is a necessary step in understanding the molecular basis of aneuploidy-dependent phenotypes.

Human chromosome 21 has a nearly complete physical map with a well-characterized contiguous set of overlapping YACs spanning most of its length (Chumakov et al., 1992; Shimizu et al., 1995; Korenberg et al., 1995). The demand for sequence-ready contigs and clones for gene isolation efforts has prompted the construction of numerous higher resolution contigs in cosmids (Patil et al., 1994; Soeda et al., 1995) and, more recently, in P1-derived artificial chromosomes (PACs; Oegawa et al. 1996 and Hubert et al. (1997) Genomics 41:218-226). Considerable mapping efforts exist in the region from CBR to D21S55 due to the common duplication of the region in partially trisomic individuals with several phenotypic features of DS, including mental retardation. However, the distal and adjacent, 4- to 5-Mb D21S55 to MX1 region is also associated with DS-CHD as well as other characteristic features of DS (Korenberg et al., 1992, 1994).

Although full monosomy of chromosome 21 is usually lethal *in utero,* there are rare cases of individuals with chromosome 21 deletions who survive. These individuals exhibit a characteristic subset of clinical features including psychomotor and growth retardation, congenital heart disease, holoprosencephaly, microphthalmia, skeletal malformations, and genital hypoplasia. Megakaryocytic abnormalities is added to this set and define a minimal "overlap" region for this feature through the clinical, cytogenetic, and molecular analysis of four patients with overlapping deletions of chromosome 21 and thrombocytopenia.

Nonchimeric YACs span this interval with a few gaps but higher resolution physical maps are not available for most of the D21S55 to MX1 region. DEL21RW carries two interstitial deletions, one in 21q21.3-22.1 defined by YAC 62G5 through YAC 760H5, and the second in 21q22.2, deleting IFNAR through CBR. DEL21LS carries an interstitial deletion of 21q22.1 from YAC 760H5 through the AML1 gene. Korenberg et al. reported that the deletion of patient DEL21HJ includes D21S93 through AML1. DEL21SV has a possible terminal deletion, 21q22.13-qter, extending from just proximal to D21S324 through D21S123. The common deleted region, or overlap region, is therefore from D21S324 through AML1, a region of less than 2Mb that contains only three known genes, AML1, KCNE1, and UN02. Bone marrow examination of two of the patients, DEL21HJ and Del 21RW, showed normocellular marrow with normal myelopoiesis, normal erythropoiesis, and small, dysplastic megakaryocytes with hypolobated nuclei. These two patients have decreased platelet activation by agonists with normal platelet ultrastructures. All four patients have platelet dysfunction characterized by low platelet counts in the range of 31-113 x 10⁹ /L. Further, all four subjects with chromosome 21 deletions that do not include this region have normal number of platelets.

A 3' fragment of SH3P17 gene was found in a study to isolate SH3 domain contain ing genes (Sparks et al. 1996, *Nature Biotechnology* 14:741). This was mapped to 21 or large sub-region of 21 by a number of groups by using database matches to the published sequence. Katsanis N, et al (Hum Genet 1997 Sep;100(3-4):477-480) utilized information generated by various EST sequencing projects to enrich the transcription map of chromosome 21 and report the mapping of SH3P17 to 21q22.1 and the localisation of two genes previously mapped to HSA21 by Nagase and colleagues, KIAA0136 and KIAA0179 to 21q22.2 and 21q22.3 respectively. Chen H, and Antonarakis SE (Cytogenet Cell Genet 1997;78(3-4):213-215) identified portions of genes on human chromosome 21 and mapped the gene to YACs and cosmids within 21q22.1-->q22.2 between DNA markers D21S319 and D21S65 using hybridization and PCR amplification. Lastly, Guipponi *et. al.* 1998, *Genomics* 53:369-376 reported that they identified two isoforms of the human homolog of *Xenopus* Intersectin (ITSN) produced from alternate transcripts, the first of which, a short transcript is reportedly ubiquitously expressed, while the second longer transcript is exclusively expressed in brain tissue. Later, Guipponi *et. al.* 1998 *Cytogenet Cell Genet.*
83:218-220 reported that they had identified the genomic structure, sequence and precise mapping of the human intersectin gene and speculated that it may play a role in the determination of certain of the phenotypic characteristics of Down syndrome. The authors did not present evidence and corresponding observations or speculation regarding the role of the discovered genes apart from a possible relation to Down syndrome, and as such, are distinguishable from the research and discoveries embodied in the present invention.

The present invention provides the complete nucleotide sequence of several SH3 genes, including the SH3D1A gene and clones thereof, their association with platelet dysfunction and leukemia, including a part of the increased risk of leukemia seen in Down Syndrome, and with dysfunctions associated with neural development and particularly development in the CNS.

### SUMMARY OF THE INVENTION

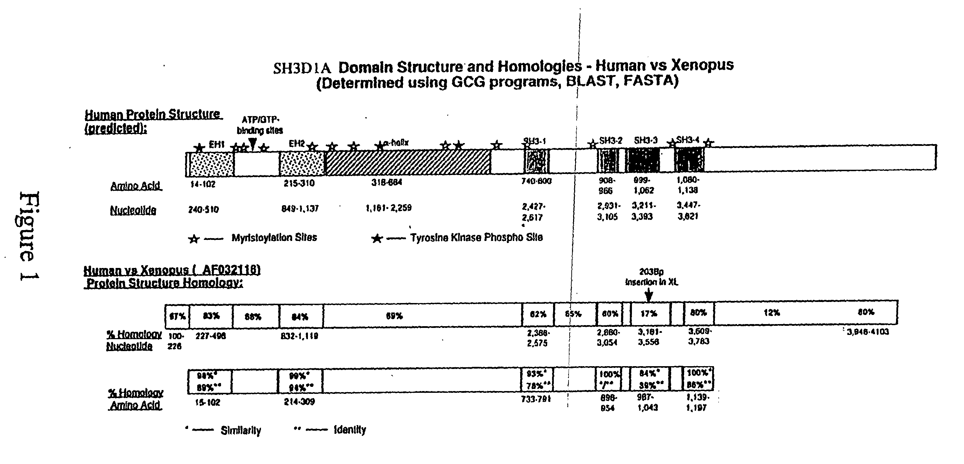

In one embodiment, this invention provides an isolated nucleic acid which encodes the human SH3 gene SH3D1A and cDNA clones thereof. This invention is directed to an isolated nucleic acid encoding an amino acid sequence which forms one or more myristoylation sites in the EH domain and SH3 domain. This invention provides an isolated nucleic acid encoding an amino acid sequence which forms one or more EH domains and one or more SH3 domains. In one embodiment the nucleic acid which encodes an amino acid sequence which forms two EH domains and four SH3 domains. As shown in Figure 1 the nucleic acid encoding the amino acid sequence comprises one or more myristoylation sites in the EH domain and SH3 domain.

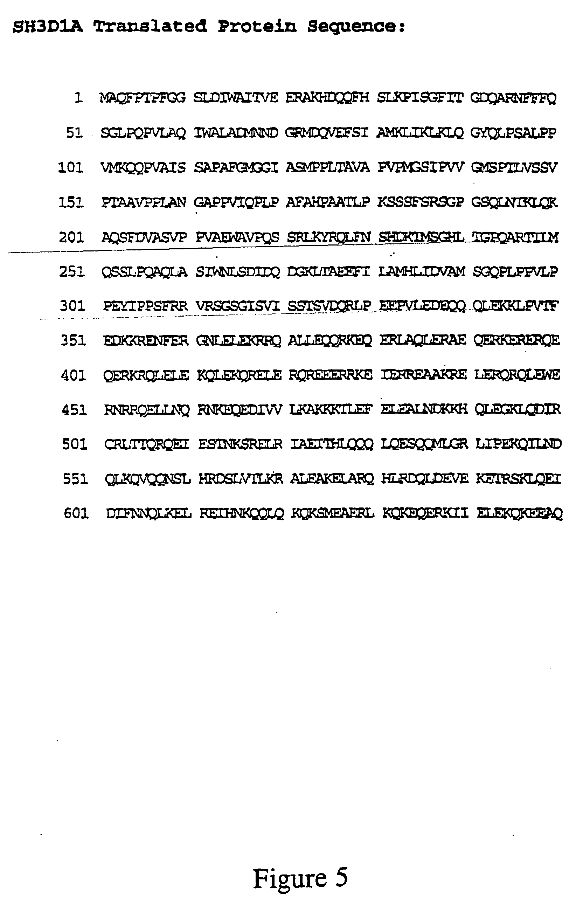

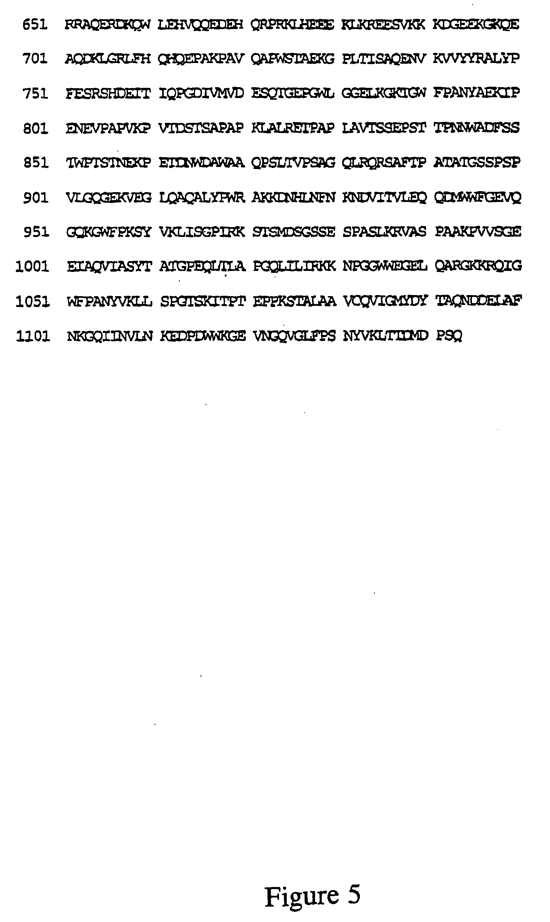

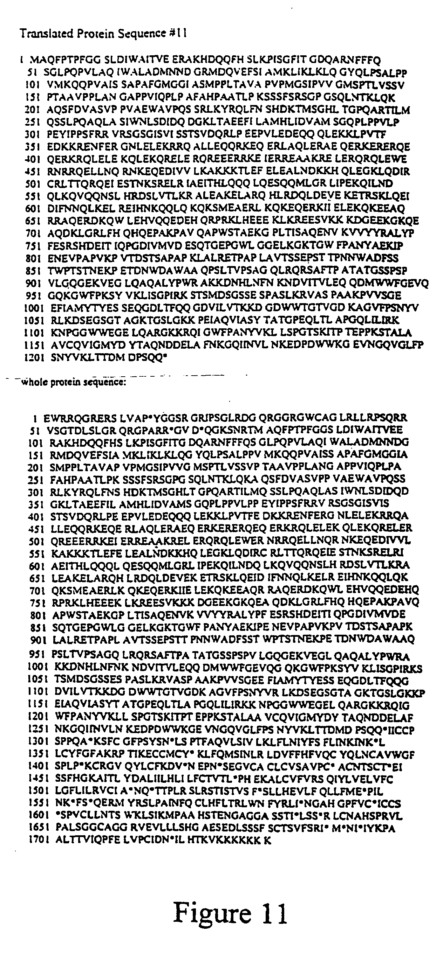

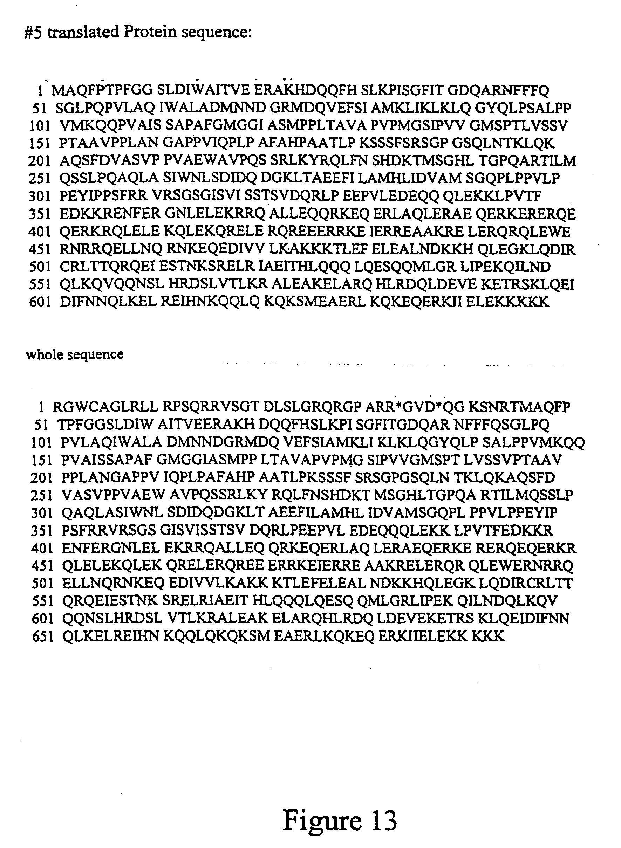

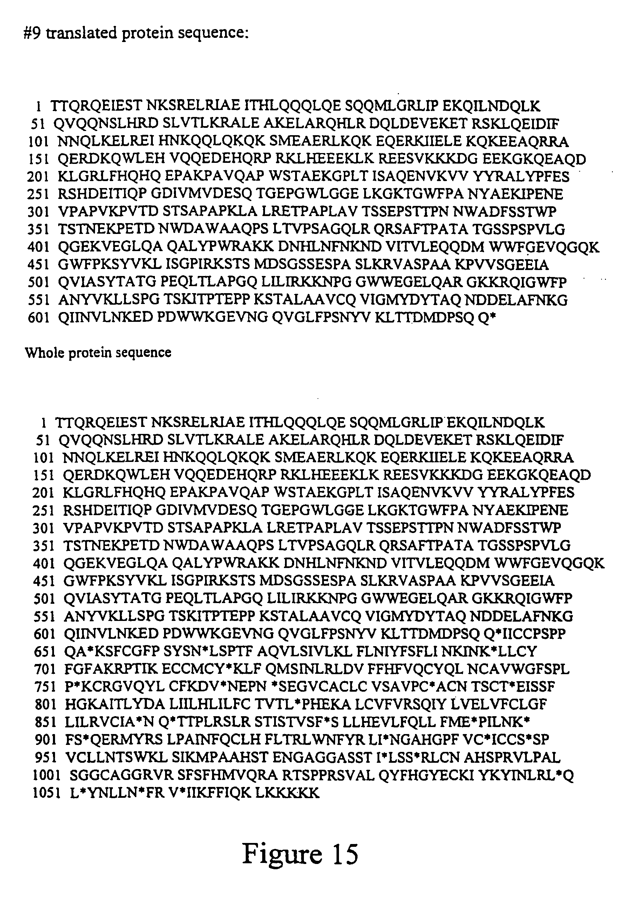

The nucleic acid encodes an amino acid sequence as set forth in SEQ. ID. NO. 2, and as set forth in Figures 5, 9, 11, 13 and 15.

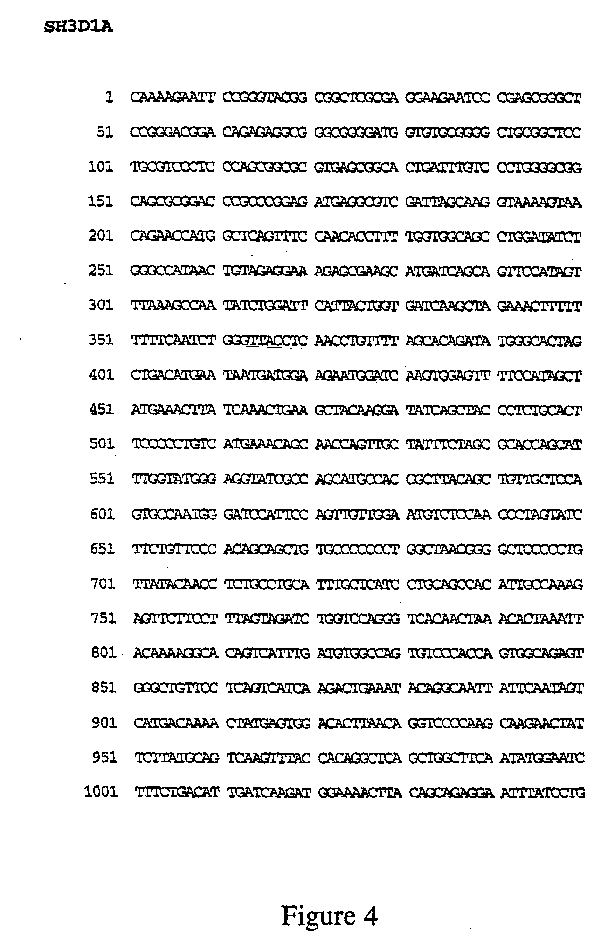

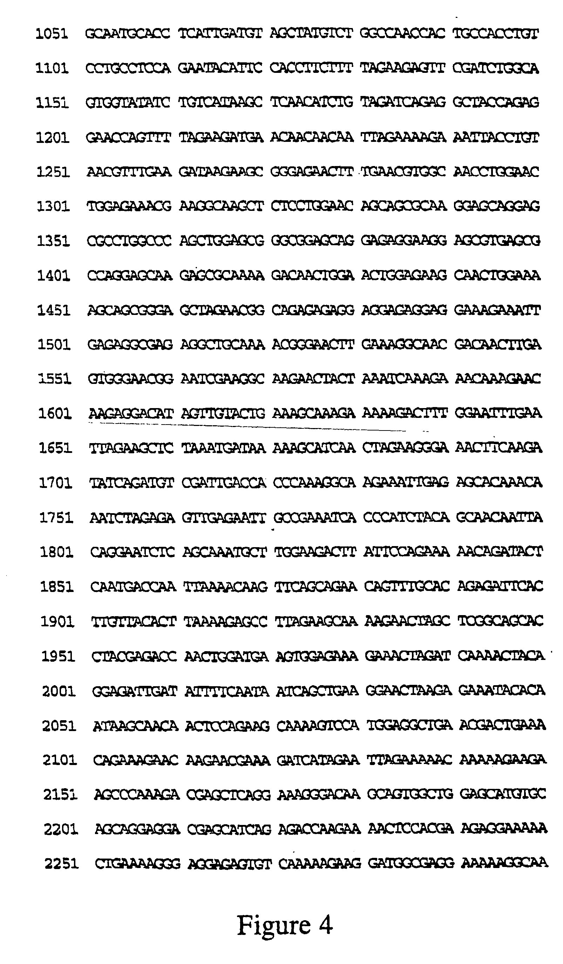

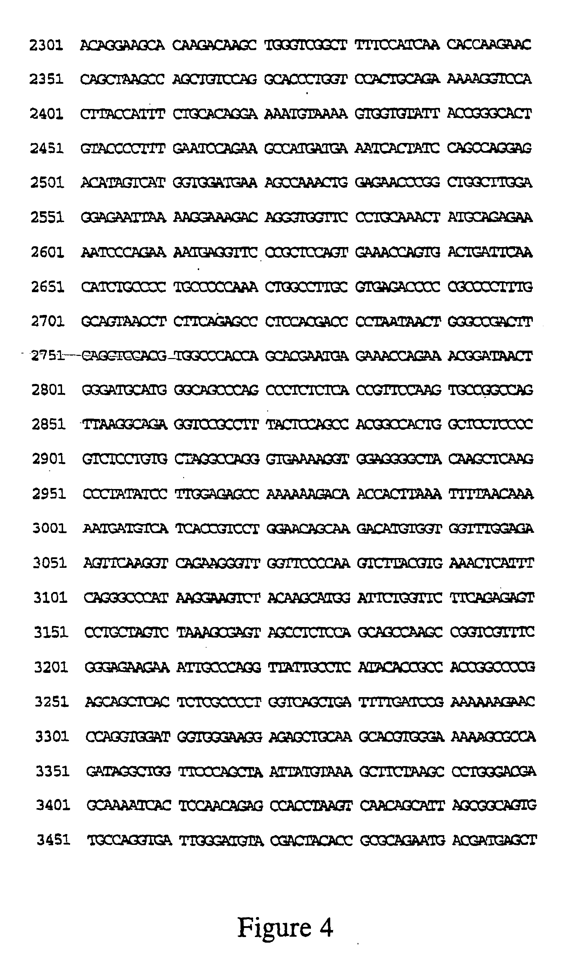

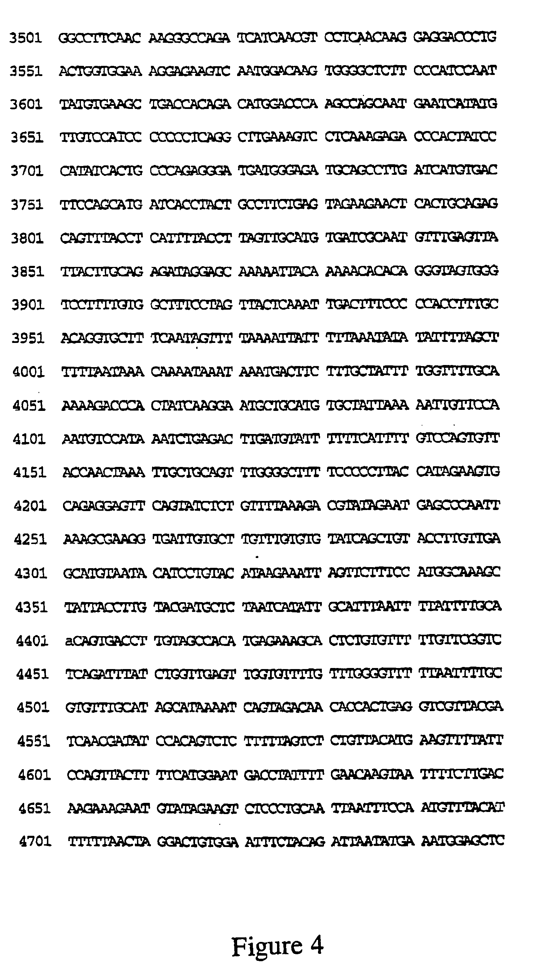

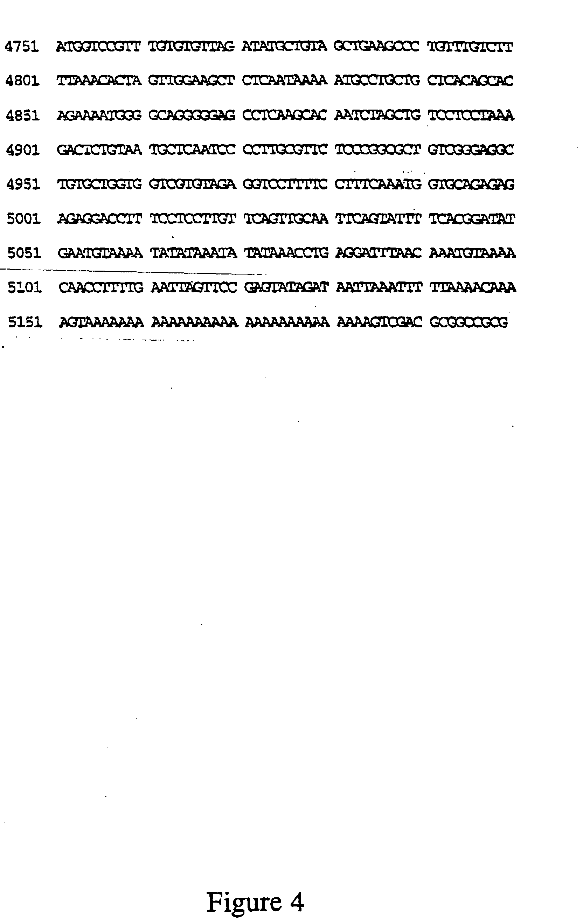

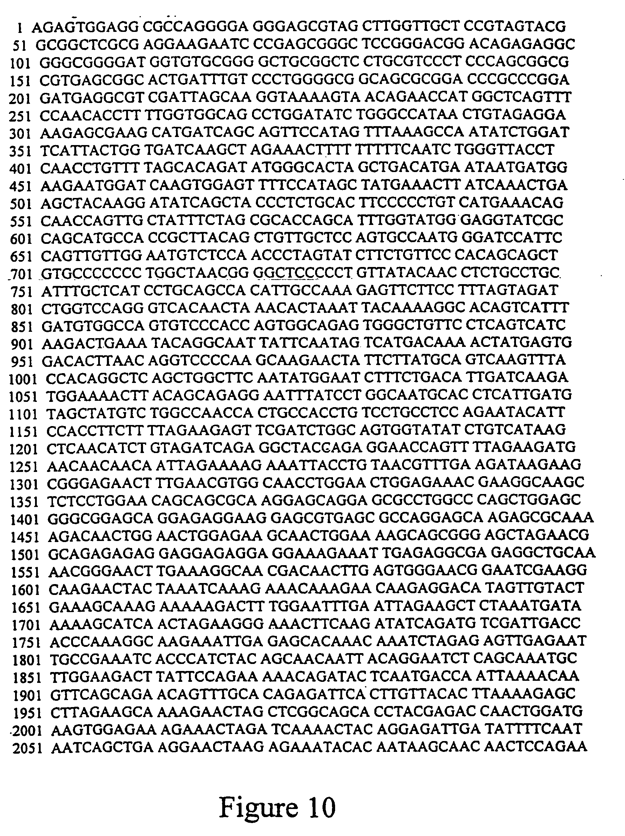

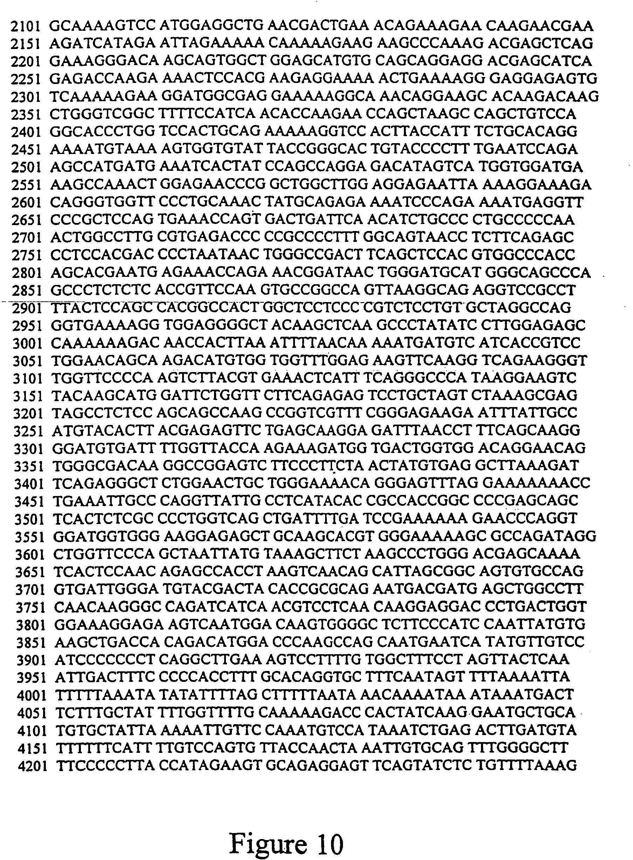

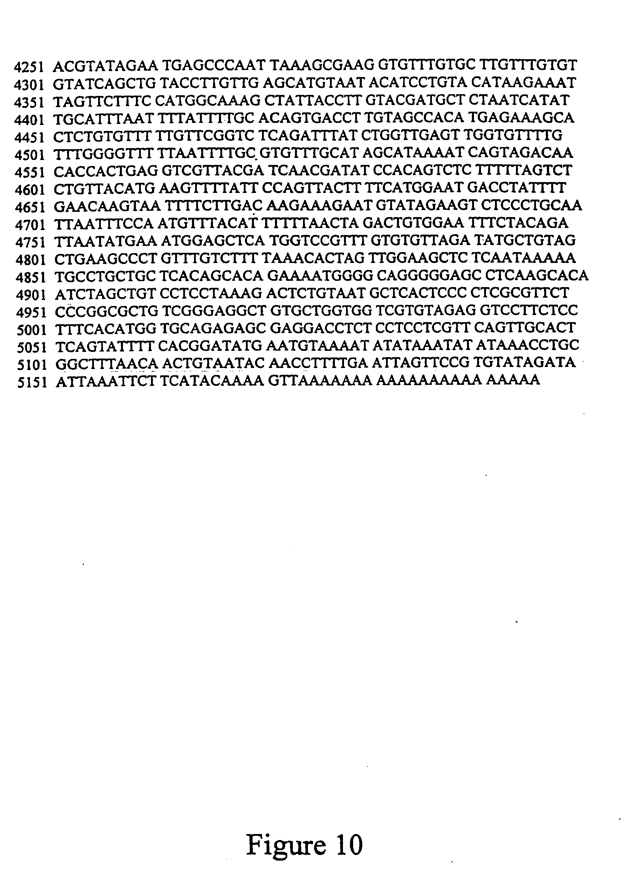

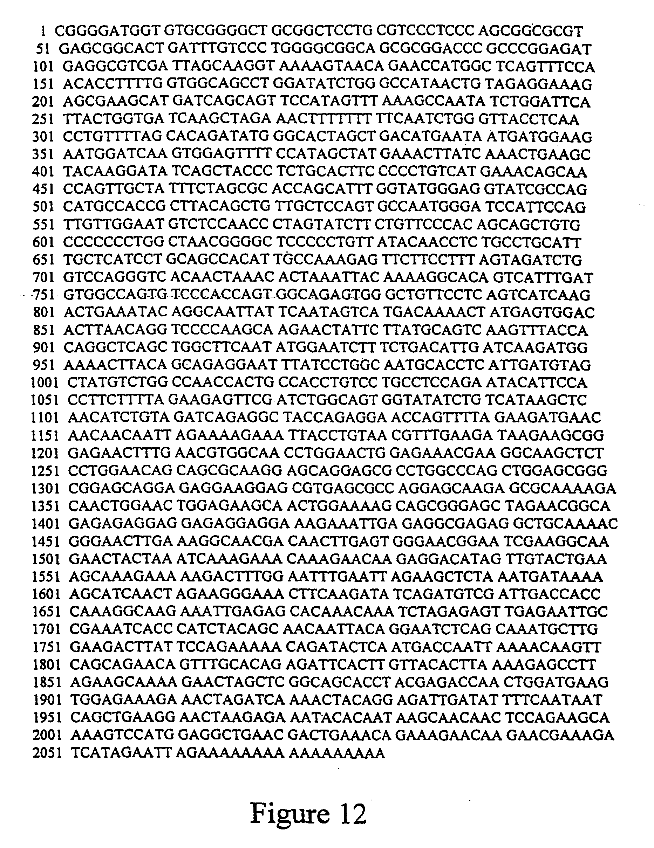

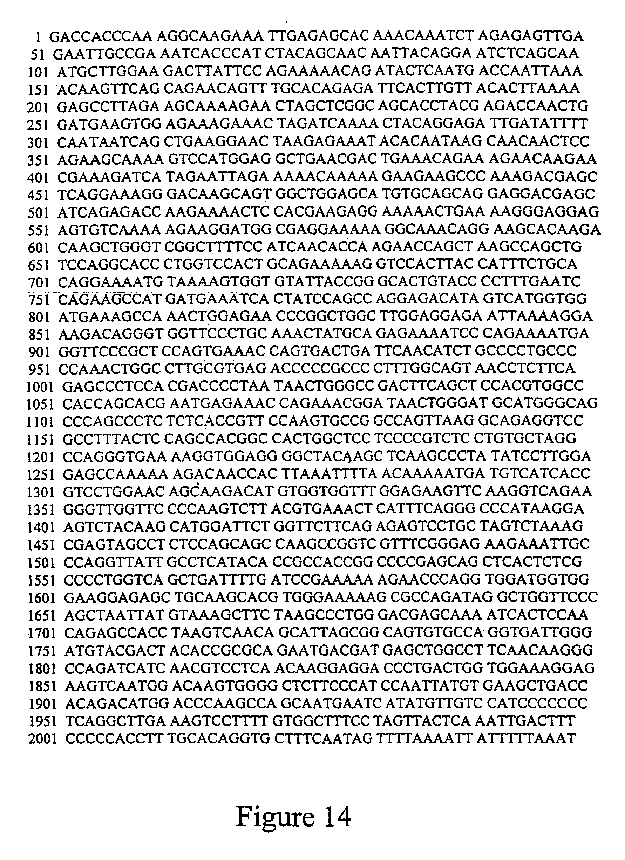

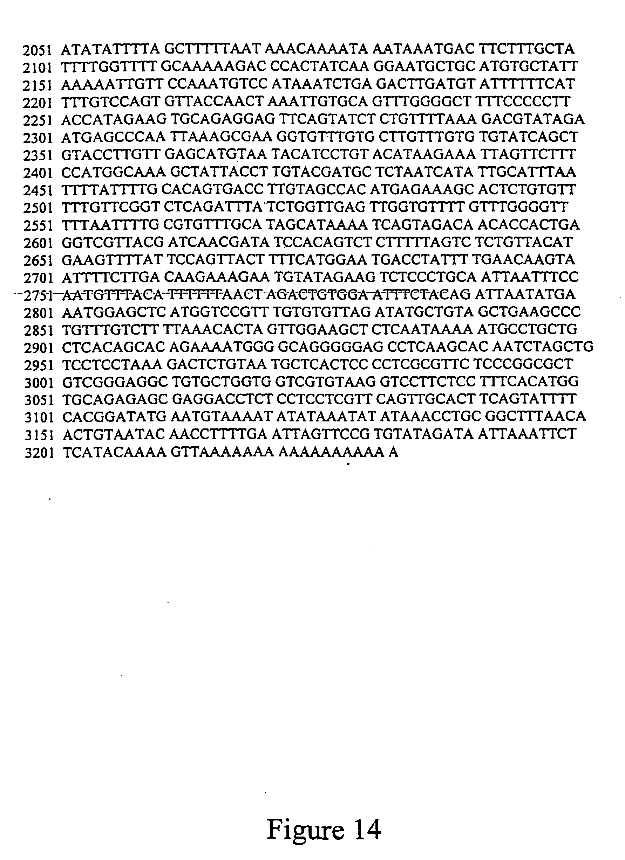

This invention provides for an isolated nucleic acid which encodes SH3D1A, and clones thereof as set forth herein. The acid may be DNA or RNA, specifically cDNA or genomic DNA. This isolated nucleic acid also encodes mutant SH3D1A or the wildtype protein. The isolated nucleic acid may also encode a human SH3D 1A having substantially the same amino acid sequence as the sequence designated Figure 5. As used herein and in the claims, the terms nucleic acids encoding or expressing SH3D1A is intended to comprehend and include isolated nucleic acids that may have the sequence set forth in Figures 4, 8, 10, 12 or 14.

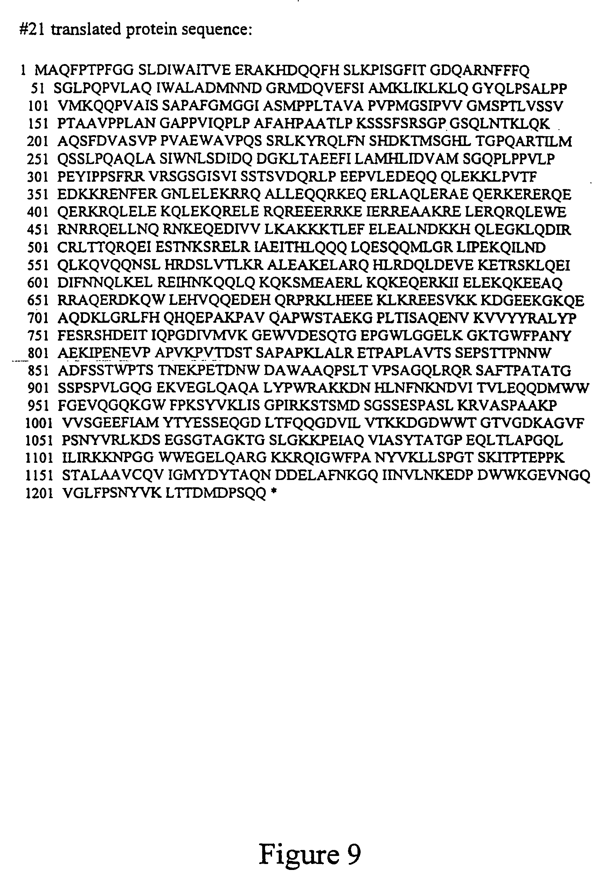

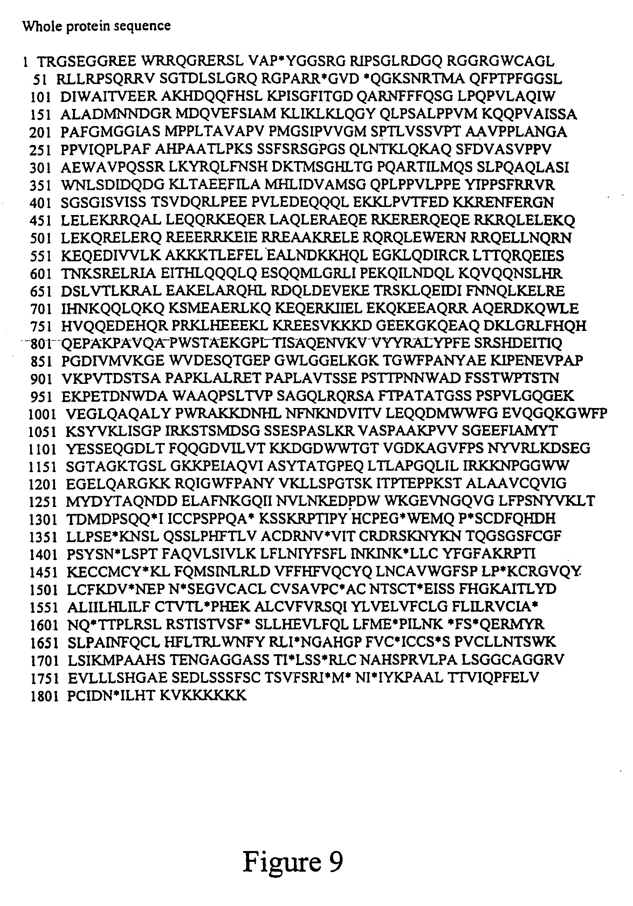

This invention is directed to a polypeptide comprising the amino acid sequence of a human SH3D1A or to a clone thereof. As used herein and in the claims, polypeptide or protein of SH3D1A is intended to comprehend and include polypeptides that comprise or otherwise correspond to those set forth in Figures 9, 11, 13, or 15 herein. Further, polyclonal and monoclonal antibodies which specifically bind to the polypeptide are disclosed and chimeric (bi-specific) antibodies are likewise contemplated.

This invention provides a method for determining whether a subject carries a mutation in the SH3D1A gene which comprises: (a) obtaining an appropriate nucleic acid sample from the subject; and (b) determining whether the nucleic acid sample from step (a) is, or is derived from, a nucleic acid which encodes mutant SH3D1A so as to thereby determine whether a subject carries a mutation in the SH3D1A gene.

This invention provides a method for determining whether a subject has a megakaryocytic abnormality, myeloproliferative disorder, platelet disorder, or leukemia, or a neural disorder which comprises: (a) obtaining an appropriate sample from the subject; and (b) contacting the sample with the antibody so as to thereby determine whether a subject has the megakaryocytic abnormality, myeloproliferative disorder, platelet disorder, leukemia or neural disorder.

This invention provides a method for determining whether a subject has a predisposition for a megakaryocytic abnormality, myeloproliferative disorder, platelet disorder, leukemia, or a neural disorder, which comprises: (a) obtaining an appropriate nucleic acid sample from the subject; and (b) determining whether the nucleic acid sample from step (a) is, or is derived from, a nucleic acid which encodes SH3D1A so as to thereby determine whether a subject has a predisposition for a megakaryocytic abnormality, myeloproliferative disorder, platelet disorder or leukemia, or a neural disorder.

This invention provides a method for determining whether a subject has a megakaryocytic abnormality, myeloproliferative disorder, platelet disorder, leukemia, or a neural disorder, which comprises: (a) obtaining an appropriate nucleic acid sample from the subject; and (b) determining whether the nucleic acid sample from step (a) is, or is derived from, a nucleic acid which encodes the human SH3D1A so as to thereby determine whether a subject has megakaryocytic abnormality, myeloproliferative disorder, platelet disorder, leukemia, or a neural disorder.

This invention provides a method for screening a tumor sample from a human subject for a somatic alteration in a SH3D1A gene in said tumor which comprises gene comparing a first sequence selected form the group consisting of a SH3D1A gene from said tumor sample, SH3D1A RNA from said tumor sample and SH3D1A cDNA made from mRNA from said tumor sample with a second sequence selected from the group consisting of SH3D1A gene from a nontumor sample of said subject, SH3D1A RNA from said nontumor sample and SH3D1A cDNA made from mRNA from said nontumor sample, wherein a difference in the sequence of the SH3D1A gene, SH3D1A RNA or SH3D1A cDNA from said tumor sample from the sequence of the SH3D1A gene, SH3D1A RNA or SH3D1A cDNA from said nontumor sample indicates a somatic alteration in the SH3D1A gene in said tumor sample.

This invention provides an ex vivo method for monitoring the progress and adequacy of treatment in a subject who has received treatment for a megakaryocytic abnormality, myeloproliferative disorder, platelet disorder, leukemia or an abnormal neural condition which comprises monitoring the level of nucleic acid encoding the human SH3D1A at various stages of treatment.

The present invention provides the means necessary for production of gene-based therapies directed at cancer cells; diagnosis of the predisposition to, and diagnosis and treatment, of megakaryocytic abnormality, hematopoietic disorders, myeloproliferative disorder platelet disorder, Down Syndrome, leukemia, other disorders based in whole or in part from neural abnormalities or dysfunctions and prenatal diagnosis and treatment of tumors. These therapeutic agents may take the form of polynucleotides comprising all or a portion of the SH3D1A gene placed in appropriate vectors or delivered to target cells in more direct ways such that the function of the SH3D1A protein is reconstituted. Therapeutic agents may also take the form of polypeptides based on either a portion of, or the entire protein sequence of SH3D1A.

This invention provides a pharmaceutical composition comprising an amount of the polypeptide of the human SH3D1A as defined herein, and a pharmaceutically effective carrier or diluent.

The invention also provides claim 29.

### BRIEF DESCRIPTION OF THE DRAWINGS

- **FIGURE 1.**: Human SH3D1A structure and homology
- **FIGURE 2.**: SH3D1A domain structure and homologies - human vs. Xenopus
- **FIGURE 3.**: Region of chromosome 21 responsible for megakaryocytic abnormalities.
- **FIGURE 4.**: Nucleic acid sequence of haman SH3D1A.
- **FIGURE 5.**: Amino acid sequence of human SH3D1A.
- **FIGURE 6.**: Northern Blot of SH3D1A expressed in heart, brain, placenta, lung, liver, muscle, kidney and pancreas.

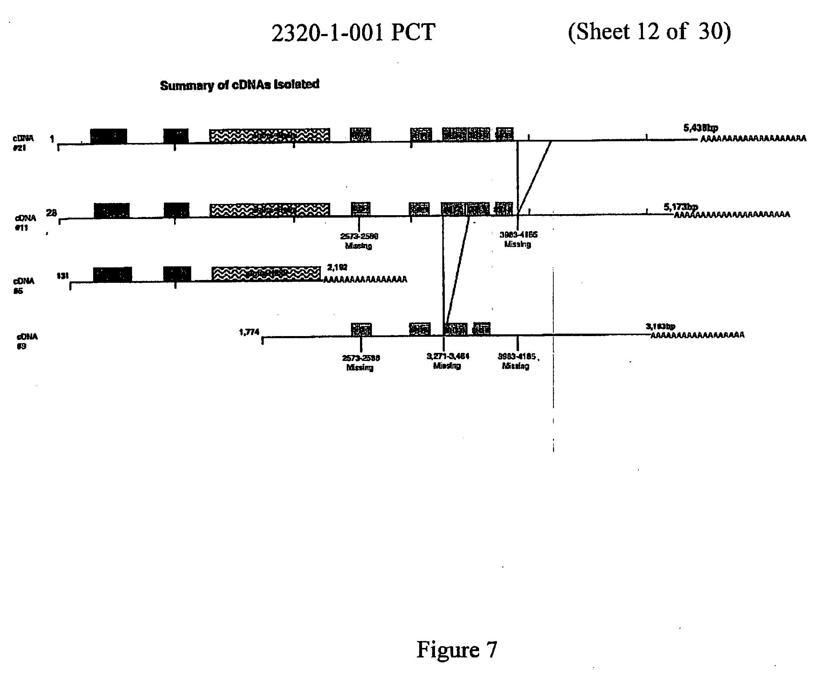

- **FIGURE 7.**: Map presenting four cDNA clones in accordance with the invention, including length and protein domains.
- **FIGURE 8.**: Nucleic acid sequence of cDNA clone also identified herein as Clone #21.
- **FIGURE 9.**: Amino acid sequence of Clone #21. Upper part of Figure presents translated protein sequence; lower portion of Figure presents whole protein sequence.
- **FIGURE 10.**: Nucleic acid sequence ofcDNA clone also identified herein as Clone #11.
- **FIGURE 11.**: Amino acid sequence of Clone #11. Upper part of Figure presents translated protein sequence; lower portion of Figure presents whole protein sequence.
- **FIGURE 12.**: Nucleic acid sequence of cDNA clone also identified herein as Clone #5.
- **FIGURE 13.**: Amino acid sequence of Clone #5. Upper part of Figure presents translated protein sequence; lower portion of Figure presents whole protein sequence.
- **FIGURE 14.**: Nucleic acid sequence of cDNA clone also identified herein as Clone #9.
- **FIGURE 15.**: Amino acid sequence of Clone #5. Upper part of Figure presents translated protein sequence; lower portion of Figure presents whole protein sequence.

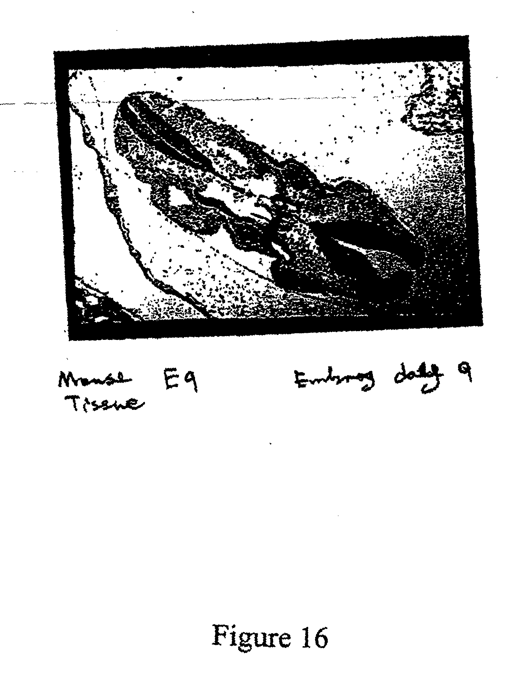

- **FIGURE 16.**: Tissue immunochemical staining on mouse embryo (Day 9) showing ITSN expression in neural blasts during migration and formation in CNS.
- **FIGURE 17.**: **Summary of Studies on ITSN:**
**I. Gene sequence:** First line showing the scale of ITSN cDNA; Second line showing the total numbers of the exons and the positions of each exon located.
**II. Protein domains vs nucleotide sequence:** ITSN was predicted consists of 11 protein domains as listed on the map - 2 EH domains, 5 SH3 domains and 1 of each GEF, pH and C2 domains. Their relative positions on the cDNA level were numbered under each domain.
**III. Gene expression of human adult and fetal tissues:** This part summarized the Northern blot results showing ITSN was ubiquitously expressed with extensive alternative splicing generating tissue and developmental stage-specific expression.

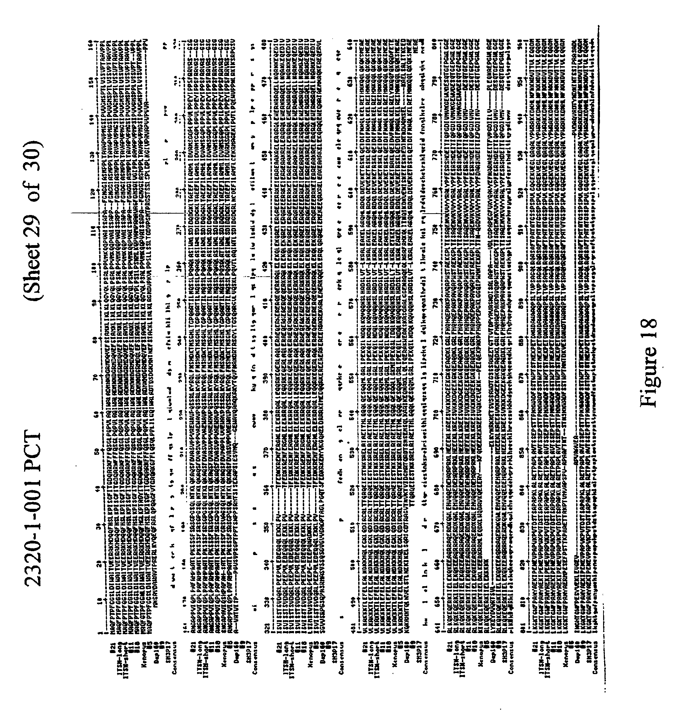

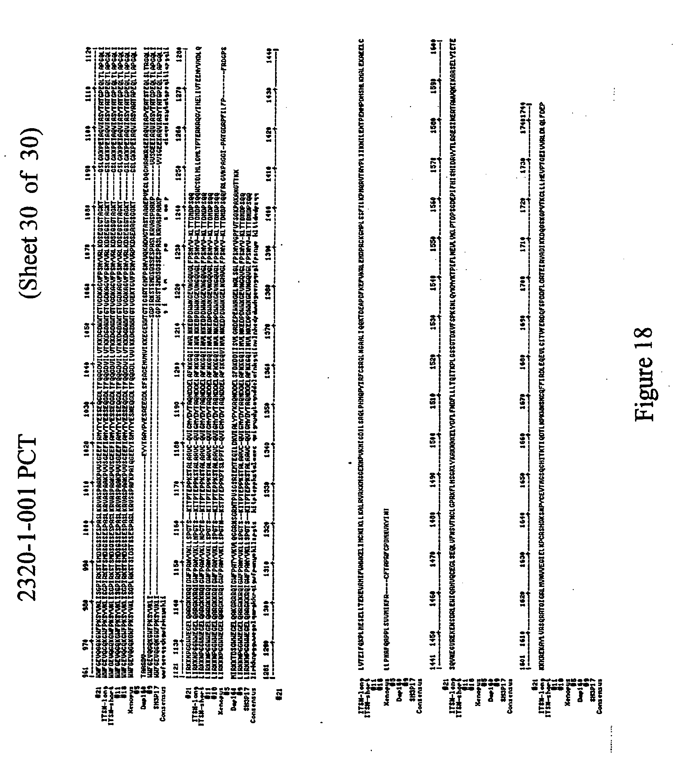

- **FIGURE 18.**: Sequence comparisons between nucleic acid molecules of present invention, and Intersectins (ITSN), including a consensus sequence.

### DETAILED DESCRIPTION OF THE INVENTION

The present invention discloses a novel SH3D1A gene, and clones, and corresponding proteins, both translated and full length, which SH3D1A gene is on chromosome 21, and that contributes to the development of platelets and the pathogenesis of leukemias, both in general and in particular those involving the megakaryocytic lineage. The invention provides methods useful for diagnosing and treating the following acute leukemias, thrombocytopenia, megakaryocytic abnormality, hematopoetic disorders, myeloproliferative disorder, platelet disorder, leukemia, leukemia in Down syndrome, leaukemia, platelet disorder on chromosome 21, low platelets in deletion for 21, association of gains in chromosome 21 with leukemias and disorders associated with associated with dysfunction; and neural abnormalities, dysfunctions and disorders, including brain malformations and corresponding cognitive dysfunctions, microcephaly, lissencephaly, colpocephaly, holoprosencephaly.

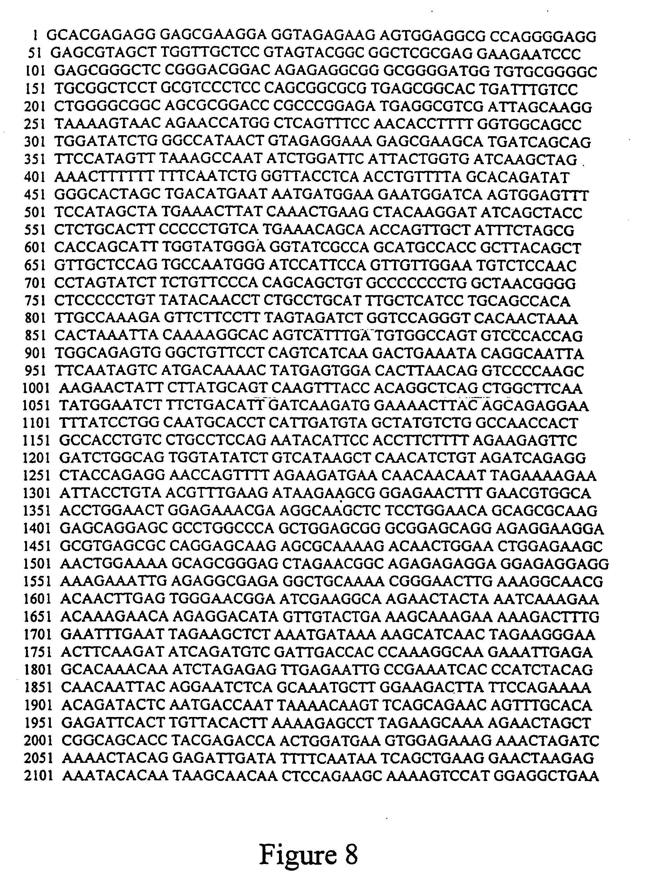

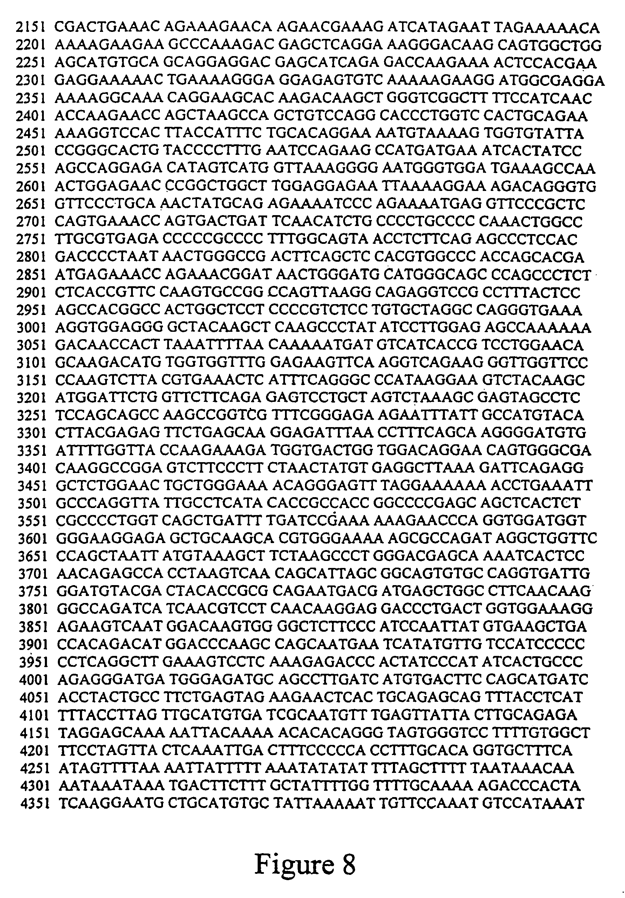

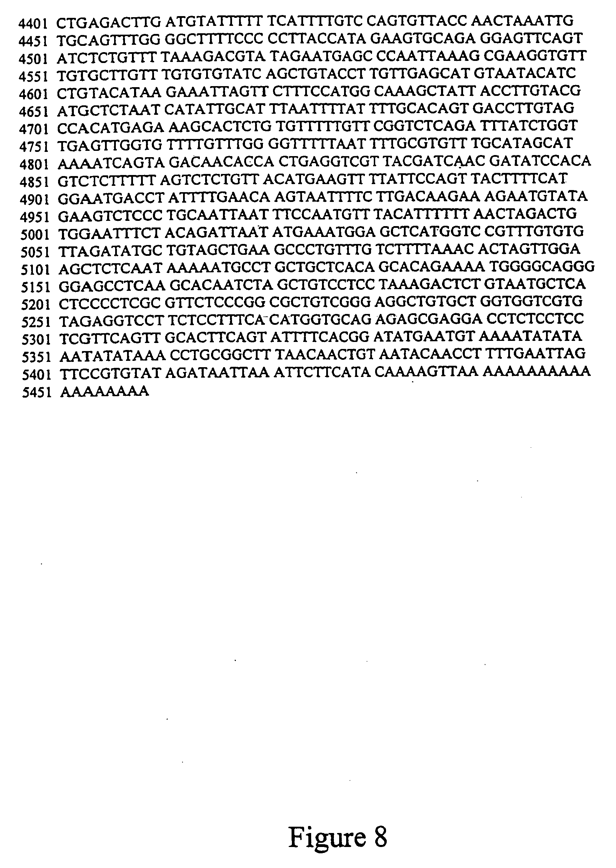

This invention provides an isolated nucleic acid which encodes a human SH3D1A, as defined hereinabove, such as the nucleic acids set forth in Figures 8, 10, 12 and 14, fragments. This invention is directed to an isolated nucleic acid encoding an amino acid sequence which forms one or more myristoylation sites in the EH domain and SH3 domain. This invention provides a isolated nucleic acid encoding an amino acid sequence which forms one or more EH domains and one or more SH3 domains. In one embodiment the nucleic acid which encodes an amino acid sequence which forms two EH domains and four SH3 domains. As show in Figure 1 the nucleic acid encoding the amino acid sequence comprising one or more myristoylation sites in the EH domain and SH3 domain.

The nucleic acid of the invention encodes an amino acid sequence as set forth in Figure 5.

This invention provides for an isolated nucleic acid which encodes SH3D1A. This isolated nucleic acid may be DNA or RNA, specifically cDNA or genomic DNA. The isolated nucleic acid encodes a human SH3D1A having the same amino acid sequence as the sequence designated Figure 5. Specifically the isolated nucleic acid has the sequence designated Figure 4.

This invention provides for a replicable vector comprising the isolated nucleic acid molecule of the DNA virus. The vector includes, but is not limited to a plasmid, cosmid, λ phage or yeast artificial chromosome (YAC) which contains at least a portion of the isolated nucleic acid molecule. As an example to obtain these vectors, insert and vector DNA can both be exposed to a restriction enzyme to create complementary ends on both molecules which base pair with each other and are then ligated together with DNA ligase. Alternatively, linkers can be ligated to the insert DNA which correspond to a restriction site in the vector DNA, which is then digested with the restriction enzyme which cuts at that site. Other means are also available and known to an ordinary skilled practitioner.

Regulatory elements required for expression include promoter or enhancer sequences to bind RNA polymerase and transcription initiation sequences for ribosome binding. For example, a bacterial expression vector includes a promoter such as the lac promoter and for transcription initiation the Shine-Dalgamo sequence and the start codon AUG. Similarly, a eukaryotic expression vector includes a heterologous or homologous promoter for RNA polymerase II, a downstream polyadenylation signal, the start codon AUG, and a termination codon for detachment of the ribosome. Such vectors may be obtained commercially or assembled from the sequences described by methods well-known in the art, for example the methods described above for constructing vectors in general.

This invention provides a host cell containing the above vector. The host cell may contain the isolated DNA molecule artificially introduced into the host cell. The host cell may be a eukaryotic or bacterial cell (such as E.coli), yeast cells, fungal cells, insect cells and animal cells. Suitable animal cells include, but are not limited to Vero cells, HeLa cells, Cos cells, CV 1 cells and various primary mammalian cells.

The term "vector", refers to viral expression systems, autonomous self-replicating circular DNA (plasmids), and includes both expression and nonexpression plasmids. Where a recombinant microorganism or cell culture is described as hosting an "expression vector," this includes both extrachromosomal circular DNA and DNA that has been incorporated into the host chromosome(s). Where a vector is being maintained by a host cell, the vector may either be stably replicated by the cells during mitosis as an autonomous structure, or is incorporated within the host's genome.

The term "plasmid" refers to an autonomous circular DNA molecule capable of replication in a cell, and includes both the expression and nonexpression types. Where a recombinant microorganism or cell culture is described as hosting an "expression plasmid", this includes latent viral DNA integrated into the host chromosome(s). Where a plasmid is being maintained by a host cell, the plasmid is either being stably replicated by the cells during mitosis as an autonomous structure or is incorporated within the host's genome.

The following terms are used to describe the sequence relationships between two or more nucleic acid molecules or polynucleotides: "reference sequence", "comparison window", "sequence identity", "percentage of sequence identity", and "substantial identity". A "reference sequence" is a defined sequence used as a basis for a sequence comparison; a reference sequence may be a subset of a larger sequence, for example, as a segment of a full-length cDNA or gene sequence given in a sequence listing or may comprise a complete cDNA or gene sequence.

Optimal alignment of sequences for aligning a comparison window may be conducted by the local homology algorithm of Smith and Waterman (1981) *Adv. Appl. Math.* 2:482, by the homology alignment algorithm of Needleman and Wunsch (1970) *J. Mol. Biol.* 48:443, by the search for similarity method of Pearson and Lipman (1988) *Proc. Natl. Acad. Sci. (USA)* 85:2444, or by computerized implementations of these algorithms (GAP, BESTFIT, FASTA, and TFASTA in the Wisconsin Genetics Software Package Release 7.0, Genetics Computer Group, 575 Science Dr., Madison, WI).

"Substantial identity" or "substantial sequence identity" mean that two peptide sequences, when optimally aligned, such as by the programs GAP or BESTFIT using default gap which share at least 90 percent sequence identity, preferably at least 95 percent sequence identity, more preferably at least 99 percent sequence identity or more. "Percentage amino acid identity" or "percentage amino acid sequence identity" refers to a comparison of the amino acids of two polypeptides which, when optimally aligned, have approximately the designated percentage of the same amino acids. For example, "95% amino acid identity" refers to a comparison of the amino acids of two polypeptides which when optimally aligned have 95% amino acid identity. Preferably, residue positions which are not identical differ by conservative amino acid substitutions. For example, the substitution of amino acids having similar chemical properties such as charge or polarity are not likely to effect the properties of a protein. Examples include glutamine for asparagine or glutamic acid for aspartic acid.

The phrase "nucleic acid molecule encoding" refers to a nucleic acid molecule which directs the expression of a specific protein or peptide. The nucleic acid sequences include both the DNA strand sequence that is transcribed into RNA and the RNA sequence that is translated into protein. The nucleic acid molecule include both the full length nucleic acid sequences as well as non-full length sequences derived from the full length protein. It being further understood that the sequence includes the degenerate codons of the native sequence or sequences which may be introduced to provide codon preference in a specific host cell.

This invention provides a nucleic acid having a sequence complementary to the sequence of the isolated nucleic acid of the human SH3D1A gene. Specifically, this invention provides an oligonucleotide of at least 15 nucleotides capable of specifically hybridizing with a sequence of nucleotides present within a nucleic acid which encodes the human SH3D1A. In one embodiment the nucleic acid is DNA or RNA. In another embodiment the oligonucleotide is labeled with a detectable marker. In another embodiment the oligonucleotide is a radioactive isotope, a fluorophor or an enzyme.

Oligonucleotides which are complementary may be obtained as follows: The polymerase chain reaction is then carried out using the two primers. See *PCR Protocols: A Guide to Methods and Applications* [74]. Following PCR amplification, the PCR-amplified regions of a viral DNA can be tested for their ability to hybridize to the three specific nucleic acid probes listed above. Alternatively, hybridization of a viral DNA to the above nucleic acid probes can be performed by a Southern blot procedure without viral DNA amplification and under stringent hybridization conditions as described herein.

Oligonucleotides for use as probes or PCR primers are chemically synthesized according to the solid phase phosphoramidite triester method first described by Beaucage and Carruthers [19] using an automated synthesizer, as described in Needham-VanDevanter [69]. Purification of oligonucleotides is by either native acrylamide gel electrophoresis or by anion-exchange HPLC as described in Pearson, J.D. and Regnier, F.E. [75A]. The sequence of the synthetic oligonucleotide can be verified using the chemical degradation method of Maxam, A.M. and Gilbert, W. [63].

High stringency hybridization conditions are selected at about 5°C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 60°C. As other factors may significantly affect the stringency of hybridization, including, among others, base composition and size of the complementary strands, the presence of organic solvents, ie. salt or formamide concentration, and the extent of base mismatching, the combination of parameters is more important than the absolute measure of any one. For Example high stringency may be attained for example by overnight hybridization at about 68°C in a 6x SSC solution, washing at room temperature with 6x SSC solution, followed by washing at about 68°C in a 6x SSC in a 0.6x SSX solution.

Hybridization with moderate stringency may be attained for example by: 1) filter pre-hybridizing and hybridizing with a solution of 3x sodium chloride, sodium citrate (SSC), 50% formamide, 0.1M Tris buffer at Ph 7.5, 5x Denhardt's solution; 2.) pre-hybridization at 37°C for 4 hours; 3) hybridization at 37°C with amount of labelled probe equal to 3,000,000 cpm total for 16 hours; 4) wash in 2x SSC and 0.1% SDS solution; 5) wash 4x for 1 minute each at room temperature at 4x at 60°C for 30 minutes each; and 6) dry and expose to film.

The phrase "selectively hybridizing to" refers to a nucleic acid probe that hybridizes, duplexes or binds only to a particular target DNA or RNA sequence when the target sequences are present in a preparation of total cellular DNA or RNA. By selectively hybridizing it is meant that a probe binds to a given target in a manner that is detectable in a different manner from non-target sequence under high stringency conditions of hybridization. in a different "Complementary" or "target" nucleic acid sequences refer to those nucleic acid sequences which selectively hybridize to a nucleic acid probe. Proper annealing conditions depend, for example, upon a probe's length, base composition, and the number of mismatches and their position on the probe, and must often be determined empirically. For discussions of nucleic acid probe design and annealing conditions, see, for example, Sambrook *et al.,* [81] or Ausubel, F., *et al.,* [8].

It will be readily understood by those skilled in the art and it is intended here, that when reference is made to particular sequence listings, such reference includes sequences which substantially correspond to its complementary sequence and those described including allowances for minor sequencing errors, single base changes, deletions, substitutions and the like, including the clonal varients set forth herein, such that any such sequence variation corresponds to the nucleic acid sequence of the pathogenic organism or disease marker to which the relevant sequence listing relates.

Nucleic acid probe technology is well known to those skilled in the art who readily appreciate that such probes may vary greatly in length and may be labeled with a detectable label, such as a radioisotope or fluorescent dye, to facilitate detection of the probe. DNA probe molecules may be produced by insertion of a DNA molecule having the full-length or a fragment of the isolated nucleic acid molecule of the DNA virus into suitable vectors, such as plasmids or bacteriophages, followed by transforming into suitable bacterial host cells, replication in the transformed bacterial host cells and harvesting of the DNA probes, using methods well known in the art. Alternatively, probes may be generated chemically from DNA synthesizers.

RNA probes may be generated by inserting the full length or a fragment of the isolated nucleic acid molecule of the DNA virus downstream of a bacteriophage promoter such as T3, T7 or SP6. Large amounts of RNA probe may be produced by incubating the labeled nucleotides with a linearized isolated nucleic acid molecule of the DNA virus or its fragment where it contains an upstream promoter in the presence of the appropriate RNA polymerase.

As defined herein nucleic acid probes may be DNA or RNA fragments. DNA fragments can be prepared, for example, by digesting plasmid DNA, or by use of PCR, or synthesized by either the phosphoramidite method described by Beaucage and Carruthers, [19], or by the triester method according to *Matteucci, et al.,* [62], both incorporated herein by reference. A double stranded fragment may then be obtained, if desired, by annealing the chemically synthesized single strands together under appropriate conditions or by synthesizing the complementary strand using DNA polymerase with an appropriate primer sequence. Where a specific sequence for a nucleic acid probe is given, it is understood that the complementary strand is also identified and included. The complementary strand will work equally well in situations where the target is a double-stranded nucleic acid. It is also understood that when a specific sequence is identified for use a nucleic probe, a subsequence of the listed sequence which is 25 basepairs or more in length is also encompassed for use as a probe.

The DNA molecules of the subject invention also include DNA molecules coding for polypeptide analogs, fragments or derivatives of antigenic polypeptides which differ from naturally-occurring forms in terms of the identity or location of one or more amino acid residues (deletion analogs containing less than all of the residues specified for the protein, substitution analogs wherein one or more residues specified are replaced by other residues and addition analogs where in one or more amino acid residues is added to a terminal or medial portion of the polypeptides) and which share some or all properties of naturally-occurring forms. These molecules include: the incorporation of codons "preferred" for expression by selected non-mammalian hosts; the provision of sites for cleavage by restriction endonuclease enzymes; and the provision of additional initial, terminal or intermediate DNA sequences that facilitate construction of readily expressed vectors.

Also, this invention provides an antisense molecule capable of specifically hybridizing with the isolated nucleic acid of the human SH3D1A gene. This invention provides an antagonist capable of blocking the expression of the peptide or polypeptide encoded by the isolated DNA molecule. In one embodiment the antagonist is capable of hybridizing with a double stranded DNA molecule. In another embodiment the antagonist is a triplex oligonucleotide capable of hybridizing to the DNA molecule. In another embodiment the triplex oligonucleotide is capable of binding to at least a portion of the isolated DNA molecule with a nucleotide sequence..

The antisense molecule may be DNA or RNA or variants thereof (i.e. DNA or RNA with a protein backbone). The present invention extends to the preparation of antisense nucleotides and ribozymes that may be used to interfere with the expression of the receptor recognition proteins at the translation of a specific mRNA, either by masking that MRNA with an antisense nucleic acid or cleaving it with a ribozyme.

Antisense nucleic acids are DNA or RNA molecules that are complementary to at least a portion of a specific MRNA molecule. In the cell, they hybridize to that MRNA, forming a double stranded molecule. The cell does not translate an MRNA in this double-stranded form. Therefore, antisense nucleic acids interfere with the expression of MRNA into protein.

Antisense nucleotides or polynucleotide sequences are useful in preventing or diminishing the expression of the SH3D1A gene, as will be appreciated by those skilled in the art. For example, polynucleotide vectors containing all or a portion of the SH3D1A gene or other sequences from the SH3D1A region (particularly those flanking the SH3D1A gene) may be placed under the control of a promoter in an antisense orientation and introduced into a cell. Expression of such an antisense construct within a cell will interfere with SH3D1A transcription and/or translation and/or replication. Oligomers of about fifteen nucleotides and molecules that hybridize to the AUG initiation codon are particularly efficient, since they are easy to synthesize and are likely to pose fewer problems than larger molecules upon introduction to cells.

This invention provides a transgenic nonhuman mammal which comprises at least a portion of the isolated DNA molecule introduced into the mammal at an embryonic stage. Methods of producing a transgenic nonhuman mammal are known to those skilled in the art.

This invention also provides a method of producing a polypeptide encoded by isolated DNA molecule, which comprises growing the above host vector system under suitable conditions permitting production of the polypeptide and recovering the polypeptide so produced.

This invention provides a polypeptide comprising the amino acid sequence of a human SH3D1A. The amino acid sequence is set forth in Figure 5. Further, the isolated polypeptide encoded by the isolated DNA molecule may be linked to a second polypeptide encoded by a nucleic acid molecule to form a fusion protein by expression in a suitable host cell. In one embodiment the second nucleic acid molecule encodes beta-galactosidase. Other nucleic acid molecules which are used to form a fusion protein are known to those skilled in the art.

Antibodies may be made which specifically bind to the polypeptide encoded by the isolated DNA molecule. The antibody may be a monoclonal antibody or a polyclonal antibody. The antibody or DNA molecule may be labelled with a detectable marker including, but not limited to: a radioactive label, or a colorimetric, a luminescent, or a fluorescent marker, or gold. Radioactive labels include, but are not limited to: ³H, ¹⁴C, ³²P, ³³P; ³⁵S, ³⁶Cl, ⁵¹Cr, ⁵⁷CO, ⁵⁹Co, ⁵⁹Fe, ⁹⁰Y, ¹²⁵I, ¹³¹I, and ¹⁵⁶Re. Fluorescent markers include but are not limited to: fluorescein, rhodamine and auramin. Colorimetric markers include, but are not limited to: biotin, and digoxigenin. Methods of producing the polyclonal or monoclonal antibody are known to those of ordinary skill in the art.

Further, the antibody or nucleic acid molecule complex may be detected by a second antibody which may be linked to an enzyme, such as alkaline phosphatase or horseradish peroxidase. Other enzymes which may be employed are well known to one of ordinary skill in the art.

"Specifically binds to an antibody" or "specifically immunoreactive with", when referring to a protein or peptide, refers to a binding reaction which is determinative of the presence of the SH3D1A of the invention in the presence of a heterogeneous population of proteins and other biologies including viruses other than the SH3D1A. Thus, under designated immunoassay conditions, the specified antibodies bind to the SH3D1A antigens and do not bind in a significant amount to other antigens present in the sample. Specific binding to an antibody under such conditions may require an antibody that is selected for its specificity for a particular protein. For example, antibodies raised to the human SH3D1A immunogen described herein can be selected to obtain antibodies specifically immunoreactive with the SH3D1A proteins and not with other proteins. These antibodies recognize proteins homologous to the human SH3D1A protein. A variety of immunoassay formats may be used to select antibodies specifically immunoreactive with a particular protein. For example, solid-phase ELISA immunoassays are routinely used to select monoclonal antibodies specifically immunoreactive with a protein. See Harlow and Lane [32] for a description of immunoassay formats and conditions that can be used to determine specific immunoreactivity.

Methods may be used to select specific regions on the polypeptide encoded by the isolated DNA molecule of the DNA virus to generate antibodies. The protein sequence may be determined from the cDNA sequence. Amino acid sequences may be analyzed by methods well known to those skilled in the art to determine whether they produce hydrophobic or hydrophilic regions in the proteins which they build. In the case of cell membrane proteins, hydrophobic regions are well known to form the part of the protein that is inserted into the lipid bilayer of the cell membrane, while hydrophilic regions are located on the cell surface, in an aqueous environment. Usually, the hydrophilic regions will be more immunogenic than the hydrophobic regions. Therefore the hydrophilic amino acid sequences may be selected and used to generate antibodies specific to polypeptide encoded by the isolated nucleic acid molecule encoding the DNA virus. The selected peptides may be prepared using commercially available machines. As an alternative, DNA, such as a cDNA or a fragment thereof, may be cloned and expressed and the resulting polypeptide recovered and used as an immunogen.

Polyclonal antibodies against these peptides may be produced by immunizing animals using the selected peptides. Monoclonal antibodies are prepared using hybridoma technology by fusing antibody producing B cells from immunized animals with myeloma cells and selecting the resulting hybridoma cell line producing the desired antibody. Alternatively, monoclonal antibodies may be produced by *in vitro* techniques known to a person of ordinary skill in the art Also as set forth earlier herein, chimeric (bi-specific) antibodies may be prepared by techniques well known in the art, and are likewise contemplated herein. Any and all of these antibodies are useful to detect the expression of polypeptide encoded by the isolated DNA molecule of the DNA virus in living animals, in humans, or in biological tissues or fluids isolated from animals or humans.

The antibodies may be detectably labeled, utilizing conventional labeling techniques well-known to the art. Thus, the antibodies may be radiolabeled using, for example, radioactive isotopes such as ³H, ¹²⁵I, ¹³¹I, and ³⁵S. The antibodies may also be labeled using fluorescent labels, enzyme labels, free radical labels, or bacteriophage labels, using techniques known in the art. Typical fluorescent labels include fluorescein isothiocyanate, rhodamine, phycoerythrin, phycocyanin, alophycocyanin, and Texas Red.

Since specific enzymes may be coupled to other molecules by covalent links, the possibility also exists that they might be used as labels for the production of tracer materials. Suitable enzymes include alkaline phosphatase, beta-galactosidase, glucose-6-phosphate dehydrogenase, maleate dehydrogenase, and peroxidase. Two principal types of enzyme immunoassay are the enzyme-linked immunosorbent assay (ELISA), and the homogeneous enzyme immunoassay, also known as enzyme-multiplied immunoassay (EMIT, Syva Corporation, Palo Alto, CA). In the ELISA system, separation may be achieved, for example, by the use of antibodies coupled to a solid phase. The EMIT system depends on deactivation of the enzyme in the tracer-antibody complex; the activity can thus be measured without the need for a separation step.

Additionally, chemiluminescent compounds may be used as labels. Typical chemiluminescent compounds include luminol, isoluminol, aromatic acridinium esters, imidazoles, acridinium salts, and oxalate esters. Similarly, bioluminescent compounds may be utilized for labelling, the bioluminescent compounds including luciferin, luciferase, aequorin, and fluorescent proteins such as green fluorescent protein (GFP). Once labeled, the antibody may be employed to identify and quantify immunologic counterparts (antibody or antigenic polypeptide) utilizing techniques well-known to the art.

A description of a radioimmunoassay (RIA) may be found in *Laboratory Techniques in Biochemistry and Molecular Biology* [52], with particular reference to the chapter entitled "An Introduction to Radioimmune Assay and Related Techniques" by Chard, T., incorporated by reference herein. A description of general, immunometric assays of various types can be found in the following U.S. Pat. Nos. 4,376,110 (David *et al*.) or 4,098,876 (Piasio).

One can use immunoassays to detect for the SH3D1A gene, specific peptides, or for antibodies to the virus or peptides. A general overview of the applicable technology is in Harlow and Lane [32], incorporated by reference herein.

For example, antibodies to the human SH3D1A can be used to detect the agent in the sample. In brief, to produce antibodies to the agent or peptides, the sequence being targeted is expressed in transfected cells, preferably bacterial cells, and purified. The product is injected into a mammal capable of producing antibodies. Either monoclonal or polyclonal antibodies (as well as any recombinant antibodies) specific for the gene product can be used in various immunoassays. Such assays include competitive immunoassays, radioimmunosssays, Western blots, ELISA, indirect immunofluorescent assays and the like. For competitive immunoassays, see Harlow and Lane [32] at pages 567-573 and 584-589.

Furthermore, commercial test kits suitable for use by a medical specialist may be prepared to determine the presence or absence of predetermined binding activity or predetermined binding activity capability to suspected target cells. In accordance with the testing techniques discussed above, one class of such kits will contain at least the labeled polypeptide or its binding partner, for instance an antibody specific thereto, and directions, of course, depending upon the method selected, e.g., "competitive," "sandwich," "DASP" and the like. The kits may also contain peripheral reagents such as buffers, stabilizers, etc.

Monoclonal antibodies or recombinant antibodies may be obtained by various techniques familiar to those skilled in the art. Briefly, spleen cells or other lymphocytes from an animal immunized with a desired antigen are immortalized, commonly by fusion with a myeloma cell (see, Kohler and Milstein [50], incorporated herein by reference). Alternative methods of immortalization include transformation with Epstein Barr Virus, oncogenes, or retroviruses, or other methods well known in the art. Colonies arising from single immortalized cells are screened for production of antibodies of the desired specificity and affinity for the antigen, and yield of the monoclonal antibodies produced by such cells may be enhanced by various techniques, including injection into the peritoneal cavity of a vertebrate host. New techniques using recombinant phage antibody expression systems can also be used to generate monoclonal antibodies. See for example: McCafferty, J *et al.* [64]; Hoogenboom, H.R. *et al.* [39]; and Marks, J.D. *et al.* [60].

Such peptides may be produced by expressing the specific sequence in a recombinantly engineered cell such as bacteria, yeast, filamentous fungal, insect (especially employing baculoviral vectors), and mammalian cells. Those of skill in the art are knowledgeable in the numerous expression systems available for expression of herpes virus protein.

Briefly, the expression of natural or synthetic nucleic acids encoding viral protein will typically be achieved by operably linking the desired sequence or portion thereof to a promoter (which is either constitutive or inducible), and incorporated into an expression vector. The vectors are suitable for replication or integration in either prokaryotes or eukaryotes. Typical cloning vectors contain antibiotic resistance markers, genes for selection of transformants, inducible or regulatable promoter regions, and translation terminators that are useful for the expression of viral genes.

Methods for the expression of cloned genes in bacteria are also well known. In general, to obtain high level expression of a cloned gene in a prokaryotic system, it is advisable to construct expression vectors containing a strong promoter to direct mRNA transcription. The inclusion of selection markers in DNA vectors transformed in *E*. *coli* is also useful. Examples of such markers include genes specifying resistance to antibiotics. See [81] *supra,* for details concerning selection markers and promoters for use in *E*. *coli.* Suitable eukaryote hosts may include plant cells, insect cells, mammalian cells, yeast, and filamentous fungi.

The peptides derived form the nucleic acids, peptide fragments are produced by recombinant technology may be purified by standard techniques well known to those of skill in the art. Recombinantly produced sequences can be directly expressed or expressed as a fusion protein. The protein is then purified by a combination of cell lysis (*e.g.,* sonication) and affinity chromatography. For fusion products, subsequent digestion of the fusion protein with an appropriate proteolytic enzyme releases the desired peptide.

The proteins may be purified to substantial purity by standard techniques well known in the art, including selective precipitation with such substances as ammonium sulfate, column chromatography, immunopurification methods, and others. See, for instance, Scopes, R. [84].

This invention provides a method for determining whether a subject carries a mutation in the SH3D1A gene which comprises: a) obtaining an appropriate nucleic acid sample from the subject; and (b) determining whether the nucleic acid sample from step (a) is, or is derived from, a nucleic acid which encodes mutant SH3D1A so as to thereby determine whether a subject carries a mutation in the SH3D1A gene. In one embodiment, the nucleic acid sample in step (a) comprises mRNA corresponding to the transcript of DNA encoding a mutant SH3D1A, and wherein the determining of step (b) comprises: (i) contacting the mRNA with the oligonucleotide under conditions permitting binding of the mRNA to the oligonucleotide so as to form a complex; (ii) isolating the complex so formed; and (iii) identifying the mRNA in the isolated complex so as to thereby determine whether the mRNA is, or is derived from, a nucleic acid which encodes mutant SH3D1A. In another embodiment, the determining of step (b) comprises: i) contacting the nucleic-acid sample of step (a), and the isolated nucleic acid with restriction enzymes under conditions permitting the digestion of the nucleic acid sample, and the isolated nucleic acid into distinct, distinguishable pieces of nucleic acid; (ii) isolating the pieces of nucleic acid; and (iii) comparing the pieces of nucleic acid derived from the nucleic acid sample with the pieces of nucleic acid derived from the isolated nucleic acid so as to thereby determine whether the nucleic acid sample is, or is derived from, a nucleic acid which encodes mutant SH3D1A.

The present invention further provides methods of screening the SH3D1A gene to identify mutations. Such methods may further comprise the step of amplifying a portion of the SH3D1A gene, and may further include a step of providing a set of polynucleotides which are primers for amplification of said portion of the SH3D1A gene. The method is useful for identifying mutations for use in either diagnosis of the predisposition to, and diagnosis and treatment of megakaryocytic abnormality, hematopoietic disorders, myeloproliferative disorder, platelet disorder, leukemia; neural abnormality or other disorder, and prenatal diagnosis and treatment of tumors. Useful diagnostic techniques include, but are not limited to fluorescent in situ hybridization (FISH), direct DNA sequencing, PFGE analysis, Southern blot analysis, single stranded conformation analysis (SSCA), Rnase protection assay, allele-specifrc oligonucleotide (ASO), dot blot analysis and PCR-SSCP, as discussed in detail further below.

There are several methods that can be used to detect DNA sequence variation. Direct DNA sequencing, either manual sequencing or automated fluorescent sequencing can detect sequence variation. For a gene as large as SH3D1A, manual sequencing is very labor-intensive, but under optimal conditions, mutations in the coding sequence of a gens are rarely missed. Another approach is the single-stranded conformation polymorphism assay (SSCA) (Orita et aL, 1989). This method does not detect all sequence changes, especially if the DNA fragment size is greater than 200 bp, but can be optimized to detect most DNA sequence variation. The reduced detection sensitivity is a disadvantage, but the increased throughput possible with SSCA makes it an attractive, viable alternative to direct sequencing for mutation detection on a research basis. The fragments which have shifted mobility on SSCA gels are then sequenced to determine the exact nature of the DNA sequence variation. Other approaches based on the detection of mismatches between the two complementary DNA strands include clamped denaturing gel electrophoresis (CDGE) (Sheffield et al., 1991), heteroduplex analysis (HA) (White et al., 1992) and chemical mismatch cleavage (CMC) (Grompe et al., 1989). None of the methods described above will detect large deletions, duplications or insertions, nor will they detect a regulatory mutation which affects transcription or translation of the protein. Other methods which might detect these classes of mutations such as a protein truncation assay or the asymmetric assay, detect only specific types of mutations and would not detect missense mutations. A review of currently available methods of detecting DNA sequence variation can be found in a recent review by Grompe (1993). Once a mutation is known, an allele specific detection approach such as allele specific oligonucleotide (ASO) hybridization can be utilized to rapidly screen large numbers of other samples for that same mutation.

A rapid preliminary analysis to detect polymorphisms in DNA sequences can be performed by looking at a series of Southern blots of DNA cut with one or more restriction enzymes, preferably with a large number of restriction enzymes. Each blot contains a series of normal individuals and a series of tumors. Southern blots displaying hybridizing fragments (differing in length from control DNA when probed with sequences near or including the SH3D1A gene) indicate a possible mutation. If restriction enzymes which produce very large restriction fragments are used, then pulsed field gel electrophoresis (PFGE) is employed.

Detection of point mutations may be accomplished by molecular cloning of the SH3D1A allele(s) and sequencing the allele(s) using techniques well known in the art. Alternatively, the gene sequences can be amplified directly from a genomic DNA preparation from the tumor tissue, using known techniques. The DNA sequence of the amplified sequences can then be determined. There are six well known methods for a more complete, yet still indirect, test for confirming the presence of a susceptibility allele: 1) single stranded conformation analysis (SSCA) (Orita et al., 1989); 2) denaturing gradient gel electrophoresis (DGGE) (Wartell et al., 1990; Sheffield et al., 1989); 3) RNase protection assays (Finkelstein et al., 1990; Kinszleret al., 1991); 4) allele-specific oligonucleotides (ASOs) (Conner et al., 1983); 5) the use of proteins which recognize nucleotide mismatches, such as the E. coli mutS protein (Modrich, 1991); and 6) allele-specific PCR (Rano & Kidd, 1989). For allele-specific PCR, primers are used which hybridize at their 3' ends to a particular SH3D1A mutation. If the particular SH3D1A mutation is not present, an amplification product is not observed. Amplification Refractory Mutation System (ARMS) can also be used, as disclosed in European Patent Application Publication No. 0332435 and in Newton et al., 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected individual for the presence of the SH3D1A mutation found in that individual. Other techniques for detecting insertions and deletions as known in the art can be used.

In similar fashion, DNA probes can be used to detect mismatches, through enzymatic or chemical cleavage. See, *e.g*., Cotton et al., 1988; Shenk et al., 1975; Novack et al., 1986. Alternatively, mismatches can be detected by shifts in the electrophoretic mobility of mismatched duplexes relative to matched duplexes. See, *e.g.,* Cariello, 1988. With either riboprobes or DNA probes, the cellular mRNA or DNA which might contain a mutation can be amplified using PCR (see below) before hybridization. Changes in DNA of the SH3D1A gene can also be detected using Southern hybridization, especially if the changes are gross rearrangements, such as deletions and insertions.

DNA sequences of the SH3D1A gene which have been amplified by use of PCR may also be screened using allele-specific probes. These probes are nucleic acid oligomers, each of which contains a region of the SH3D1A gene sequence harboring a known mutation. For example, one oligomer may be about 30 nucleotides in length, corresponding to a portion of the SH3D1A gene sequence. By use of a battery of such allele-specific probes, PCR amplification products can be screened to identify the presence of a previously identified mutation in the SH3D1A gene. Hybridization of allele-specific probes with amplified SH3D1A sequences can be performed, for example, on a nylon filter. Hybridization to a particular probe under stringent hybridization conditions indicates the presence of the same mutation in the tumor tissue as in the allele-specific probe.

Alteration of SH3D1A mRNA expression can be detected by any techniques known in the art These include Northern blot analysis, PCR amplification and RNase protection. Diminished mRNA expression indicates an alteration of the wild-type SH3D1A gene. Alteration of wild-type SH3D1A genes can also be detected by screening for alteration of wild-type SH3D1A protein. For example, monoclonal antibodies immunoreactive with SH3D1A can be used to screen a tissue. Lack of cognate antigen would indicate a SH3D1A mutation. Antibodies specific for products of mutant alleles could also be used to detect mutant SH3D1A gene product Such immunological assays can be done in any convenient formats known in the art. These include Western blots, immunohistochemical assays and ELISA assays. Any means for detecting an altered SH3D1A protein can be used to detect alteration of wild-type SH3D1A genes. Functional assays, such as protein binding determinations, can be used. In addition, assays can be used which detect SH3D1A biochemical function. Finding a mutant SH3D1A gene product indicates alteration of a wild-type SH3D1A gene. Mutant SH3D1A genes or gene products can also be detected in other human body samples, such as serum, stool, urine and sputum.

The present invention also provides for fusion polypeptides, comprising SH3D1A polypeptides. Homologous polypeptides may be fusions between the sequences of SH3D1A and a related protein. Likewise, heterologous fusions may be constructed which would exhibit a combination of properties or activities of the derivative proteins. For example, ligand-binding or other domains may be "swapped" between different new fusion polypeptides or fragments. Such homologous or heterologous fusion polypeptides may display, for example, altered strength or specificity of binding. Fusion partners include immunoglobulins, bacterial beta -galactosidase, trpE, protein A, beta -lactamase, alpha amylase, alcohol dehydrogenase and yeast alpha mating factor. See, e.g., Godowski et al., 1988. Fusion proteins will typically be made by either recombinant nucleic acid methods, as described below, or may be chemically synthesized. Techniques for the synthesis of polypeptides are described, for example, in Merrifield, 1963.

This invention provides a method for determining whether a subject has a megakaryocytic abnormality, myeloproliferative disorder, platelet disorder, or leukemia which comprises: (a) obtaining an appropriate sample from the subject; and (b) contacting the sample with the antibody so as to thereby determine whether a subject has the megakaryocytic abnormality, myeloproliferative disorder, platelet disorder, or leukemia.

This invention provides a method for determining whether a subject has a predisposition for a megakaryocytic abnormality, myeloproliferative disorder, platelet disorder, leukemia or a neural abnormality or other disorder, which comprises: (a) obtaining an appropriate nucleic acid sample from the subject; and (b) determining whether the nucleic acid sample from step (a) is, or is derived from, a nucleic acid which encodes SH3D1A so as to thereby determine whether a subject has a predisposition for a megakaryocytic abnormality, myeloproliferative disorder, platelet disorder, or leukemia.

This invention provides a method for determining whether a subject has a megakaryocytic abnormality, myeloproliferative disorder, platelet disorder, leukemia or a neural abnormality or other disorder, which comprises: (a) obtaining an appropriate nucleic acid sample from the subject; and (b) determining whether the nucleic acid sample from step (a) is, or is derived from, a nucleic acid which encodes the human SH3D1A so as to thereby determine whether a subject has megakaryocytic abnormality, myeloproliferative disorder, platelet disorder, leukemia or a neural abnormality or other disorder. In one embodiment the nucleic acid sample in step (a) comprises mRNA corresponding to the transcript of DNA encoding a human SH3D1A, and wherein the determining of step (b) comprises: (i) contacting the mRNA with the oligonucleotide under conditions permitting binding of the mRNA to the oligonucleotide so as to form a complex; (ii) isolating the complex so formed; and (iii) identifying the mRNA in the isolated complex so as to thereby determine whether the mRNA is, or is derived from, a nucleic acid which encodes a human SH3D1A. A particular finding in accordance with the invention, is that such disorders as may occur in adult brain have been observed with respect to the present invention, and accordingly adult patients may be diagnosed, and if possible, treated by the application of the inventive subject matter hereof.

This invention provides an ex vivo method for monitoring the progress and adequacy of treatment in a subject who has received treatment for a megakaryocytic abnormality, myeloproliferative disorder, platelet disorder, leukemia or a condition involving a neural abnormality or dysfunction, which comprises monitoring the level of nucleic acid encoding the human SH3D1A at various stages of treatment.

This invention provides a pharmaceutical composition comprising an amount of a polypeptide of the present invention, and a pharmaceutically effective carrier or diluent.

This invention provides claim 29.

This invention is directed to diagnostic methods and therepeutic treatments relating to th e following: Wilms tumor, Li-Fraumcini syndrome, retinoblastoma, familiar colon cancer, and acute myelogenous leukemia (AML), and myelodysplastic syndromes (MDSs).

This invention provides a method of diagnosing megakaryocytic abnormality, myeloproliferative disorder, platelet disorder, or leukemia in a subject which comprises: (a) obtaining a nucleic acid molecule from a tumor lesion of the subject; (b) contacting the nucleic acid molecule with a labelled nucleic acid molecule of at least 15 nucleotides capable of specifically hybridizing with the isolated DNA, under hybridizing conditions; and (c) determining the presence of the nucleic acid molecule hybridized, the presence of which is indicative of megakaryocytic abnormality, myeloproliferative disorder, platelet disorder, or leukemia in the subject, thereby diagnosing megakaryocytic abnormality, myeloproliferative disorder, platelet disorder, or leukemia in the subject.

In one embodiment the DNA molecule from the tumor lesion is amplified before step (b). In another embodiment FCR is employed to amplify the nucleic acid molecule. Methods of amplifying nucleic acid molecules are known to those skilled in the art.

In the above described methods, a size fractionation may be employed which is effected by a polyacrylamide gel. In one embodiment, the size fractionation is effected by an agarose gel. Further, transferring the DNA fragments into a solid matrix may be employed before a hybridization step. One example of such solid matrix is nitrocellulose paper.

This invention provides a method of diagnosing megakaryocytic abnormality, myeloproliferative disorder, platelet disorder, leukemia or a neural abnormality or dysfunction, in a subject which comprises (a) obtaining a nucleic acid molecule from a suitable bodily fluid of the subject, (b) contacting the nucleic acid molecule with a labelled nucleic acid molecules of at least 15 nucleotides capable of specifically hybridizing with the isolated DNA, under hybridizing conditions; and (c) determining the presence of the nucleic acid molecule hybridized, the presence of which is indicative of megakaryocytic abnormality, myeloproliferative disorder, platelet disorder, leukemia or neural abnormality or dysfunction, in the subject, thereby diagnosing megakaryocytic abnormality, myeloproliferative disorder, platelet disorder, or leukemia in the subject.

This invention provides a method of determining the presence of a megakaryocytic abnormality, myeloproliferative disorder, platelet disorder, or leukemia in a subject, which comprises (a) obtaining a suitable bodily fluid sample from the subject, (b) contacting the suitable bodily fluid of the subject to a support having already bound thereto an antigen, so as to bind antigen to a specific antibody, (c) removing unbound bodily fluid from the support, and (d) determining the level of the antigen bound by the antibody, thereby diagnosing megakaryocytic abnormality, myeloproliferative disorder, platelet disorder, leukemia or neural disorder.

A suitable bodily fluid includes, but is not limited to: serum, plasma, cerebrospinal fluid, lymphocytes, urine, transudates, or exudates. In the preferred embodiment, the suitable bodily fluid sample is serum or plasma. In addition, the bodily fluid sample may be cells from bone marrow, or a supernatant from a cell culture. Methods of obtaining a suitable bodily fluid sample from a subject are known to those skilled in the art. Methods of determining the level of antibody or antigen include, but are not limited to: ELISA, IFA, and Western blotting.

The diagnostic assays of the invention can be nucleic acid assays such as nucleic acid hybridization assays and assays which detect amplification of specific nucleic acid to detect for a nucleic acid sequence of the human SH3D1A described herein.

Accepted means for conducting hybridization assays are known and general overviews of the technology can be had from a review of: *Nucleic Acid Hybridization: A Practical Approach* [72]; *Hybridization of Nucleic Acids Immobilized on Solid Supports [41]; Analytical Biochemistry* [4] and Innis *et al., PCR Protocols* [74], *supra,* all of which are incorporated by reference herein.

Target specific probes may be used in the nucleic acid hybridization diagnostic. The probes are specific for or complementary to the target of interest. For precise allelic differentiations, the probes should be about 14 nucleotides long and preferably about 20-30 nucleotides. For more general detection of the human SH3D1A of the invention, nucleic acid probes are about 50 to about 1000 nucleotides, most preferably about 200 to about 400 nucleotides.

The specific nucleic acid probe can be RNA or DNA polynucleotide or oligonucleotide, or their analogs. The probes may be single or double stranded nucleotides. The probes of the invention may be synthesized enzymatically, using methods well known in the art (e.g., nick translation, primer extension, reverse transcription, the polymerase chain reaction, and others) or chemically (*e.g*., by methods such as the phosphoramidite method described by Beaucage and Carruthers [19], or by the triester method according to Matteucci, *et al.* [62], both incorporated herein by reference).

An alternative means for determining the presence of the human SH3D1A is *in situ* hybridization, or more recently, in situ polymerase chain reaction. In situ PCR is described in Neuvo *et al.* [71], Intracellular localization of polymerase chain reaction (PCR)-amplified Hepatitis C cDNA; Bagasra *et al.* [10], Detection of Human Immunodeficiency virus type 1 provirus in mononuclear cells by in situ polymerase chain reaction; and Heniford *et al.* [35], Variation in cellular EGF receptor mRNA expression demonstrated by in situ reverse transcriptase polymerase chain reaction. *In situ* hybridization assays are well known and are generally described in *Methods Enzymol. [67]* incorporated by reference herein. In an *in situ* hybridization, cells are fixed to a solid support, typically a glass slide. The cells are then contacted with a hybridization solution at a moderate temperature to permit annealing of target-specific probes that are labeled. The probes are preferably labelled with radioisotopes or fluorescent reporters.

The above described probes are also useful for in-situ hybridization or in order to locate tissues which express this gene, or for other hybridization assays for the presence of this gene or its MRNA in various biological tissues. In-situ hybridization is a sensitive localization method which is not dependent on expression of antigens or native vs. denatured conditions.
In brief, inhibitory nucleic acid therapy approaches can be classified into those that target DNA sequences, those that target RNA sequences (including pre-mRNA and mRNA), those that target proteins (sense strand approaches), and those that cause cleavage or chemical modification of the target nucleic acids.

Approaches targeting DNA fall into several categories. Nucleic acids can be designed to bind to the major groove of the duplex DNA to form a triple helical or "triplex" structure. Alternatively, inhibitory nucleic acids are designed to bind to regions of single stranded DNA resulting from the opening of the duplex DNA during replication or transcription.

More commonly, inhibitory nucleic acids are designed to bind to mRNA or mRNA precursors. Inhibitory nucleic acids are used to prevent maturation of pre-mRNA. Inhibitory nucleic acids may be designed to interfere with RNA processing, splicing or translation.

The inhibitory nucleic acids can be targeted to mRNA. In this approach, the inhibitory nucleic acids are designed to specifically block translation of the encoded protein. Using this approach, the inhibitory nucleic acid can be used to selectively suppress certain cellular functions by inhibition of translation of mRNA encoding critical proteins. For example, an inhibitory nucleic acid complementary to regions of c-myc mRNA inhibits c-myc protein expression in a human promyelocytic leukemia cell line, HL60, which overexpresses the c-myc proto-oncogene. See Wickstrom E.L., *et al.* [93] and Harel-Bellan, A., *et al.* [31A]. As described in Helene and Toulme, inhibitory nucleic acids targeting mRNA have been shown to work by several different mechanisms to inhibit translation of the encoded protein(s).

Lastly, the inhibitory nucleic acids can be used to induce chemical inactivation or cleavage of the target genes or mRNA. Chemical inactivation can occur by the induction of crosslinks between the inhibitory nucleic acid and the target nucleic acid within the cell. Other chemical modifications of the target nucleic acids induced by appropriately derivatized inhibitory nucleic acids may also be used.

Cleavage, and therefore inactivation, of the target nucleic acids may be effected by attaching a substituent to the inhibitory nucleic acid which can be activated to induce cleavage reactions. The substituent can be one that affects either chemical, or enzymatic cleavage. Alternatively, cleavage can be induced by the use of ribozymes or catalytic RNA. In this approach, the inhibitory nucleic acids would comprise either naturally occurring RNA (ribozymes) or synthetic nucleic acids with catalytic activity.

As used herein, "pharmaceutical composition" could mean therapeutically effective amounts of polypeptide products of the invention together with suitable diluents, preservatives, solubilizers, emulsifiers, adjuvant and/or carriers useful in SCF (stem cell factor) therapy. A "therapeutically effective amount" as used herein refers to that amount which provides a therapeutic effect for a given condition and administration regimen. Such compositions are liquids or lyophilized or otherwise dried formulations and include diluents of various buffer content (e.g., Tris-HCL, acetate, phosphate), pH and ionic strength, additives such as albumin or gelatin to prevent absorption to surfaces, detergents (e.g., Tween 20, Tween 80, Pluronic F68, bile acid salts) solubilizing agents (e.g., glycerol, polyethylene glycerol), anti-oxidants (e.g., ascorbic acid, sodium metabisulfite), preservatives (e.g., Thimerosal, benzyl alcohol parabens), bulking substances or tonicity modifiers (e.g., lactose, mannitol), covalent attachment of polymers such as polyethylene glycol to the protein, complexation with metal ions, or incorporation of the material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts. Such compositions will influence the physical state, solubility, stability, rate of in vivo release, and rate of *in vivo* clearance of SCF. The choice of compositions will depend on the physical and chemical properties of the protein having SCF activity. For example, a product derived from a membrane-bound form of SCF may require a formulation containing detergent. Controlled or sustained release compositions include formulation in lipophilic depots (e.g., fatty acids, waxes, oils). Also comprehended by the invention are particulate compositions coated with polymers (e.g., poloxamers or poloxamines) and SCF coupled to antibodies directed against tissue-specific receptor, ligands or antigens or coupled to ligands of tissue-specific receptors. Other embodiments of the compositions of the invention incorporate particulate forms protective coatings, protease inhibitors or permeation enhancers for various routes of administration, including parenteral, pulmonary, nasal and oral.

Further, as used herein "pharmaceutically acceptable carrier" are well known to those skilled in the art and include, but are not limited to, 0.01-0.1M and preferably 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

The term "adjuvant" refers to a compound or mixture that enhances the immune response to an antigen. An adjuvant can serve as a tissue depot that slowly releases the antigen and also as a lymphoid system activator that non-specifically enhances the immune response (Hood et al., *Immunology, Second Ed.,* 1984, Benjamin/Cummings: Menlo Park, California, p. 384). Often, a primary challenge with an antigen alone, in the absence of an adjuvant, will fail to elicit a humoral or cellular immune response. Adjuvant include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, keyhole limpet hemocyanins, dinitrophenol, and potentially useful human adjuvant such as BCG (*bacille Calmette-Guerin)* and *Corynebacterium parvum.* Preferably, the adjuvant is pharmaceutically acceptable.

Controlled or sustained release compositions include formulation in lipophilic depots (e.g. fatty acids, waxes, oils). Also comprehended by the invention are particulate compositions coated with polymers (e.g. poloxamers or poloxamines) and the compound coupled to antibodies directed against tissue-specific receptors, ligands or antigens or coupled to ligands of tissue-specific receptors. Other embodiments of the compositions of the invention incorporate particulate forms protective coatings, protease inhibitors or permeation enhancers for various routes of administration, including parenteral, pulmonary, nasal and oral.

When administered, compounds are often cleared rapidly from mucosal surfaces or the circulation and may therefore elicit relatively short-lived pharmacological activity. Consequently, frequent administrations of relatively large doses of bioactive compounds may by required to sustain therapeutic efficacy. Compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds (Abuchowski et al., 1981; Newmark et al., 1982; and Katre et al., 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

*Dosages.* The sufficient amount may include but is not limited to from about 1 µg/kg to about 1000 mg/kg. The amount may be 10 mg/kg. The pharmaceutically acceptable form of the composition includes a pharmaceutically acceptable carrier.

The preparation of therapeutic compositions which contain an active component is well understood in the art. Typically, such compositions are prepared as an aerosol of the polypeptide delivered to the nasopharynx or as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH buffering agents which enhance the effectiveness of the active ingredient.

An active component can be formulated into the therapeutic composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

A composition comprising "A" (where "A" is a single protein, DNA molecule, vector, etc.) is substantially free of "B" (where "B" comprises one or more contaminating proteins, DNA molecules, vectors, etc.) when at least about 75% by weight of the proteins, DNA, vectors (depending on the category of species to which A and B belong) in the composition is "A". Preferably, "A" comprises at least about 90% by weight of the A+B species in the composition, most preferably at least about 99% by weight.

The phrase "therapeutically effective amount" is used herein to mean an amount sufficient to reduce by at least about 15 percent, preferably by at least 50 percent, more preferably by at least 90 percent, and most preferably prevent, a clinically significant deficit in the activity, function and response of the host.

According to the invention, the component or components of a therapeutic composition of the invention may be introduced parenterally, transmucosally, *e.g.,* orally, nasally, pulmonarailly, or rectally, or transdermally. Preferably, administration is parenteral, *e.g.,* via intravenous injection, and also including, but is not limited to, intra-arteriole, intramuscular, intradermal, subcutaneous, intraperitoneal, intraventricular, and intracranial administration. Oral or pulmonary delivery may be preferred to activate mucosal immunity; since pneumococci generally colonize the nasopharyngeal and pulmonary mucosa, mucosal immunity may be a particularly effective preventive treatment. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; *i.e.,* carrier, or vehicle.

In another embodiment, the active compound can be delivered in a vesicle, in particular a liposome (see Langer, Science 249:1527-1533 (1990); Treat et al., in *Liposomes in the Therapy of Infectious Disease and Cancer,* Lopez-Berestein and Fidler (eds.), Liss, New York, pp. 353-365 (1989); Lopez-Berestein, ibid., pp. 317-327; see generally ibid).

In yet another embodiment, the therapeutic compound can be delivered in a controlled release system. For example, the polypeptide may be administered using intravenous infusion, an implantable osmotic pump, a transdermal patch, liposomes, or other modes of administration. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see *Medical Applications of Controlled Release,* Langer and Wise (eds.), CRC Pres., Boca Raton, Florida (1974); *Controlled Drug Bioavailability, Drug Product Design and Performance,* Smolen and Ball (eds.), Wiley, New York (1984); Ranger and Peppas, J. Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, i.e., the brain, thus requiring only a fraction of the systemic dose (see, e.g., Goodson, in Medical Applications of Controlled Release, supra, vol. 2, pp. 115-138 (1984)). Preferably, a controlled release device is introduced into a subject in proximity of the site of inappropriate immune activation or a tumor. Other controlled release systems are discussed in the review by Langer 1990, *Science* **249**:1527-1533.

A subject in whom administration of an active component as set forth above is an effective therapeutic regimen for a bacterial infection is preferably a human, but can be any animal. Thus, as can be readily appreciated by one of ordinary skill in the art, the methods and pharmaceutical compositions of the present invention are particularly suited to administration to any animal, particularly a mammal, and including, but by no means limited to, domestic animals, such as feline or canine subjects, farm animals, such as but not limited to bovine, equine, caprine, ovine, and porcine subjects, wild animals (whether in the wild or in a zoological garden), research animals, such as mice, rats, rabbits, goats, sheep, pigs, dogs, cats, etc., *i.e.,* for veterinary medical use.

In the therapeutic methods and compositions of the invention, a therapeutically effective dosage of the active component is provided. A therapeutically effective dosage can be determined by the ordinary skilled medical worker based on patient characteristics (age, weight, sex, condition, complications, other diseases, etc.), as is well known in the art. Furthermore, as further routine studies are conducted, more specific information will emerge regarding appropriate dosage levels for treatment of various conditions in various patients, and the ordinary skilled worker, considering the therapeutic context, age and general health of the recipient, is able to ascertain proper dosing. Generally, for intravenous injection or infusion, dosage may be lower than for intraperitoneal, intramuscular, or other route of administration. The dosing schedule may vary, depending on the circulation half-life, and the formulation used. The compositions are administered in a manner compatible with the dosage formulation in the therapeutically effective amount. Precise amounts of active ingredient required to be administered depend on the judgment of the practitioner and are peculiar to each individual. However, suitable dosages may range from about 0.1 to 20, preferably about 0.5 to about 10, and more preferably one to several, milligrams of active ingredient per kilogram body weight of individual per day and depend on the route of administration. Suitable regimes for initial administration and booster shots are also variable, but are typified by an initial administration followed by repeated doses at one or more hour intervals by a subsequent injection or other administration. Alternatively, continuous intravenous infusion sufficient to maintain concentrations of ten nanomolar to ten micromolar in the blood are contemplated.

This invention is illustrated in the Experimental Details section which follows. These sections are set forth to aid in an understanding of the invention but are not intended to, and should not be construed to, limit in any way the invention as set forth in the claims which follow thereafter.

### EXPERIMENTAL DETAILS SECTION

The invention discloses a small candidate region of 50-200 kb for low platelets in deletion for chromosome 21. At present, the candidate region for the familial platelet disorder is greater than 3,000 kb, a region containing as many as 150 genes. The SH3D1A is mapped to the small candidate region for low platelets for chromosome 21. Northern analysis using new sequence from SH3D1A reveals an abnormal band with significantly higher expression in RNA from lymphoblastoid cells derived from an affected individual vs. normal controls. DNA sequence analyses reveal homologies to domains that suggest involvement in developmental and/or cell regulatory phenomena such as lead to cancers when disturbed. These include the SH3 domains as well as EH domains, both associated with protein-protein interactions and the latter associated with maintenance of the cytoskeleton. Therefore, mutations, or increased or decreased expression are ultimately responsible for familial platelet disorder and possibly also for DS leukemias, subsets of non-DS leukemias and the processes that ultimately lead to abnormal platelets associated with deletion of chromosome 21.

### Materials and Methods

**Genomic clone obtained by screening the BAC library with EST:** In order to study the gene structure of SH3D1A, the genomic clones were obtained by screening a human BAC library B with a radio-labeled EST (cDNA) (dbEST#482496, Research Genetics, AL) according to the procedure described by Hurbet et al., 1997. Three positive clones were observed.

**Fluorescence in situ hybridization (FISH) to confirm the cytogenetic location of BAC 119E16 on chromosomes 21q22,11-12:** BAC DNAs were made as described in the previous publication (Hurbert et al., 1997). The BAC DNAs as probes were biotinylated and FISHed onto normal human chromosome preparations following the procedure described by Korenberg and Chen (1995). BAC 119E16 was confirmed to map on chromosome 21q22.11-12 by reviewing more than 50 cells. This was further confirmed as well by PCR using custom-designed primers for SH3D1A based on sequencing information.

**Sequencing cDNA and part of the genomic DNA:** The cDNA was sequenced using RT-PCR products templated on total brain cDNA or directly on BAC 119E16 containing the gene.

**Reverse transcription - polymerase chain reaction (RT-PCT):** SH3D1A cDNA was amplified by RT-PCR using a standard method. Briefly, the control RNA was isolated from a normal male cell line using the TRI reagent kit (Molecular Research Center, Inc. Cincinnati, OH). The first strand of cDNA was then produced using SuperScript Choice System (Pharmacia LKB Biotechnology). The PCR reaction was performed using custome designed primers with PCT-100 Programmable Thermal Controller by a standard PCR procedure. The PCR products for sequencing were prepared by purification with Geneclean Kit (BIO 101, Inc., Vista, CA) prior to sequencing. To produce clearer sequence, some PCR products were subcloned into pCR-2.1 Vector (CLONETECH Laboratory, Inc.) prior to sequencing.

**PCR of genomic DNA:** three genomic (exon) fragments were generated via PCR by using the BAC 119E16 DNA as template, and purified and sequenced as described above and below.

### Sequencing SH3D1A:

The nucleotide sequence of both the coding and non-coding strands were determined in their entirety by the dideoxy chain termination methods using the ABI PRISM Sequences DNA sequencing kit (PERKIN ELMER) with custom-made primers. The template for DNA sequencing were either PCR products or subclones as described above.

### Sequencing the upstream region of SH3D1A:

In order to complete sequencing of the 5' end of SH3D1A and identify the site of initiation of transcription, the following two methods were utilized:

### 1.5' RACE:

5' RACE was performed by using 5' Marathon RACE kit (CLONETECH Laboratories, Inc. CA). The reaction products were then electrophoresed onto 1% of SeaPlaque GTG agarose (FMC BioProducts, Rockland, ME). The products with the longest srizes (>2Kb) were then further confirmed by sequencing nested PCR fragments.

### 2. cDNA isolation from cDNA library:

The human cDNA clones were obtained from a cDNA library screening as described in Yamakama et al., (1995). The cDNAs were oligo (dT) primed and cloned undirectionally into the EcoRI and ChoI sites of the vector. The size of the clones were analyzed by electrophoresis and then using for sequencing.

### Sequencing Analysis:

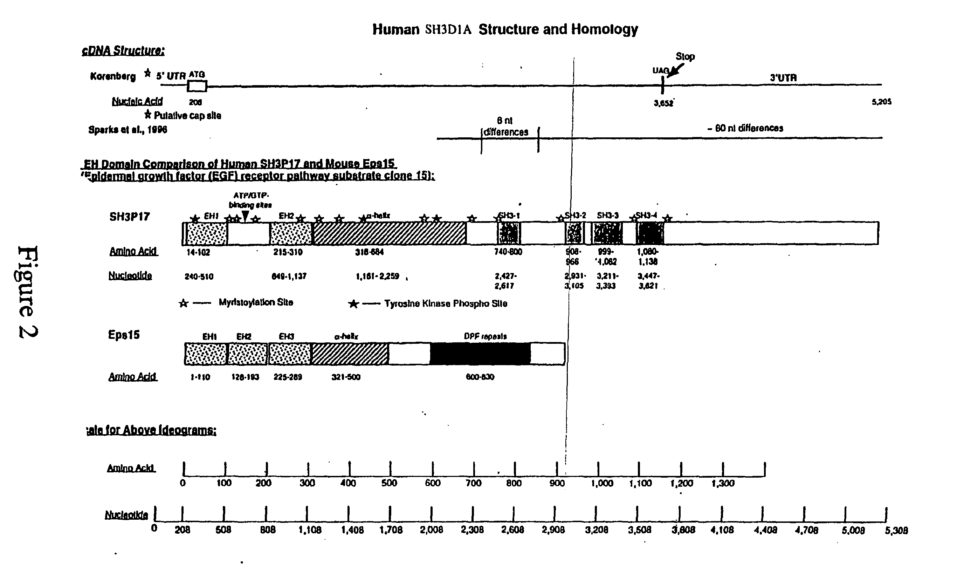

Data processing was performed using ABI Sequencing Analysis software which assessed trace quality and assembled sequence data (ABI Autoassemble program). The vector clipping was performed manually. To ensure the accuracy of the sequence, all regions of the finished sequence was covered by more than one subclone or PCR fragments, usually 3-5X and always were sequenced in opposite orientations. The sequence of the human SH3D1A was screened against Genbank (BLASTN & BLASTX). It was also compared with the previously published SH3P17 sequence (Hsu61166) by using V-gcg program. Significant differences between the previously published SH3P17 and this newly sequenced SH3D1A were found. These equalled about 8% of the nucleotides. Previous sequence totalled only 3,230bps of the 3' end vs. the subject invention's sequence of 5,200bp. Comparison using with the complete homology sequence gb#AF032118 in Xenopus Leavis indicated the same protein start site and a similar but not identical domain structure, see Figures 1 and 2.

### SH3DIA Gene Structure:

Protein structure was based on cNDA sequence analysis. The four SH3 domains were confirmed previously (Sparks et al., 1996). However, most significant was the definition of additional domains including EH domain (Eps Homolog domain) in the N terminal end that have been associated with protein interactions involved with cell cycle control and morphogenesis. These suggested a possible role, both in human embryogenesis and in cancers, notably the leukemias associated with Down Syndrome (DS), the decreased platelets associated with deletion of chromosome 21 reported by Fannin et al., 1995, and the familial platelet disorder reported by Dowton et al. (1985) and Ho et al. (1996), all of whose map positions include SH3P17.

### Gene expression study by Northern Blotting:

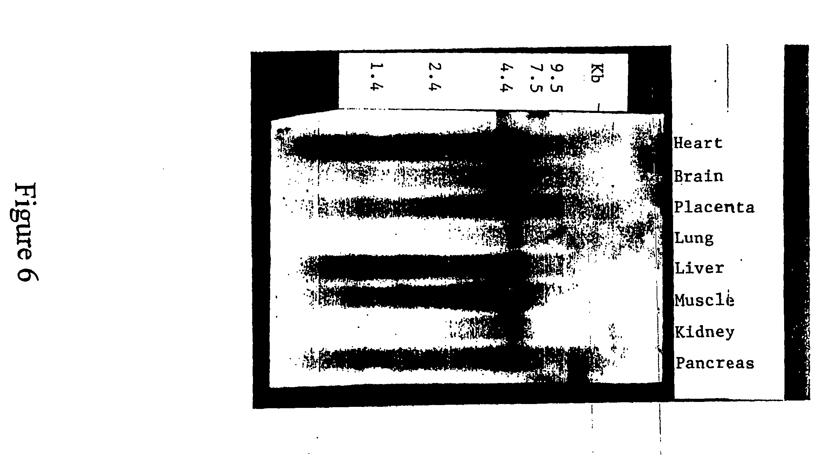

Northern blots made from human multiple tissues were used to perform this study according to the manufacturer's instruction (CLONETHch Laboratory, Inc., CA). Referring to Figure 6, the gene was found to be expressed in all adult human tissues tested, those included Heart, brain, placenta, lung, liver, muscle, kidney and pancreas.

### Preparation of full length cDNA Clones corresponding to SH3D1A

A cDNA library based on fetal brain was screened in the same manner as described above with respect to the isolation and sequencing of SH3D1A. Accordingly, Sequencing of 5 different sizes of the cDNA clones was conducted, and indicated that there are at least three isoforms that exist. As all of the sequenced cDNA clones shown in Figure 8, #21 was a full-length cDNA that contains 5438 nucleotides and codes for 1221 amino acids; #11 was a shorter full-length cDNA that contains 5179 nucleotides and codes for 1215 amino acids; clone #s 5 and #9 represent 2192bp, 3193bp and 3128bp length cDNA respectively, while #5 was identical to #21 and #11 at the 5'UTR containing only two EH domains.

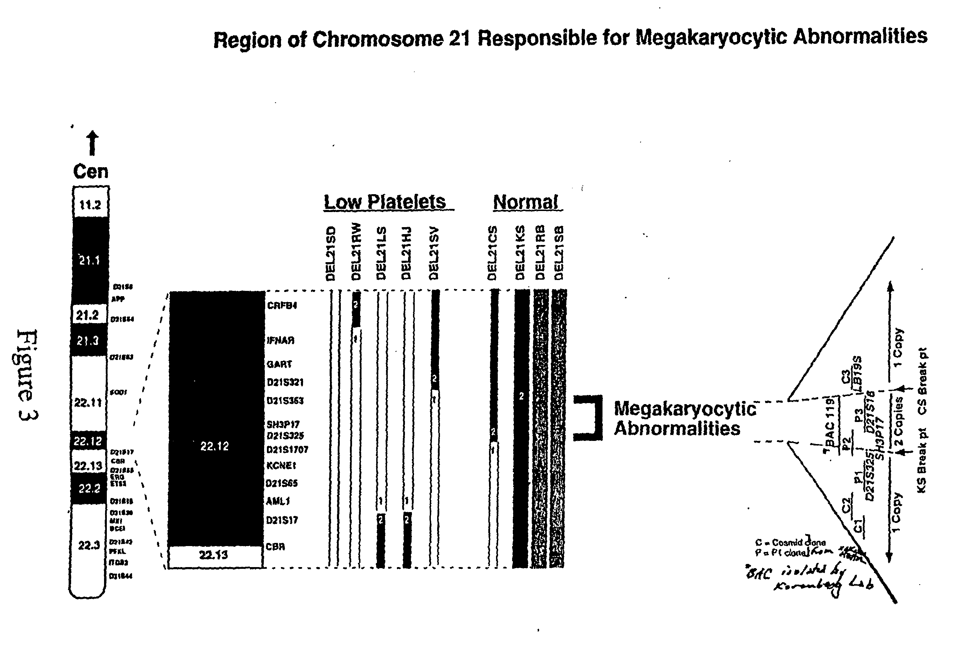

The comparison between cDNAs generated in this study vs previously published homologous, or the comparison between each cDNAs islated in this study, we found significant differences as shown in Figure 18. The differences between #21 vs ITSs, #21 vs #11 and #9 vs SH3P17 are listed here: #21 is 99.8% identical to ITSs (AF064243; Guipponi et al., 1998) at protein level showing only 1 amino acid different at the position of 114, while at the 5' UTR, the extra 160bp and XXbp difference at the 3' UTR of #21 that gives a 96.7% identity at neuleotides level; #11 was missing 5 amino acids at the position of cDNA 2573-2586 within SH3-A domain and missing 222 neucliotides within 3' UTR region while comparing to #21; #9 was 100% identical to SH3P17 (GenBank Hsu61166, Sparks et al., 1996) at coding region, but it shows 76.8% identity at neucleotides level, the major difference is at the 3' UTR, that is a total of 222bp is missing at the position of 2189 (3963-1774) to 2411 and presents at the same position as shown at #11 vs #21. #9 and SH3P17 only showed four SH3 domains missing SH3-C domain (Guipponi et al., 1998) (Figure 3).

The homologies of ITSN to other proteins were also included in Figure 2. (Sparks et al. 1996 and Guipponi et al. 1998) as discussed by Guipponi et al., 1998.

### Genomic organization of the ITSN gene and comparison to SH3P17 and ITSs/ITSI:

The comparison of the human SH3D1A to sequenced human genomic DNA (GenBank No AP000050, AP000049 and AP000048) in this region on chromosome 21 revealed that this gene consistes of 29 exons (Figure 3 and Table 2 for exact exon-intron boundaries), the sizes of which vary from 44 to 1516 bp. The sizes of the introns range from 355bp to 7.5Kb. All introns have splice donor and acceptor sites that confirm to the general GT-AG consensus motif. The putative SHD1A translation initiation codon is located on exon 2, while the stop codon is on exon 28.

### Characterization of the 5' upstream sequence

To determine the 5' upstream sequence of the human SH3D1A gene, the sequence from PAC T1276 was used to carry out the analysis for searching the promoter(s).

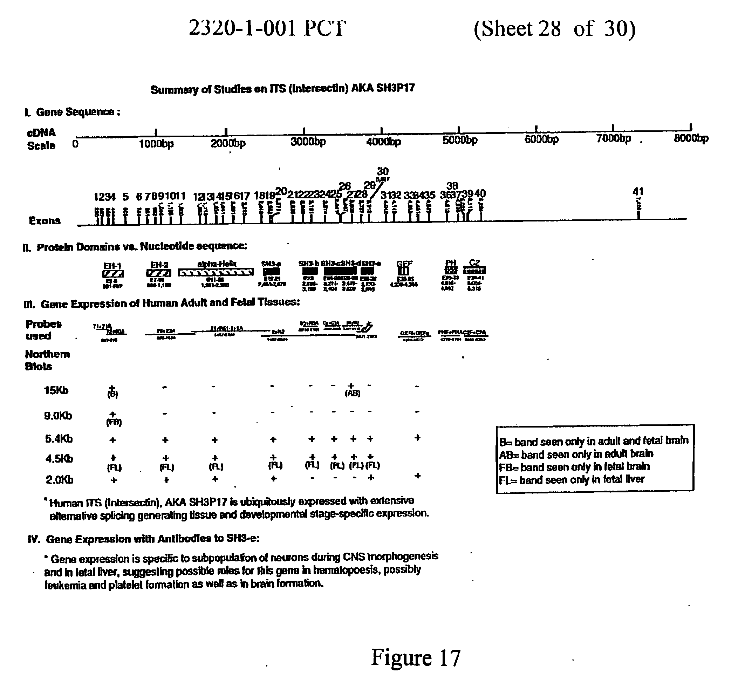

### Complex mRNA expression on multiple adult and fetal tissues (See Figure 17: Summary of studies on ITS)

As shown in the table and figure, Northern blot of SH3D1A on mutiple adult and fetal tissues revealed unexpectedly complicated results. A total of 14 probes were used for expression study (Part 1). There were 6 major mRNA transcripts detected, including a 5.4kb of mRNA fragment that was expressed ubiquitously (Heart, brain, placenta, lung, liver, muscle, kidney and pancreas) in adult and fetal tissues (brain, lung, liver and kidney) using any of the probes used as shown in the top portion of the Figure; a 25kb fragment expressed in adult ubiquitously, but strong in muscle while using probe #1 (exon 1); a 2.0 kb fragment that was expressed ubiquitously in adult and fetal while using all of the probes except for probes #2, 3 and #12-13 (exon 2-7 and exon 28-29); the strongest expression were shown on muscle in adult and on liver and brain in fetal; a 4.5kb fragment expressed ubiquitously, but stronger on liver, only seen in fetal while using probes #4, 6, 9 and 12 (exon 7 to 17 and exon 23-25, finally, a fragment larger than 11kb that was expressed specifically on brain by using probes #2 and 3 (exons 2 to 7) in adult and fetal tissue, and only seen in adult by using probe #9 (exon 22-28). Further, there was a small fragment 1.0 kb also seen on liver in fetal tissue by using probes #4 and 6(exon 7 to 17).

### RESULTS

The data presented herein confirm the role of the genes of the invention in conditions relating to leukemia as well as neural abnormalities and dysfunctions. As mentioned earlier, the genes are observed as to changes that occur in regions related to leukemia, and in relation to brain abnormalities observed with adult brain. The role of this family of genes in the regulation of both neural and leukemic conditions supports a broad modulatory influence on both development and homeostasis that commends their application in the diagnostic and therapeutic modalities presented herein.

## Claims

1. An isolated nucleic acid having the nucleotide sequence of SEQ ID NO: 1.

2. The isolated nucleic acid of claim 1, wherein the nucleic acid is DNA, such as cDNA or genomic DNA, or RNA.

3. The isolated nucleic acid of claim 1 or claim 2, wherein the nucleic acid encodes an amino, acid sequence which forms two EH domains and four SH3 domains.

4. The isolated nucleic acid of claim 3, wherein the nucleic acid encodes an amino acid sequence which forms one or more mytistoylation sites in the EH domains and SH3 domains.

5. The isolated nucleic acid of any one of claims 1 to 4, wherein the nucleic acid encodes an amino acid sequence as set forth in SEQ ID NO. 2.

6. The isolated nucleic acid of any one of claims 1 to 5, which is labeled with a detectable marker.

7. The isolated nucleic acid of claim 6, wherein the label is a radioactive isotope, a fluorophore or an enzyme.

8. A nucleic acid having a sequence complementary to the entire sequence of the isolated nucleic acid of any one of claims 1 to 5.

9. An antisense molecule capable of specifically hybridizing with the isolated nucleic acid of any of claims 1 to 5 over the entire length of said isolated nucleic acid under high stringency conditions, wherein said high stringency conditions comprise a salt concentration of at least 0.02M at pH7 and a temperature of at least 60°C.

10. A vector comprising the isolated nucleic acid of any of claims 1 to 5.

11. The vector of claim 10, further comprising a promoter ofRNA transcription or an expression element operatively linked to the nucleic acid.

12. The vector of claim 10 or claim 11 which includes a bacterial, yeast, insect or mammalian promoter.

13. The vector of any one of claims 10 to 12, further comprising plasmid, cosmid, yeast artificial chromosome (YAC), BAC, P1, bacteriophage or eukaryotic viral DNA.

14. A host/vector system for the production of a polypeptide which comprises the vector of any one of claims 10 to 13 in a suitable host which is not a human being.

15. The host/vector system of claim 14, wherein the suitable host is a prokaryotic or eukaryotic cell, such as a yeast, insect, plant or mammalian cell.

16. A method for producing a polypeptide which comprises growing the host/vector system of claim 14 or claim 15 under suitable conditions permitting production of the polypeptide and recovering the polypeptide so produced.

17. A method of obtaining a polypeptide in purified form which comprises:
(a) introducing the vector of any of claims 10 to 13 into a suitable host cell;
(b) culturing the resulting cell so as to produce the polypeptide;
(c) recovering the polypeptide produced in step (b); and
(d) purifying the polypeptide so recovered.

18. A polypeptide consisting of the amino acid sequence of SEQ ID NO. 2.

19. A fusion protein or chimeric protein comprising the polypeptide of claim 18.

20. A method for determining whether a gene encoding SEQ ID NO:2 carries a mutation comprising the steps of:
(i) contacting a nucleic acid sample and the isolated nucleic acid of any one of claims 1 to 5 with restriction enzymes under conditions permitting the digestion of the nucleic acid sample and the isolated nucleic acid into distinct, distinguishable pieces of nucleic acid;
(ii) isolating the pieces of nucleic acid; and
(iii) comparing the pieces of nucleic acid derived from the nucleic acid sample with the pieces of nucleic acid derived from the isolated nucleic acid so as to thereby determine whether the nucleic sample is, or is derived from, a nucleic acid which encodes mutant SEQ ID NO. 2.

21. A method for determining the presence of a megakaryocytic abnormality, myeloproliferative disorder, platelet disorder, leukemia or neural disorder, which comprises contacting a tissue sample with an antibody that binds to the polypeptide of claim 18 so as to thereby determine whether a subject has the megakaryocytic abnormality, myeloproliferative disorder, platelet disorder, leukemia or neural disorder.

22. A method for predicting a predisposition for a megakaryocytic abnormality, hematopoietic disorder, myeloproliferative disorder, platelet disorder, leukemia or neural disorder, which comprises determining whether a nucleic acid sample, is, or is derived from, a nucleic acid which encodes SEQ ID NO. 2 so as to thereby determine whether a subject has a predisposition for a megakaryocytic abnormality, hematopoietic disorder, myeloproliferative disorder, platelet disorder, leukemia or neural disorder.

23. A method for determining the presence of a megakaryocytic abnormality, myeloproliferative disorder, platelet disorder, leukemia or neural disorder, which comprises determining whether a nucleic acid sample is, or is derived from a nucleic acid which encodes SEQ ID NO. 2 so as to thereby determine whether a subject has megakaryocytic abnormality, myeloproliferative disorder, platelet disorder, leukemia or neural disorder.

24. The method of any one of claims 21, 22 or 23, wherein the sample comprises blood, tissue or sera.

25. An *ex vivo* method for monitoring the progress and adequacy of treatment in a subject who has received treatment for a megakaryocytic abnormality, myeloproliferative disorder, platelet disorder, leukemia condition or neural disorder which comprises monitoring the level of nucleic acid encoding SEQ ID NO. 2 at various stages of treatment.

26. An *ex vivo* method for monitoring a prenatal for tumor risk progress or megakaryocytic abnormality, myeloproliferative disorder, hematopoietic disorder, platelet disorder or leukemia which comprises monitoring the level of nucleic acid encoding SEQ ID NO. 2.

27. A pharmaceutical composition comprising an amount of the polypeptide of claim 18 or claim 19 and a pharmaceutically effective carrier or diluent.

28. A composition of claim 27, adapted for administration by topical, oral, aerosol, subcutaneous, infusion, intralesional, intramuscular, intraperitoneal, intratumoral, intratracheal, intravenous or liposome-mediated delivery.

29. The isolated nucleic acid or any one of claims 1 to 5, the antisense nucleic acid of claim 9 or the pharmaceutical composition of claim 27 or claim 28 for use in therapy.

## Patentansprüche

1. Isolierte Nukleinsäure mit der Nucleotidsequenz SEQ ID NO. 1.

2. Isolierte Nukleinsäure nach Anspruch 1, bei der es sich um DNA, wie cDNA oder genomische DNA, oder RNA handelt.

3. Isolierte Nukleinsäure nach Anspruch 1 oder 2, die für eine Aminosäuresequenz, die zwei EH-Domänen und vier SH3-Domänen bildet, kodiert.

4. Isolierte Nukleinsäure nach Anspruch 3, die für eine Aminosäuresequenz, die eine oder mehrere Myristoylierungsstellen in den EH-Domänen und SH3-Domänen bildet, kodiert.

5. Isolierte Nukleinsäure nach einem der Ansprüche 1 bis 4, die für eine Aminosäuresequenz gemäß SEQ ID NO. 2 kodiert.

6. Isolierte Nukleinsäure nach einem der Ansprüche 1 bis 5, die mit einem detektierbaren Marker markiert ist.

7. Isolierte Nukleinsäure nach Anspruch 6, wobei es sich bei dem Marker um ein radioaktives Isotop, ein Fluorophor oder ein Enzym handelt.

8. Nukleinsäure mit einer zur gesamten Sequenz der isolierten Nukleinsäure gemäß einem der Ansprüche 1 bis 5 komplementären Sequenz.

9. Antisense-Molekül, das zur spezifischen Hydridisierung mit der isolierten Nukleinsäure gemäß einem der Ansprüche 1 bis 5 über die gesamte Länge der isolierten Nukleinsäure unter hochstringenten Bedingungen befähigt ist, wobei die hochstringenten Bedingungen eine Salzkonzentration von mindestens 0,02 M bei pH 7 und eine Temperatur von mindestens 60°C umfassen.

10. Vektor, umfassend die isolierte Nukleinsäure gemäß einem der Ansprüche 1 bis 5.

11. Vektor nach Anspruch 10, ferner umfassend einen Promotor der RNA-Transkription oder ein Expressionselement, das mit der Nukleinsäure operativ verknüpft ist.

12. Vektor nach Anspruch 10 oder 11, der einen Bakterien-, Hefe-, Insekten- oder Säuger-Promotor enthält,

13. Vektor nach einem der Ansprüche 10 bis 12, ferner umfassend Plasmid-DNA, Cosmid-DNA, YAC-DNA (YAC = künstliches Hefechromosom), BAC-DNA, P1-DNA, Bakteriophagen-DNA oder eukaryotische virale DNA.

14. Wirt-Vektor-System zur Produktion eines Polypeptids, das den Vektor gemäß einem der Ansprüche 10 bis 13 in einem geeigneten Wirt, bei dem es sich nicht um einen Menschen handelt, umfaßt.

15. Wirt-Vektor-System nach Anspruch 14, wobei es sich bei dem geeigneten Wirt um eine prokaryotische oder eukaryotische Zelle, wie eine Hefe-, Insekten-, Pflanzen- oder Säugerzelle, handelt.

16. Verfahren zur Produktion eines Polypeptids, bei dem man das Wirt-Vektor-System gemäß Anspruch 14 oder 15 unter geeigneten Bedingungen, die die Produktion des Polypeptids gestatten, kultiviert und das so produzierte Polypeptid gewinnt.

17. Verfahren zum Erhalt eines Polypeptids in gereinigter Form, bei dem man:
(a) den Vektor gemäß einem der Ansprüche 10 bis 13 in eine geeignete wirtszelle einführt;
(b) die resultierende Zelle zur Produktion des Polypeptids kultiviert;
(c) das in Schritt (b) produzierte Polypeptid gewinnt und
(d) das so gewonnene Polypeptid reinigt.

18. Polypeptid, bestehend aus der Aminosäuresequenz SEQ ID NO. 2.

19. Fusionsprotein oder chimäres Protein, umfassend das Polypeptid gemäß Anspruch 18.

20. Verfahren zur Bestimmung, ob ein für SEQ ID NO. 2 kodierendes Gen eine Mutation trägt, bei dem man:
(i) eine Nukleinsäureprobe und die isolierte Nukleinsäure gemäß einem der Ansprüche 1 bis 5 unter Bedingungen, die den Verdau der Nukleinsäureprobe und der isolierten Nukleinsäure zu separaten, unterscheidbaren Nukleinsäurestücken gestatten, mit Restriktionsenzymen in Kontakt bringt;
(ii) die Nukleinsäurestücke isoliert und
(iii) die Nukleinsäurestücke aus der Nukleinsäureprobe mit den Nukleinsäurestücken aus der isolierten Nukleinsäure vergleicht, um **dadurch** zu bestimmen, ob die Nukleinprobe eine für mutierte SEQ ID NO. 2 kodierende Nukleinsäure ist oder sich davon ableitet.

21. Verfahren zur Bestimmung des Vorliegens einer megakaryozytischen Anomalie, myeloproliferativen Erkrankung, Thrombozytenerkrankung, Leukämie oder neuralen Erkrankung, bei dem man eine Gewebeprobe mit einem an das Polypeptid gemäß Anspruch 18 bindenden Antikörper in Kontakt bringt, um **dadurch** zu bestimmen, ob ein Patient eine megakaryozytische Anomalie, myeloproliferative Erkrankung, Thrombozytenerkrankung, Leukämie oder neurale Erkrankung hat.

22. Verfahren zur Vorhersage einer Veranlagung für eine megakaryozytische Anomalie, hämatopoetische Erkrankung, myeloproliferative Erkrankung, Thrombozytenerkrankung, Leukämie oder neurale Erkrankung, bei dem man bestimmt, ob eine Nukleinsäureprobe eine für SEQ ID NO. 2 kodierende Nukleinsäure ist oder sich davon ableitet, um **dadurch** zu bestimmen, ob ein Patient eine Veranlagung für eine megakaryozytische Anomalie, hämatopoetische Erkrankung, myeloproliferative Erkrankung, Thrombozytenerkrankung, Leukämie oder neurale Erkrankung hat.

23. Verfahren zur Bestimmung des Vorliegens einer megakaryozytischen Anomalie, myeloproliferativen Erkrankung, Thrombozytenerkrankung, Leukämie oder neuralen Erkrankung, bei dem man bestimmt, ob eine Nukleinsäureprobe eine für SEQ ID NO. 2 kodierende Nukleinsäure ist oder sich davon ableitet, um **dadurch** zu bestimmen, ob ein Patient eine megakaryozytische Anomalie, myeloproliferative Erkrankung, Thrombozytenerkrankung, Leukämie oder neurale Erkrankung hat.

24. Verfahren nach einem der Ansprüche 21, 22 oder 23, bei dem die Probe Blut, Gewebe oder Seren umfaßt.

25. Ex-vivo-Verfahren zur Überwachung des Fortschritts und der Adäquatheit einer Behandlung bei einem Patienten, der eine Behandlung für eine megakaryozytische Anomalie, myeloproliferative Erkrankung, Thrombozytenerkrankung, Leukämie oder neurale Erkrankung empfangen hat, bei dem man in verschiedenen Behandlungsstadien den Gehalt an für SEQ ID NO. 2 kodierender Nukleinsäure überwacht.

26. Ex-vivo-Verfahren zur Überwachung eines Ungeborenen auf Tumorrisikofortschritt oder eine megakaryozytische Anomalie, myeloproliferative Erkrankung, hämatopoetische Erkrankung, Thrombozytenerkrankung oder Leukämie, bei dem man den Gehalt an für SEQ ID NO. 2 kodierender Nukleinsäure überwacht.

27. Pharmazeutische Zusammensetzung, enthaltend eine Menge des Polypeptids gemäß Ansüruch 18 oder 19 und einen pharmazeutisch effektiven Träger oder ein pharmazeutisch effektives Verdünnungsmittel.

28. Zusammensetzung nach Anspruch 27 zur topischen, oralen, subkutanen, intraläsionalen, intramuskulären, intraperitonalen, intratumoralen, intratrachealen, inravenösen oder liposomenvermittelten Zufuhr oder Zufuhr mittels Aerosol oder Infusion.

29. Isolierte Nukleinsäure nach einem der Ansprüche 1 bis 5, Antisense-Nukleinsäure nach Anspruch 9 oder pharmazeutische Zusammensetzung nach Anspruch 27 oder 28 zur Verwendung bei der Therapie.

## Revendications

1. Acide nucléique isolé ayant la séquence nucléotidique SEQ ID n° 1.

2. Acide nucléique isolé selon la revendication 1, dans lequel l'acide nucléique est de l'ADN tel que de l'ADNc ou ADN génomique, ou de l'ARN.

3. Acide nucléique isolé selon la revendication 1 ou la revendication 2, dans lequel l'acide nucléique code pour une séquence d'acides aminés, ce qui forme deux domaines EH et quatre domaines SH3.

4. Acide nucléique isolé selon la revendication 3, dans lequel l'acide nucléique code pour une séquence d'acides aminés, ce qui forme un ou plusieurs sites de myristoylation dans les domaines EH et les domaines SH3.

5. Acide nucléique isolé selon l'une quelconque des revendications 1 à 4, dans lequel l'acide nucléique code pour une séquence d'acides aminés telle qu'indiquée à la SEQ ID n° 2.

6. Acide nucléique isolé selon l'une quelconque des revendications 1 à 5, lequel est marqué à l'aide d'un marqueur détectable.

7. Acide nucléique isolé selon la revendication 6, dans lequel le marqueur est un isotope radioactif, un fluorophore ou une enzyme.

8. Acide nucléique ayant une séquence complémentaire à la séquence complète de l'acide nucléique isolé selon l'une quelconque des revendications 1 à 5.

9. Molécule antisens capable de s'hybrider spécifiquement avec l'acide nucléique isolé de l'une quelconque des revendications 1 à 5 sur toute la longueur dudit acide nucléique isolé dans des conditions de stringence élevée, où lesdites conditions de stringence élevée comprennent une concentration de sel d'au moins 0,02 M à un pH de 7 et une température d'au moins 60 °C.

10. Vecteur comprenant l'acide nucléique isolé selon l'une quelconque des revendications 1 à 5.

11. Vecteur selon la revendication 10, comprenant en outre un promoteur de transcription de l'ARN ou un élément d'expression lié de manière fonctionnelle à l'acide nucléique.

12. Vecteur selon la revendication 10 ou la revendication 11 qui comprend un promoteur de bactérie, de levure, d'insecte ou de mammifère.

13. Vecteur selon l'une quelconque des revendications 10 à 12, comprenant en outre un plasmide, un cosmide, un chromosome artificiel de levure (YAC), un BAC, un P1, un ADN viral bactériophage ou eucaryote.

14. Système hôte/vecteur pour la production d'un polypeptide qui comprend le vecteur selon l'une quelconque des revendications 10 à 13 dans un hôte approprié, qui n'est pas un être humain.

15. Système hôte/vecteur selon la revendication 14, dans lequel l'hôte approprié est une cellule procaryote ou eucaryote, telle qu'une cellule de levure, d'insecte, de plante ou de mammifère.

16. Procédé de production d'un polypeptide qui comprend la croissance du système hôte/vecteur selon la revendication 14 ou la revendication 15 dans des conditions appropriées qui permettent la production du polypeptide et la récupération du polypeptide ainsi produit.

17. Procédé pour l'obtention d'un polypeptide sous une forme purifiée qui comprend ;
(a) l'introduction du vecteur selon l'une quelconque des revendications 10 à 13 dans une cellule hôte appropriée ;
(b) la culture de la cellule résultante de façon à produire le polypeptide ;
(c) la récupération du polypeptide produit à l'étape (b) ; et
(d) la purification du polypeptide ainsi récupéré.

18. Polypeptide constitué de la séquence d'acides aminés de la SEQ ID n° 2.

19. Protéine de fusion ou protéine chimère comprenant le polypeptide selon la revendication 18.

20. Procédé pour déterminer si un gène codant la SEQ ID n° 2 porte une mutation qui comprend les étapes de :
(i) La mise en contact d'un échantillon d'acide nucléique et de l'acide nucléique isolé selon l'une quelconque des revendications 1 à 5 avec des enzymes de restriction dans des conditions qui permettent la digestion de l'échantillon d'acide nucléique et de l'acide nucléique isolé en des fragments distincts et distinguables d'acide nucléique ;
(ii) isolation des fragments d'acide nucléique ; et
(iii) a comparaison des fragments d'acide nucléique dérivés de l'échantillon d'acide nucléique avec les fragments d'acide nucléique dérivés de l'acide nucléique isolé de façon à ainsi déterminer si l'échantillon nucléique est un, ou est dérivé d'un, acide nucléique qui code un mutant de la SEQ ID n° 2.

21. Procédé pour déterminer la présence d'une anomalie mégacaryocyte, d'un trouble myéloprolifératif, d'un désordre plaquettaire, d'une leucémie ou d'un trouble neural, qui comprend la mise en contact d'un échantillon de tissu avec un anticorps qui se lie au polypeptide selon la revendication 18 de façon à ainsi déterminer si un sujet présente une anomalie mégacaryocyte, un trouble myéloprolifératif, un désordre plaquettaire, une leucémie ou un trouble neural.

22. Procédé pour prédire une prédisposition à une anomalie mégacaryocyte, à un trouble hématopoïétique, à un trouble myéloprolifératif; à un désordre plaquettaire, à une leucémie ou à un trouble neural, qui consiste à déterminer si un échantillon d'acide nucléique est un, ou est dérivé d'un, acide nucléique qui code une SEQ ID n° 2 de façon à ainsi déterminer si un sujet présente une prédisposition à une anomalie mégacaryocyte, à un trouble hématopoïétique, à un trouble myéloprolifératif, à un désordre plaquettaire, à une leucémie ou à un trouble neural.

23. Procédé pour déterminer la présence d'une anomalie mégacaryocyte, d'un trouble myéloprolifératif, d'un désordre plaquettaire, d'une leucémie ou d'un trouble neural, qui consiste à la déterminer si un échantillon d'acide nucléique est un, ou est dérivé d'un, acide nucléique qui code une SEQ ID n° 2 de façon à ainsi déterminer si un sujet présente une anomalie mégacaryocyte, un trouble myéloprolifératif, un désordre plaquettaire, une leucémie ou un trouble neural.

24. Procédé selon l'une quelconque des revendications 21, 22 ou 23, dans lequel l'échantillon comprend du sang, du tissu ou du sérum.

25. Procédé ex vivo pour suivre le progrès et l'adéquation d'un traitement chez un sujet qui a reçu un traitement pour une anomalie mégacaryocyte, un trouble myéloprolifératif, un désordre plaquettaire, un état leucémique ou un trouble neural, ledit procédé comprenant le suivi du niveau d'acide nucléique qui code pour la SEQ ID n° 2 à diverses étapes du traitement.

26. Procédé ex vivo pour suivre, pendant l'étape prénatale, le progrès des risques de tumeurs ou une anomalie mégacaryocyte, un trouble myéloprolifératif, un trouble hématopoïétique, un désordre plaquettaire ou une leucémie, ledit procédé comprenant le suivi du niveau d'acide nucléique qui code pour la SEQ ID n° 2.

27. Composition pharmaceutique qui comprend une quantité du polypeptide selon la revendication 18 ou la revendication 19 ainsi qu'un excipient ou un diluant pharmaceutiquement efficace.

28. Composition selon la revendication 27, adaptée pour une administration topique, orale, par aérosol, sous-cutanée, par perfusion, intralésionnelle, intramusculaire, intrapéritonéale, intratumorale, intratrachéale, intraveineuse ou induite par des liposomes.

29. Acide nucléique isolé selon l'une quelconque des revendications 1 à 5, acide nucléique antisens selon la revendication 9 ou composition pharmaceutique selon la revendication 27 ou la revendication 28 destiné(e) à être utilisé(e) dans une thérapie.
